# EUROPEAN PATENT APPLICATION

(11) **EP 2 845 860 A1**
(43) Date of publication of application: **11.03.2015**
(21) Application number: 13183826.0
(22) Date of filing: 10.09.2013
(51) Int. Cl.: C07H 15/04, C07K 19/00, A61K 31/7028, A61P 31/04

(54) **Synthetic vaccines against Streptococcus pneumoniae**

(71) Applicant: Max-Planck-Gesellschaft zur Förderung der Wissenschaften e.V., 80539 München (DE)
(72) Inventor: Seeberger, Peter H. Prof. Dr., 14532 Kleinmachnow, (DE); Pereira, Claney Lebev, Dr., 12203 Berlin (DE)
(74) Representative: Arth, Hans-Lothar

(57) **Abstract**

The present invention relates to the total synthesis of saccharide structures contained in the capsular polysaccharide of *Streptococcus pneumoniae* type 1, to glycoconjugates containing said saccharide structures obtained by total synthesis and to use of such glycoconjugates and pharmaceutical compositions thereof in the immunization against diseases associated with bacteria containing said saccharide structures in their capsular polysaccharide, and more specifically associated with *Streptococcus pneumoniae.*

## Description

### Field of the invention

The present invention relates to the total synthesis of saccharide structures contained in the capsular polysaccharide of *Streptococcus pneumoniae* type 1, to glycoconjugates containing said saccharide structures obtained by total synthesis and to use of such glycoconjugates and pharmaceutical composition thereof in the immunization against diseases associated with bacteria, and more specifically against diseases associated with *Streptococcus pneumoniae.*

### Background of the invention

Gram-positive encapsulated bacterium *Streptococcus pneumoniae* (pneumococcus) is a major cause of morbidity and mortality worldwide. They colonize the upper respiratory tract and cause invasive pneumococcal diseases such as meningitis, bacteremia and bactermic pneumonia, and non-invasive pneumococcal diseases including acute otis media and pneumonia. These diseases are prevalent in young children, the elderly and immunocompromised individuals of all ages. In developing countries *Streptococcus pneumoniae* related diseases cause an estimated 1.2 million deaths annually of young children.

Structurally, three distinguished layers can be seen on the bacterial surface: plasma membrane, cell wall and capsule. The cell wall consists of a peptidoglycan backbone anchoring the cell wall polysaccharide (C-polysaccharide) and the capsular polysaccharide (CPS). The C-polysaccharide is a structure common to all the pneumococcal serotypes, whereas CPS is specific to each of the 90 know serotypes and is the main virulence factor.

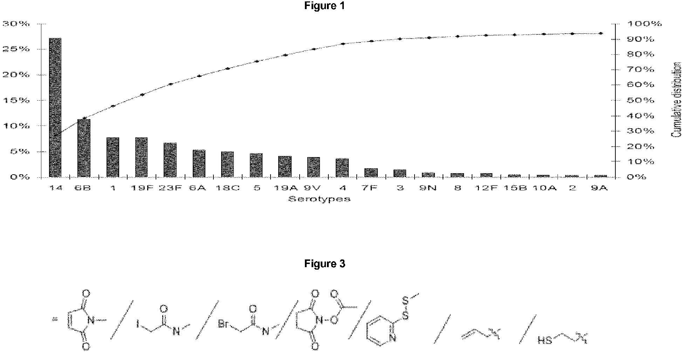

Out of the 90 serotypes the most common and prevalent serotypes found in the world are shown in figure 1. This distribution varies also based on geography and age difference. Thus, a vaccine comprising glycoconjugates containing an immunogenic carrier and saccharide structures derived from the capsular polysaccharide of the most common and prevalent *Streptococcus pneumoniae* serotypes would provide immunization against a high percentage of the diseases caused by this class of Gram-positive bacteria.

Several poly-valent pneumococcal vaccines were manufactured up to present. The commercially available 23-valent pneumococcal polysaccharide vaccine (PPV), contains purified capsular polysaccharide (CPS) antigens of 23 serotypes. However, this vaccine is not effective in the case of infants and young children. The currently marketed pneumococcal conjugate vaccine (PCV), PCV-7 (PrevnarTM) contains saccharides of capsular antigens of serotype 4, 6B, 9V, 14, 18C, 19F and 23F individually conjugated to diphtheria CRM₁₉₇ and is effective in infants.

The currently marketed vaccines are effective in North America and Europe for individuals of a particular age. The manufacturing process for these vaccines is complex and results in a higher price. Therefore, the vaccine is unaffordable in most developing countries. It is the object of the present invention to provide affordable synthetic saccharide vaccines that contain most of the prevalent serotypes of the developing world.

*Streptococcus pneumoniae* type 1 (SP1) is one of the most prevalent *S. pneumoniae* serotypes. *Streptococcus pneumoniae* type 1 capsular polysaccharide is a linear polymer having as a repeating unit: [→3)-α-2,4,6-trideoxy-4-amino-D-GalNAc-(1→4)-α-D-GalAp-(1→3)-α-D-GalAp-(1→].

Synthetic saccharide structures derived from [→3)-α-2,4,6-trideoxy-4-amino-D-GalNAc-(1→4)-α-D-GalAp-(1→3)-α-D-GalAp-(1→] trisaccharide repeating unit of *Streptococcus pneumoniae* type 1 capsular polysaccharide were already reported. However, the method developed by Bundle (Chem. Eur. J. 2010, 16, 3476.) provides α-methoxy saccharides, which are not suitable for conjugation to an immunogenic carrier. Moreover, the procedure described by Codée is low yielding due to the side-reactions and requires additional steps for providing saccharides suitable for conjugation (J. Org. Chem. 2011, 76, 1692.)

It is the objective of the present invention to provide an improved synthetic route to access saccharide structures functionalized with a linker, said saccharide structures being derived from [→3)-α-2,4,6-trideoxy-4-amino-D-GalNAc-(1→4)-α-D-GalAp-(1→3)-α-D-GalAp-(1→] trisaccharide repeating unit of *Streptococcus pneumoniae* type 1 capsular polysaccharide. Said saccharide structures have the advantage of being functionalized with a linker thus, being suitable to be conjugated to an immunogenic carrier. Therefore, it is an objective of the present invention to provide glycoconjugates and pharmaceutical compositions containing said glycoconjugates for immunization against diseases associated with bacteria containing in their capsular polysaccharide one of the following structures: α-2,4,6-trideoxy-4-amino-D-GalNAc-(1→4)-α-D-GalAp-(1→3)-α-D-GalAp-; α-2,4,6-trideoxy-4-amino-D-GalNAc-(1→4)-α-D-GalAp; α-D-GalAp-(1→3)-a-D-GalAp; α-D-GalAp; α-2,4,6-trideoxy-4-amino-D-GalNAc; α-D-GalAp-(1→3)-α-D-GalAp-(1→3)-α-2,4,6-trideoxy-4-amino-D-GaINAc; α-D-GalAp-(1→3)-α-2,4,6-trideoxy-4-amino-D-GalNAc; α-D-GalAp-(1→3)-α-2,4,6-trideoxy-4-amino-D-GalNAc-(1→4)-α-D-GalAp. The pharmaceutical compositions comprising the glycoconjugates of the present invention are useful for immunization against diseases associated with bacteria, and especially associated with *Streptococcus pneumoniae,* said diseases including pneumonia, meningitis, otitis media, bacteremia and acute exacerbation of chronic bronchitis, sinusitis, arthritis and conjunctivitis

The objective of the present invention is solved by the teaching of the independent claims. Further advantageous features, aspects and details of the invention are evident from the dependent claims, the description, the figures, and the examples of the present application.

### Description of the invention

### Definitions

The term "linker" as used herein encompasses molecular fragments capable of connecting the reducing-end monosaccharide of a saccharide with an immunogenic carrier or a solid support, optionally by binding to at least one interconnecting molecule. Thus, the function of the linker *per se* or together with the interconnecting molecule is to establish, keep and/or bridge a special distance between the reducing-end monosaccharide and an immunogenic carrier or a solid support. More specifically, one extremity of the linker is connected to the oxygen atom of the reducing-end monosaccharide and the other extremity is connected with the interconnecting molecule, or directly with the immunogenic carrier or the solid support.

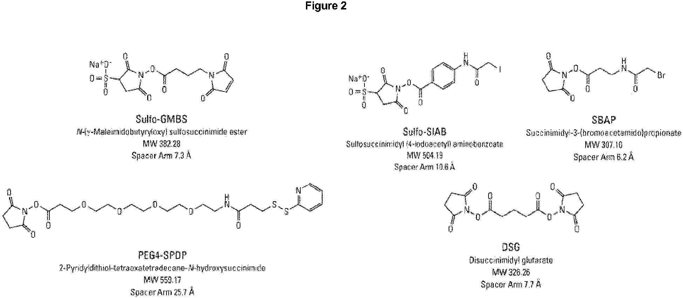

As used herein, the term "interconnecting molecule" refers to a bifunctional molecule containing functional group X and functional group Y, wherein functional group X is capable of reacting with the terminal thiol group on the linker A and the functional group Y is capable of binding to an immunogenic carrier or to a solid support. Figure 2 displays examples of interconnecting molecules, but does not restrict the interconnecting molecules that can be used according to the present invention to the examples displayed herein.

The term "adjuvant" as used herein refers to an immunological adjuvant i.e. a material used in a vaccine composition that modifies or augments the effects of said vaccine by enhancing the immune response to a given antigen contained in the vaccine without being antigenically related to it. For the person skilled in the art, classically recognized examples of adjuvants include:
- mineral-containing compositions, including calcium salts and aluminum salts (or mixtures thereof). Calcium salts include calcium phosphate. Aluminum salts include hydroxides, phosphates, sulfates, etc., with the salts taking any suitable form (e.g. gel, crystalline, amorphous, etc.). Adsorption to these salts is preferred. The mineral containing compositions may also be formulated as a particle of metal salt. The adjuvants known as aluminum hydroxide and aluminum phosphate may be also used. The invention can use any of the "hydroxide" or "phosphate" adjuvants that are in general use as adjuvants. The adjuvants known as "aluminium hydroxide" are typically aluminium oxyhydroxide salts, which are usually at least partially crystalline. The adjuvants known as "aluminium phosphate" are typically aluminium hydroxyphosphates, often also containing a small amount of sulfate (i. e. aluminium hydroxyphosphate sulfate). They may be obtained by precipitation, and the reaction conditions and concentrations during precipitation influence the degree of substitution of phosphate for hydroxyl in the salt. Mixtures of both an aluminium hydroxide and an aluminium phosphate can be employed in the formulation according to the present invention;
- saponins, which are a heterologous group of sterol glycosides and triterpenoid glycosides that are found in the bark, leaves, stems, roots and even flowers of a wide range of plant species. Saponin from the bark of the Quillaia saponaria Molina tree have been widely studied as adjuvants. Saponin can also be commercially obtained from Smilax ornata (sarsaprilla), Gypsophilla paniculata (brides veil), and Saponaria oficianalis (soap root). Saponin adjuvant formulations include purified formulations, such as QS21, as well as lipid formulations, such as ISCOMs. Saponin compositions have been purified using HPLC and RP-HPLC. Specific purified fractions using these techniques have been identified, including QS7, QS 17, QS 18, QS2 1, QH-A, QH-B and QH-C. Saponin formulations may also comprise a sterol, such as cholesterol. Combinations of saponins and cholesterols can be used to form unique particles called immunostimulating complexes (ISCOMs). ISCOMs generally include a phospholipid such as phosphatidylethanolamine or phosphatidylcholine. Any known saponin can be used in ISCOMs. Preferably, the ISCOM includes one or more of QuilA, QHA & QHC;
- microparticles (i.e. a particle of -100nm to -150pm in diameter, more preferably - 200nm to -30pm in diameter, or -500nm to -10pm in diameter) formed from materials that are biodegradable and non-toxic. Such non-toxic and biodegradable materaila include but are not restricted to poly(α-hydroxy acid), polyhydroxybutyric acid, polyorthoester, polyanhydride, polycaprolactone;
- CD1d ligands, such as an α-glycosylceramide, phytosphingosine-containing α-glycosylceramides, OCH, KRN7000 [(2*S*,3*S*,4*R*)-1-*O*-(α-D-galactopyranosyl)-2-(*N-*hexacosanoylamino)- 1,3,4-octadecanetriol], CRONY- 101, 3"-0- sulfo-galactosylceram ide;
- immunostimulatory oligonucleotides, such CpG motif containing ones (a dinucleotide sequence containing an unmethylated cytosine residue linked by a phosphate bond to a guanosine residue), or Cpl motif containing ones (a dinucleotide sequence containing cytosine linked to inosine), or a double-stranded RNA, or an oligonucleotide containing a palindromic sequence, or an oligonucleotide containing a poly(dG) sequence. Immunostimulatory oligonucleotides can include nucleotide modifications/analogs such as phosphorothioate modifications and can be double-stranded or (except for RNA) single-stranded;
- compounds containing lipids linked to a phosphate-containing acyclic backbone, such as the TLR4 antagonist E5564;
- oil emulsions (eg. Freund's adjuvant).

Theoretically, each molecule or substance that is able to favor or amplify a particular situation in the cascade of immunological events, ultimately leading to a more pronounced immunological response, can be defined as an adjuvant.

In principle, through the use of adjuvants in vaccine formulations, one can
- direct and optimize immune responses that are appropriate or desirable for the vaccine;
- enable mucosal delivery of vaccines, i.e., administration that results in contact of the vaccine with a mucosal surface such as buccal or gastric or lung epithelium and the associated lymphoid tissue;
- promote cell-mediated immune responses;
- enhance the immunogenicity of weaker immunogens, such as highly purified or recombinant antigens;
- reduce the amount of antigen or the frequency of immunization required to provide protective immunity; and
- improve the efficacy of vaccines in individuals with reduced or weakened immune responses, such as newborns, the aged, and immunocompromised vaccine recipients.

Although little is known about their mode of action, it is currently believed that adjuvants augment immune responses by one of the following mechanisms:
- increasing the biological or immunologic half-life of antigens;
- improving antigen delivery to antigen-presenting cells (APCs), as well as antigen processing and presentation by the APCs e.g., by enabling antigen to cross endosomal membranes into the cytosol after ingestion of antigen-adjuvant complexes by APC;
- mimicking danger inducing signals from stressed or damaged cells which serve to initiate an immune response;
- inducing the production of immunomodulatory cytokines;
- biasing the immune response towards a specific subset of the immune system; and
- blocking the rapid dispersal of the antigen challenge.

Saccharides are known by the person skilled in the art as TI-2 (T cell independent-2) antigens and poor immunogens. Therefore, to produce a saccharide-based vaccine, said saccharides are conjugated to an immunogenic carrier to provide a glycoconjugate, which presents an increased immunogenicity in comparison with the saccharide. In this context the term "immunogenic carrier" is defined as a structure, which is conjugated to the saccharide to form a glycoconjugate that presents an increased immunity in comparison with the saccharide *per se.* Thus, the conjugation of the saccharides to the immunogenic carrier has as effect the stimulation of the immune response against said saccharide, without inducing an immune response against the said immunogenic carrier.

Thus the present invention relates to saccharides of general formula **(I):** 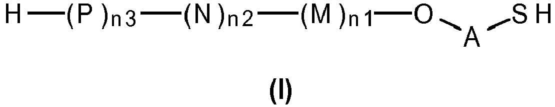 wherein A is a linker;

M, N and P represent independently of each other one of the following sugar fragments: 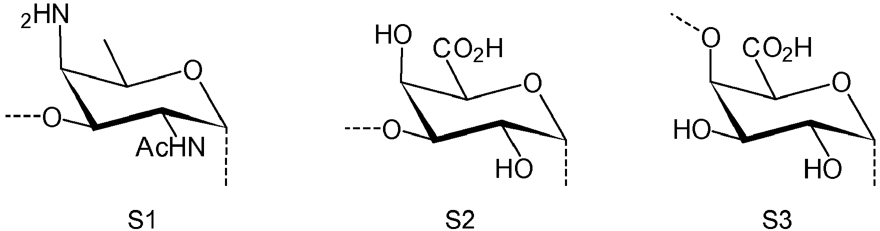 wherein each moiety S1, S2, and S3 is not more than once present in the general formula (I), sugar fragment S1 cannot be simultaneously bound to -O-A-SH and sugar fragment S2, sugar fragment S3 cannot be simultaneously bound to -O-A-SH and sugar fragment S1 and sugar fragment S2 cannot be simultaneously bound to -O-A-SH and sugar fragment S3, and n1, n2 and n3 are integers selected from 0 and 1
wherein at least one of the integers n1, n2 and n3 is 1 and
and pharmaceutically acceptable salts of these saccharides.

Preferably not more than one of n1, n2 and n3 is 0 or n1 = n2 = n3 = 1. Also saccharides of general formula (I) are preferred which comprise the sugar S1. Thus it is preferred that general formula (I) represents the following sugars: H-(S1)-(S2)-(S3)-O-A-SH, H-(S2)-(S3)-(S1)-O-A-SH, H-(S3)-(S1)-(S2)-O-A-SH, H-(S1)-(S2)-O-A-SH, H-(S2)-(S3)-O-A-SH, H-(S3)-(S1)-O-A-SH, H-(S1)-O-A-SH, H-(S2)-O-A-SH and H-(S3)-O-A-SH and more preferred H-(S1)-(S2)-(S3)-O-A-SH, H-(S2)-(S3)-(S1)-O-A-SH, H-(S3)-(S1)-(S2)-O-A-SH, H-(S1)-(S2)-O-A-SH, H-(S2)-(S3)-O-A-SH, H-(S3)-(S1)-O-A-SH, and H-(S1)-O-A-SH. Also preferred are the disulfides of such sugars, namely H-(S1)-(S2)-(S3)-O-A-S-A-O-(S3)-(S2)-(S1)-H, H-(S2)-(S3)-(S1)-O-A-S-A-O-(S1)-(S3)-(S2)-H, H-(S3)-(S1)-(S2)-O-A-S-A-O-(S2)-(S1)-(S3)-H, H-(S1)-(S2)-O-A-S-A-O-(S2)-(S1)-H, H-(S2)-(S3)-O-A-S-A-O-(S3)-(S2)-H, H-(S3)-(S1)-O-A-S-A-O-(S1)-(S3)-H, H-(S1)-O-A-S-A-O-(S1)-H, H-(S2)-O-A-S-A-O-(S2)-H, and H-(S3)-O-A-S-A-O-(S3)-H. More preferred are H-(S1)-(S2)-(S3)-0-A-S-A-0-(S3)-(S2)-(S1)-H, H-(S2)-(S3)-(S1)-O-A-S-A-O-(S1)-(S3)-(S2)-H, H-(S3)-(S1)-(S2)-O-A-S-A-O-(S2)-(S1)-(S3)-H, H-(S1)-(S2)-O-A-S-A-O-(S2)-(S1)-H, H-(S2)-(S3)-O-A-S-A-O-(S3)-(S2)-H, H-(S3)-(S1)-O-A-S-A-O-(S1)-(S3)-H and H-(S1)-O-A-S-A-O-(S1)-H.

In above formula (I) or in any other formula of the description comprising a linker A represents any suitable linker. Preferably A represents a linker containing up to 50 carbon atoms. This carbon atom based linker contains up to 60 carbon atoms and preferably up to 40 carbon atoms and more preferably between 1 and 26 carbon atoms and most preferably between 2 and 20 carbon atoms. Such a linker may further contain between 1 to 30 oxygen atoms, 1 to 10 nitrogen atoms and 1 to 10 sulfur atoms. The function of the linker is to covalently connect the reducing-end of the saccharides to an immunogenic carrier or to a solid support. Preferred are linkers A with are linear carbon chains consisting of up to 50 carbon atoms. This carbon atom based linker contains up to 60 carbon atoms and preferably up to 40 carbon atoms and more preferably between 1 and 26 carbon atoms and most preferably between 2 and 20 carbon atoms. Also preferred are polyethylene glycol (PEG) linkers A comprising up to 60 carbon atoms and preferably up to 40 carbon atoms and more preferably between 4 and 26 carbon atoms and most preferably between 4 and 20 carbon atoms. Also preferred are shorter linkers A selected from the following group: -CH₂-O-CH₂-, -CH₂-CH₂-O-CH₂-, -CH₂-O-CH₂-CH₂-, -CH₂-CH₂-O-CH₂-CH₂-, -CH₂-O-C₂H₄-O-CH₂-, -CH₂-CH₂-O-C₂H₄-O-CH₂-, -CH₂-O-C₂H₄-O-CH₂-CH₂-, -CH₂-CH₂-O-C₂H₄-O-CH₂-CH₂-. Especially preferred are linkers A of the following structure: -(CH₂)_{w}-, wherein w is an integer 1, 2, 3, 4, 5, 6, 7, 8, 9, 10, 11, 12, 13, 14, 15, 16, 17, 18, 19, 20 and wherein one, two, three or four hydrogen atoms can be replaced independently of each other by one of the substituents mentioned in list disclosed four paragraphs further down.

An interconnecting molecule according to the present invention refers to a bifunctional molecule containing functional group X and functional group Y, wherein functional group X is capable of reacting with the terminal thiol group on the linker A and the functional group Y is capable of binding to an immunogenic carrier or to a solid support.

The present invention refers to saccharides of the formula **(I),** wherein the linker *per* se or together with the interconnecting molecule is capable of establishing, keeping and/or bridging a special distance between the reducing-end monosaccharide and an immunogenic carrier or a solid support.

Thus, the term "linker" as used herein is a chain of at least one carbon atom and up to 50 carbon atoms, wherein this chain of 1 to 50 carbon atoms may be linear or branched and may further contain 1 to 30 oxygen atoms and/or 1 to 10 nitrogen atoms and/or 1 to 10 sulfur atoms and/or 1 to 3 phenyl moieties and/or 1 to 3 saturated 3-, 4-, 5- or 6-membered carbocyclic rings and/or 1 to 3 saturated 3-, 4-, 5-or 6-membered heterocyclic rings containing one or two heteroatoms selected from O, N and S, wherein this chain, the phenyl moieties and/or the carbocyclic or heterocyclic rings within said chain can be substituted with 1 to 10 substituents selected from the group consisting of:
=O (carbonyl group), -OH, -OCH₃, -OC₂H₅, -OC₃H₇₃ -O-cyclo-C₃H₅, -OCH(CH₃)₂, -OC(CH₃)₃, -OC₄H₉, -OPh, -OCH₂-Ph, -OCPh₃, -SCH₃, -SC₂H₅, -SC₃H₇, -S-cyclo-C₃H₅, -SCH(CH₃)₂, -SC(CH₃)₃, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CO-N[C(CH₃)₃]₂, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃ and -CH(CH₃)-C(CH₃)₃.

The terminal thiol group on the linker A is connected to one carbon atom of this chain, which is preferably a terminal carbon atom thus having beside the sulfur atom of the terminal thiol group only one further carbon atom to which this terminal carbon atom is connected. Also one carbon atom of this chain is connected to the oxygen atom of the reducing-end monosaccharide of the saccharide of general formula (I).

Further, the present invention refers to synthetic saccharides of the formula (I), wherein the linker is a molecular fragment capable of connecting the reducing-end monosaccharide of saccharides of general formula (I) via the thiol group with an immunogenic carrier or a solid support, optionally by binding to at least one further interconnecting molecule.

More preferably A represents -A¹-A²-A³-, -A¹-A³-, -A¹-, or -A¹-A²- A⁴-A⁵-A³-, wherein
A¹ and A⁴ represent independently of each other -(CH₂)ₒ-, -(CH₂)ₘ-, -(CH₂)ₒ-CHR¹-(CH₂)ₘ-, -(CH₂)ₒ-CR¹R²-(CH₂)ₘ-, -(C₂H₄O)ₒ-CH₂-, ₋(C₂H₄O)ₒ-C₂H₄-, -*o*-C₆H₄-, -*m*-C₆H₄-, -*p*-C₆H₄-, 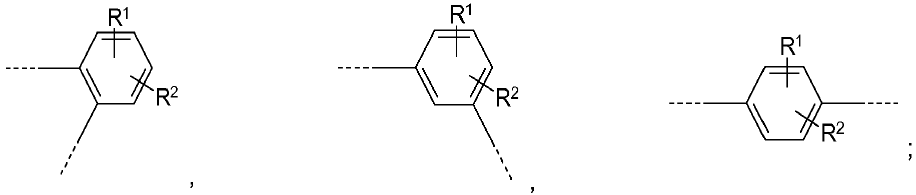 A² and A⁵ represent independently of each other -(CH₂)ₒ-, -(CH₂)_{q}-, -CHR³-, -CR³R⁴-, -O-, -S-, -S-S-, -CO-, -COO-, -O-CO-, -NH-CO-, -CO-NH-, -NH-CO-NH-, -*o*-C₆H₄-, -m-C₆H₄-, -p-C₆H₄-, -NR⁷-, -CH=CH-, 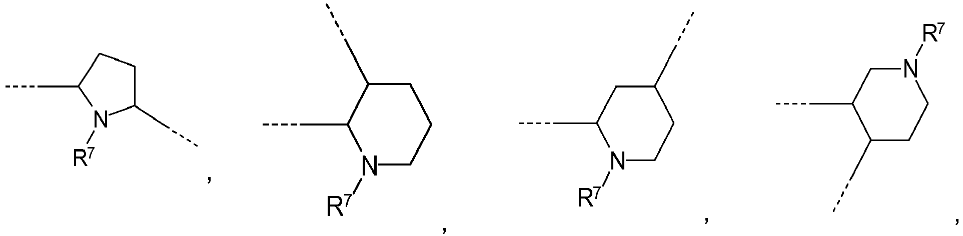 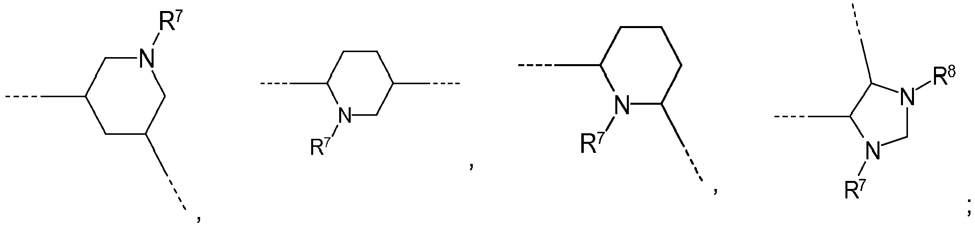 A³ represents -(CH₂)ᵣ-, -(CH₂)ᵣ-CR⁵R⁶-(CH₂)ₛ-, -*o*-C₆H₄-, -*m*-C₆H₄-, -*p*-C₆H₄-, 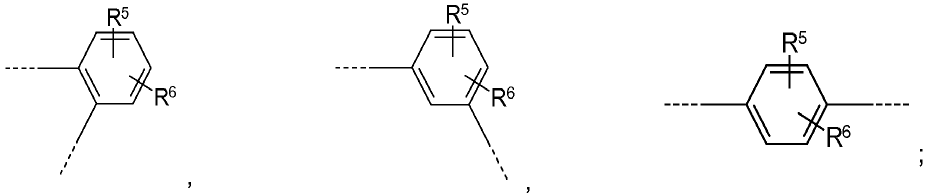 R¹ to R⁶ represent independently of each other -H, -NH₂, -OH, -OCH₃, -OC₂H₅, -OC₃H₇, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH₂NH₂, -CH₂OH, -CH₂-CH₂NH₂, -C₆H₄-OCH₃, -C₆H₄-OH₃ -CH₂-CH₂-OCH₃, -CH₂-CH₂OH, -CH₂-OCH₃, -CH₂-C₆H₄-OCH₃, -CH₂-C₆H₄-OH;
R⁷ and R⁸ represent independently of each other cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -C≡CH, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-C≡CH, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -C=C-CH₃, -CH=CH-C₃H₇, -CH₂-CH=CH-CH=CH₂, -CH=CH-CH=CH-CH₃, -CH₂NH₂, -CH₂OH, -CH₂-CH₂NH₂, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH;
o, m, r and s represent independently of each other an integer from 1 to 20;
p and q represent independently of each other an integer from 0 to 5.

Preferred are linkers wherein A represents A¹ and A¹ has the meanings as disclosed herein or preferably A¹ represents -(CH₂)ₘ-, -(CH2)ₒ-CHR¹-(CH2)ₘ-, -(CH₂)ₒ-CR¹R²-(CH₂)ₘ- -(C₂H₄O)ₒ-CH₂- -(C₂H₄O)ₒ-C₂H₄- -*o*-C₆H₄-, -*m*-C₆H₄-, or -*p*-C₆H₄- and even more preferably -(CH₂)ₘ-, -(CH₂)ₒ-CHR¹-(CH₂)ₘ-, -(CH₂)ₒ-CR¹R²-(CH₂)ₘ-, -(C₂H₄O)ₒ-CH₂-, -(C₂H₄O)ₒ-C₂H₄-, wherein m, o, R¹, and R² have the meanings as disclosed herein and preferably -H, -NH₂, -OH, -OCH₃, -OC₂H₅, -OC₃H₇, cyclo-C₃H₅, cyclo-C₄H₇, cyclo-C₅H₉, cyclo-C₆H₁₁, -Ph, -CH₂-Ph, -CH₃, -C₂H₅, -C₃H₇, -CH(CH₃)₂, -C₄H₉, -CH₂-CH(CH₃)₂, -C₅H₁₁, -CH=CH₂, -C≡CH, -CH₂-CH=CH₂, -CH=CH-CH₃, -CH₂NH₂, -CH₂OH, -CH₂-CH₂NH₂, -CH₂-CH₂-OCH₃, -CH₂-CH₂OH, -CH₂-OCH₃, and more preferably -H, -NH₂, -OH, -OCH₃, -OC₂H₅, -OC₃H₇, cyclo-C₃H₅, -CH₃, -C₂H₅, -C₃H₇, -CH(CH₃)₂, -CH=CH₂, -CH₂-CH=CH₂, -CH₂NH₂, -CH₂OH, -CH₂-CH₂NH₂, -CH₂-CH₂-OCH₃, -CH₂-CH₂OH, -CH₂-OCH₃.

The compounds of the present invention bear basic and/or acidic substituents and they may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, *p*-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, *p*-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (*o*, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Further, it is also possible that the compounds of the present invention bear simultaneously basic and acid groups. Further, it may also occur that these basic and acid groups appear to be in close vicinity to one another enabling an intramolecular proton transfer from the acidic group the basic group. Therefore, in a preferred embodiment of the present invention the compound of the formula (I) may be zwitterionic, bearing at least e.g. one -O- and one -NH₃⁺ group.

Thus, the present invention relates to saccharides of general formula (I): 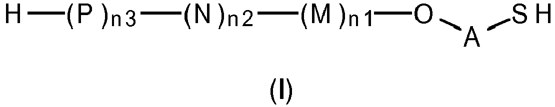 wherein A is a linker;
M, N and P represent independently of each other one of the following sugar fragments: 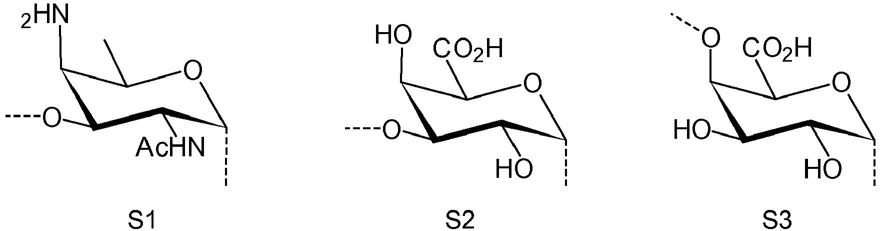 wherein the anomeric position of sugar fragment S1 can be linked only to -O-A-SH or to the sugar fragment S2, the anomeric position of sugar fragment S2 can be linked only to -O-A-SH or to the sugar fragment S3 and the anomeric position of sugar fragment S3 can be linked only to -O-A-SH or to the sugar fragment S1; n1, n2 and n3 are integers selected from 0 and 1, wherein at least one of the integers n1, n2 and n3 is 1, and pharmaceutically acceptable salts of these saccharides.

The inventive compounds of the present invention are saccharides of general formula (I): 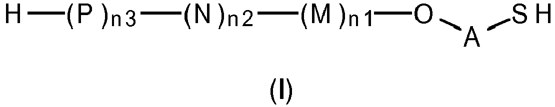 wherein A is a linker;

M, N and P represent independently of each other one of the following sugar fragments: 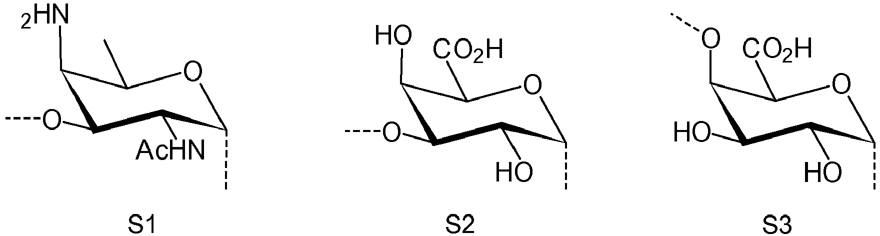 wherein if the anomeric position of sugar fragment S1 is linked to another sugar fragment, then it is linked to sugar fragment S2, and if the anomeric position of sugar fragment S2 is linked to another sugar fragment, then it is linked to the sugar fragment S3, and if the anomeric position of sugar fragment S3 is linked to another sugar fragment, the it is linked to the sugar fragment S1, and
n1, n2 and n3 are integers selected from 0 and 1, wherein at least one of the integers n1, n2 and n3 is 1,
and pharmaceutically acceptable salts of these saccharides.

Thus, included under the scope of the present invention are trissaccharides of general formula: H-(S1)-(S2)-(S3)-O-A-SH, H-(S2)-(S3)-(S1)-O-A-SH, H-(S3)-(S1)-(S2)-O-A-SH; disaccharide of general formula: H-(S1)-(S2)-O-A-SH, H-(S2)-(S3)-O-A-SH, H-(S3)-(S1)-O-A-SH, and monosaccharides of general formula: H-(S1)-O-A-SH, H-(S3)-O-A-SH, wherein A is defined as a linker.

A preferred embodiment of the present application is directed to saccharides of general formula (I) 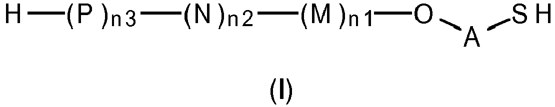 wherein A is a linker defined as above,
P represents S1,
N represents S2,
M represents S3
n1, n2 and n3 are integers selected from 0 and 1
wherein at least one of the integers n1, n2 and n3 is 1 and
and pharmaceutically acceptable salts of these saccharides.

More preferred are saccharides of general formula (I) 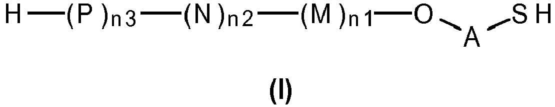 wherein A is a linker defined as above,
P represents S1,
N represents S2,
M represents S3 wherein n1 = n2 = n3 = 1 or wherein n1 = n2 = 1 and n3 = 0 or wherein n1 = 1 and n2 = n3 = 0 or wherein n1 = 0 and n2 = n3 = 1 or wherein n1 = n2 = 0 and n3 = 1.

However, not part of the present invention is a disaccharide wherein the sugar fragment S3 is bound to the linker and is bound to the sugar fragment S1. Consequently, also the corresponding disulfide intermediate is also not part of the present invention. But, included in the scope of the present invention is a disaccharide wherein the sugar fragment S1 is bound to the linker and is also bound to the sugar fragment S3.

In yet another preferred embodiment of the present invention, the compound according to the general formula **(I)** is selected from the group comprising or consisting of:

| |
|---|
| α-2,4,6-trideoxy-4-amino-D-GalNAc-(1→4)-1-(2-thio)ethyl-α-D-GalAp |
| α-D-GalAp-(1→3)-1-(2-thio)ethyl-α-D-GalAp |
| 1-(2-thio)ethyl-α-D-GalAp |
| 1-(2-thio)ethyl-α-2,4,6-trideoxy-4-amino-D-GalNAc |
| α-D-GalAp-(1→3)-α-D-GalAp-(1→3)-1-(2-thio)ethyl-α-2,4,6-trideoxy-4-amino-D-GalNAc |
| α-D-GalAp-(1→3)-1-(2-thio)ethyl-1-(2-thio)ethyl-α-2,4,6-trideoxy-4-amino-D-GalNAc |
| α-D-GalAp-(1→3)-α-2,4,6-trideoxy-4-amino-D-GalNAc-(1→4)-1-(2-thio)ethyl-α-D-GalAp |
| α-2,4,6-trideoxy-4-amino-D-GalNAc-(1→4)-α-D-GalAp-(1→3)-1-(2-thio)ethyl-α-D-GalAp |

### Chemical synthesis

Another aspect of the present invention relates to the synthesis of saccharides of general formula (I): 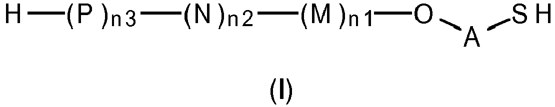 wherein A is a linker defined as above,
M, N and P represent independently of each other one of the following sugar fragments: 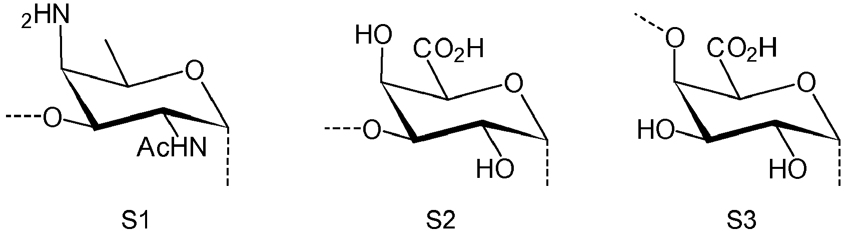 wherein each moiety S1, S2, and S3 is not more than once present in the general formula (I), sugar fragment S1 cannot be simultaneously bound to -O-A-SH and sugar fragment S2, sugar fragment S3 cannot be simultaneously bound to -O-A-SH and sugar fragment S1 and sugar fragment S2 cannot be simultaneously bound to - O-A-SH and sugar fragment S3, and n1, n2 and n3 are integers selected from 0 and 1, wherein at least one of the integers n1, n2 and n3 is 1 and
comprising the steps:
A1) Reacting the compound 2 of the formula: 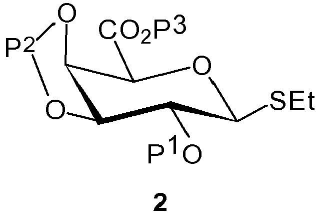 wherein P¹- P³ represent protecting groups, with the compound **3** of the formula 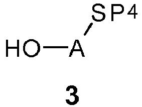 wherein P⁴ represents a protecting group, in order to obtain compound **4** of general formula: 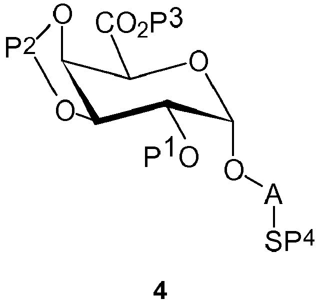 wherein P¹-P⁴ and A are defined as above;
   and
   performing removal of protecting groups P¹-P⁴ on compound **4** to afford monosaccharide disulfide 5 of general formula: 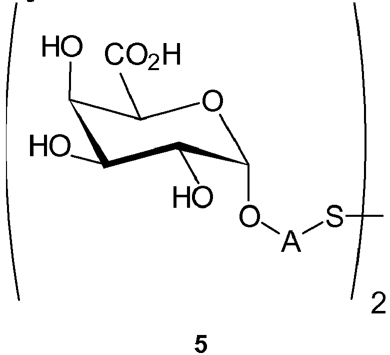 wherein A is defined as above, and wherein monosaccharide disulfide 5 is further treated with a reducing agent to afford monosaccharide 6 of general formula: 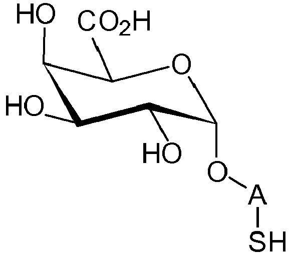 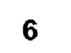 wherein A is defined as above;
   or
   performing selective deprotection on compound **4** to afford compound **7** of general formula 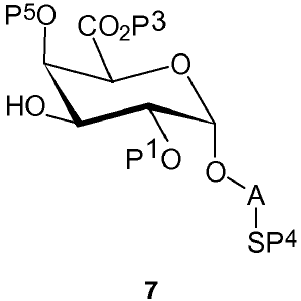 wherein P⁵ is a protecting group and P¹, P³, P4 and A are defined as above.
   or
A2) Reacting compound 8 of general formula 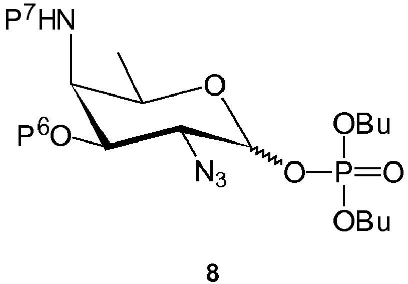 wherein P⁶ and P⁷ represent protecting groups, with compound **3** to afford compound **9** of general formula 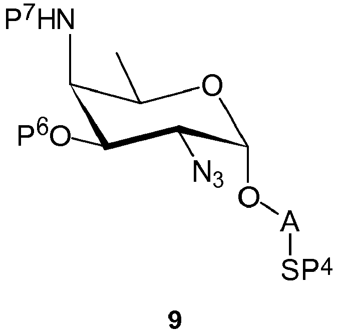 wherein P⁶, P⁷ and A are defined as above;
   and
   performing conversion of the azido group to acetamide and removal of the protecting groups P⁴, P⁶ and P⁷ on compound **9** to afford monosaccharide disulfide **10** of general formula: 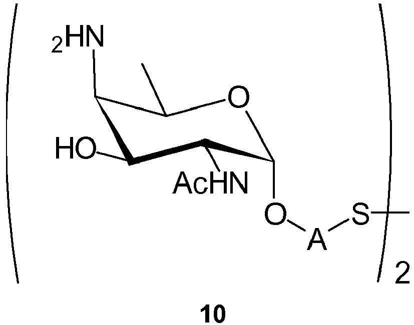 wherein A is defined as above and, wherein monosaccharide disulfide **10** is further treated with a reducing agent to afford monosaccharide **11** of general formula: 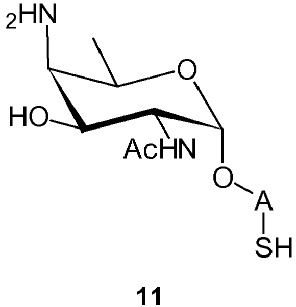 wherein A is defined as above;
   or
   performing selective deprotection on compound **9** to afford compound **12** of general formula: 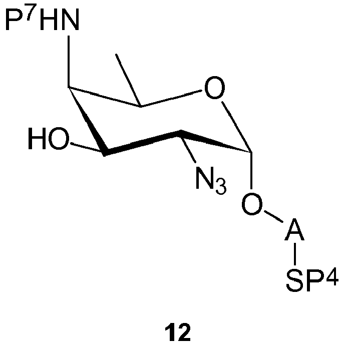 wherein P⁴, P⁷ and A are defined as above.
   or
A3) Reacting compound **13** of general formula 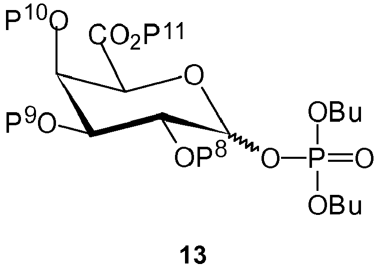 wherein P⁸- P¹¹ represent protecting groups, with compound **3** to afford compound **14** of general formula: 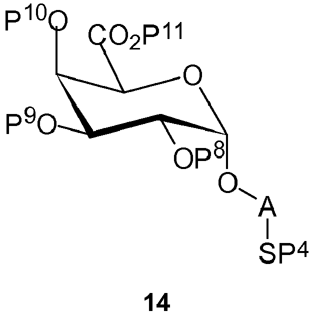 wherein P⁴, P⁸-P¹¹ are defined as above
   and
   performing selective deprotection of compound **14** to afford compound **15** of general formula: 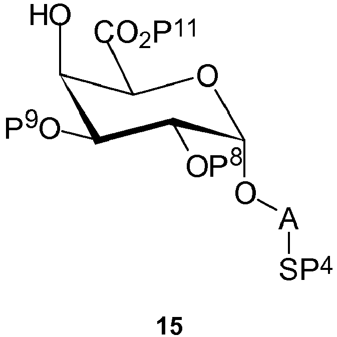 wherein P⁴, P⁸, P⁹, P¹¹ and A are defined as above.
   and
B1) Reacting compound **7** with compound **13** to afford compound **16** of general formula: 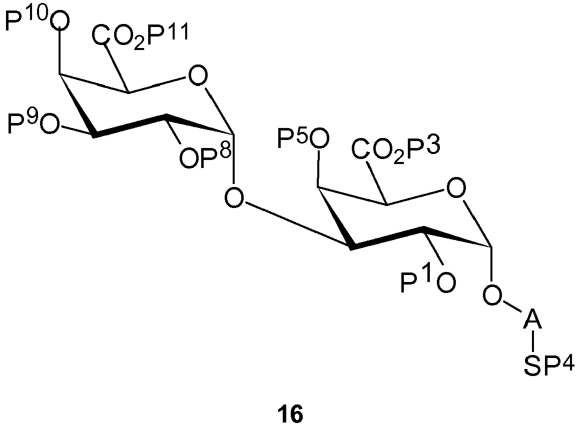 wherein P¹, P³-P⁵, P⁸-P¹¹ and A are defined as above;
   and
   performing removal of protecting groups P¹, P³-P⁵, P⁸-P¹¹ on compound **16** to afford disaccharide disulfide **17** of general formula: 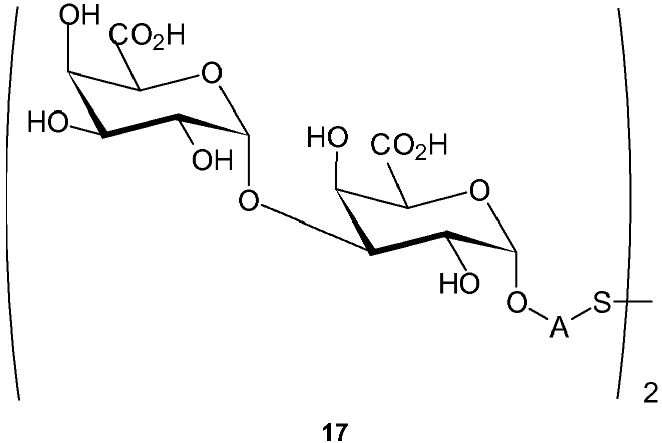 wherein A is defined as above and wherein disaccharide disulfide **17** is further treated with a reducing agent to afford disaccharide **18** of general formula: 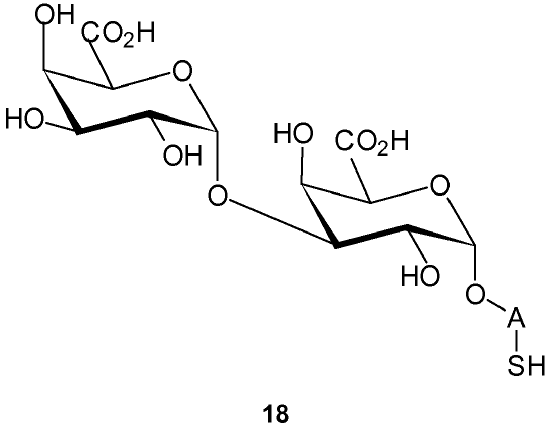 wherein A is defined as above;
   or
   performing selective removal of protecting group P¹⁰ on compound **16** to afford compound **19** of general formula: 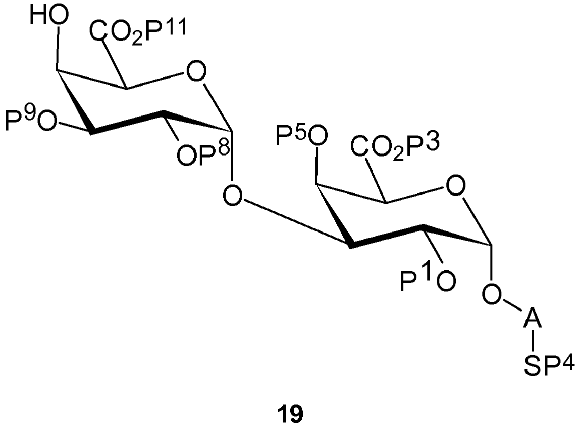 wherein P¹, P³-P⁵, P⁹, P⁹, P¹¹ and A are defined as above.
   or
B2) Reacting compound **15** with compound **8** to afford compound **20** of general formula: 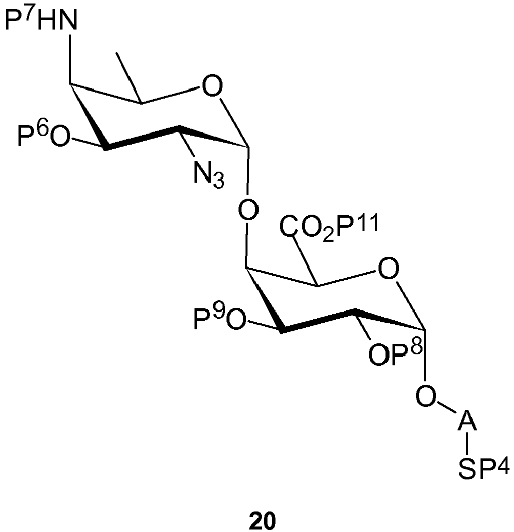 wherein P⁴, P⁶-P⁹, P¹¹ and A are defined as above;
   and
   performing conversion of the azido group to acetamide and removal of the protecting groups P⁴, P⁶-P⁹, P¹¹ on compound **20** to afford disaccharide disulfide **21** of general formula: 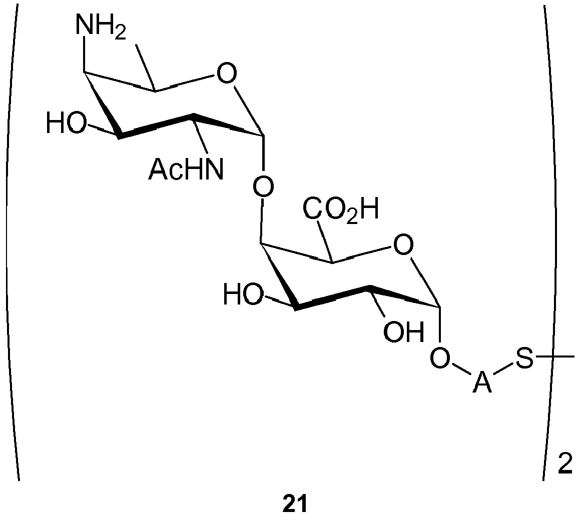 wherein A is defined as above and wherein disaccharide disulfide **21** is treated with a reducing agent to afford disaccharide **22** of general formula: 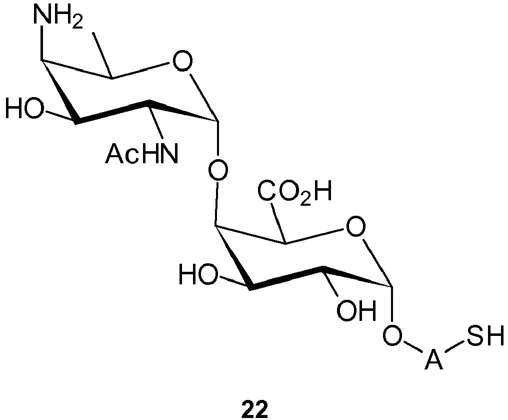 wherein A is defined as above;
   or
   performing selective removal of protecting group P⁶ on compound **20** to afford compound **23** of general formula: 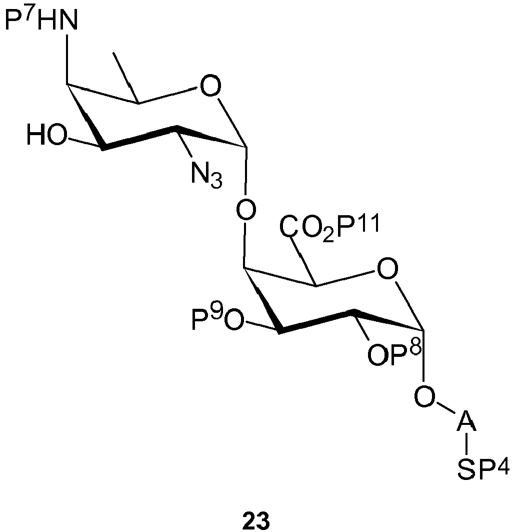 wherein P⁴, P⁷-P⁹, P¹¹ and A are defined as above;
   or
B3) Reacting compound **12** with compound **2** to afford compound **24** of general formula: 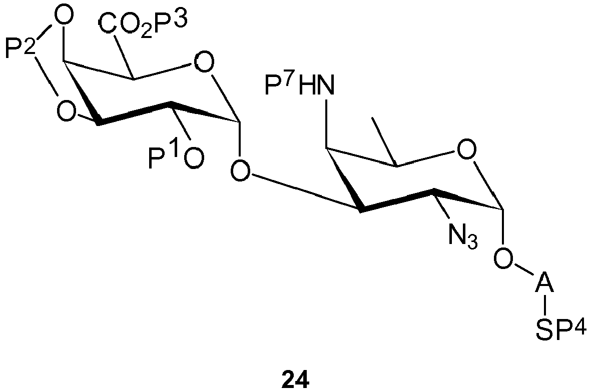 wherein P¹-P⁴, P⁷ and A are defined as above;
   and
   performing conversion of the azido group to acetamide and removal of protecting groups P¹-P⁴ and P⁷ on compound **24** to afford disaccharide disulfide **25** of general formula: 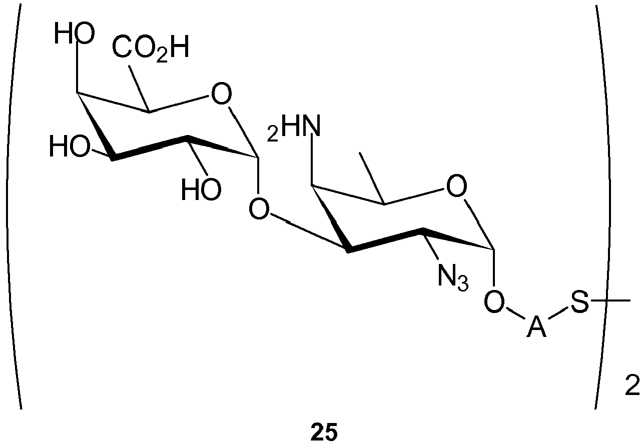 wherein A is defined as above, and wherein disaccharide disulfide **25** is further treated with a reducing agent to afford disaccharide **26** of general formula: 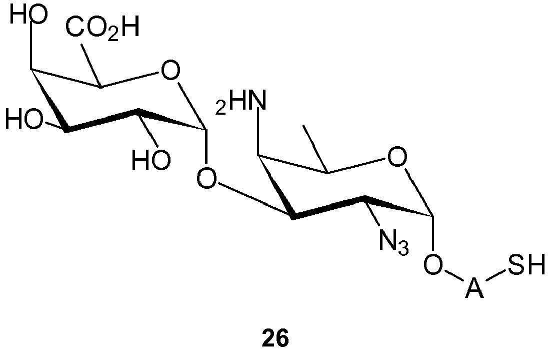 wherein A is defined as above;
   or
   performing selective deprotection on compound **24** to afford compound **27** of general formula: 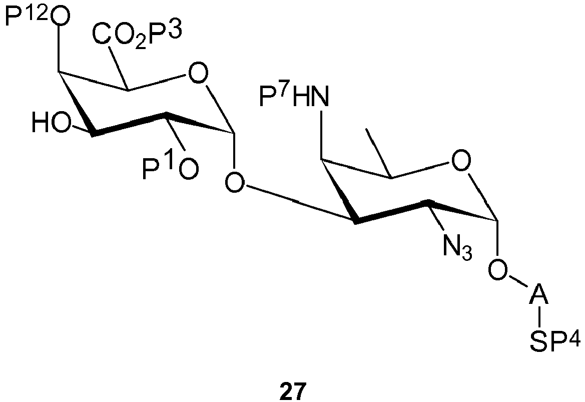 wherein P¹² is a protecting group and P¹, P³, P⁴, P⁷ and A are defined as above.
   and
C1) Reacting compound **19** with compound **8** to afford compound **28** of general formula: 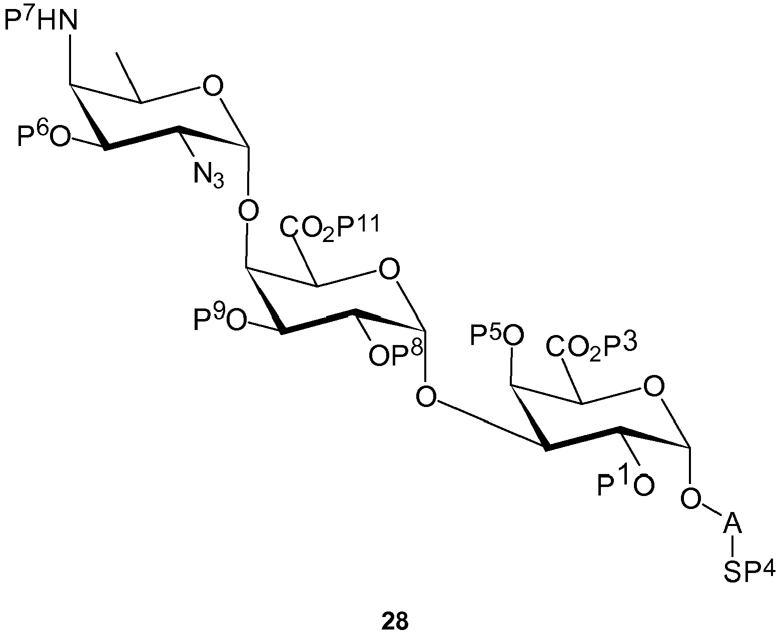 wherein P¹, P³-P⁹, P¹¹ and A are defined as above;
   and
   wherein protecting group P⁶ is replaced with protecting group P¹³ in order to obtain compound **29** of the following chemical formula: 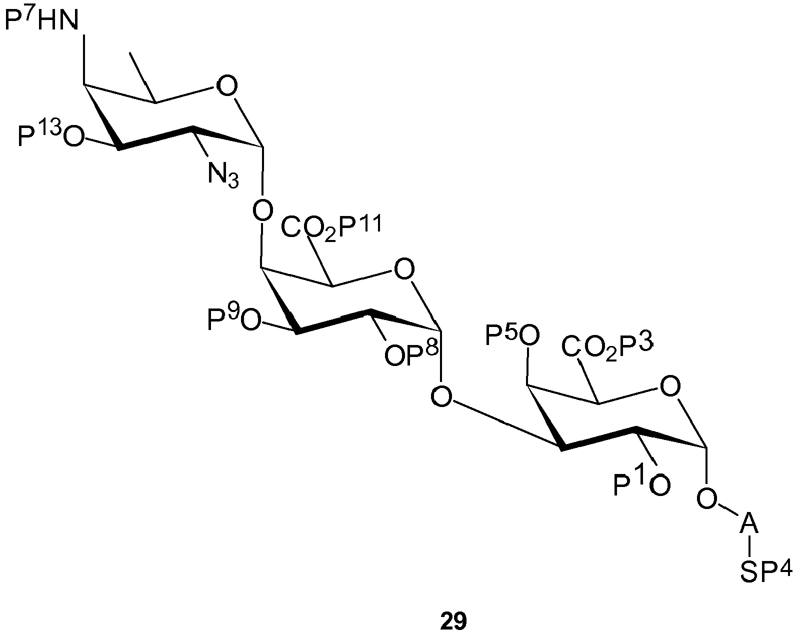 wherein P¹, P³-P⁵, P⁷-P⁹, P¹¹, P¹³ and A are defined as above;
   and
   conversion of compound **29** to trisaccharide disulfide **30** by conversion of the azide group in the acetamide group and cleavage of the protecting group P¹, P³-P⁵, P⁷-P⁹, P¹¹, P¹³, wherein compound **30** is of general formula: 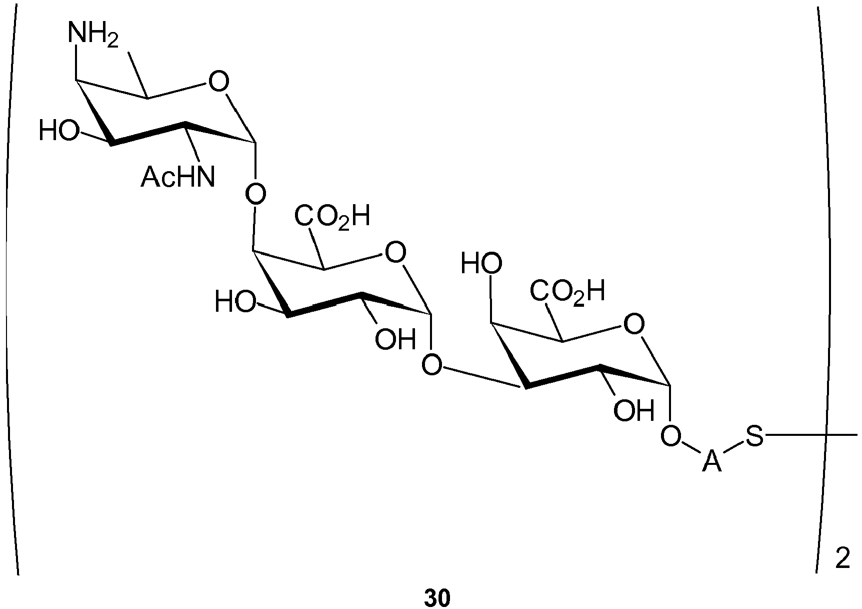 and wherein A is defined as above;
   and
   conversion of trisaccharide disulfide **30** to trisaccharide **31** by treatment with a reducing agent, wherein compound **31** is of general formula: 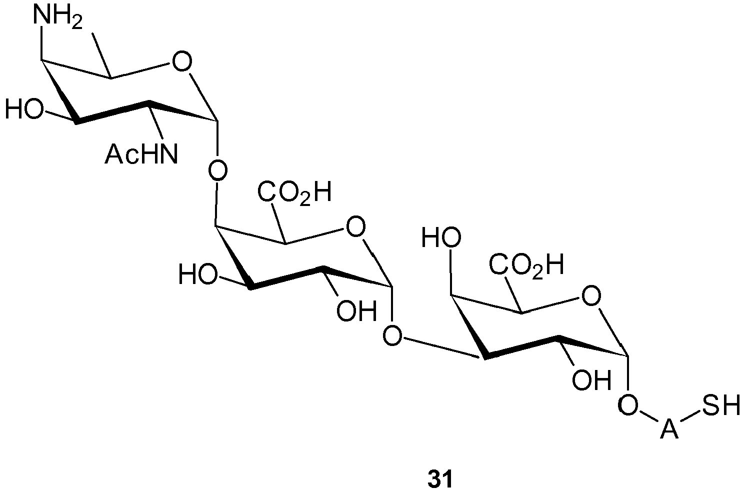 and wherein A is defined as above.
   or
C2) Reacting compound **23** with compound **2** to afford compound **32** of general formula: 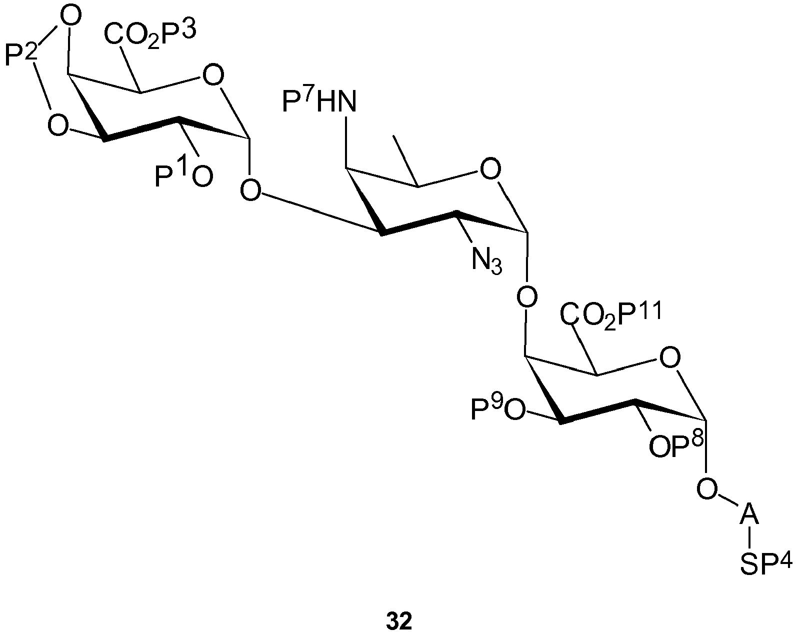 wherein P¹ - P⁴, P⁷- P⁹, P¹¹ and A are defined as above;
   and
   conversion of compound **32** to trisaccharide disulfide **33** by conversion of the azide group in the acetamide group and cleavage of the protecting group P¹ - P⁴, P⁷ - P⁹, P¹¹, wherein compound **33** is of general formula: 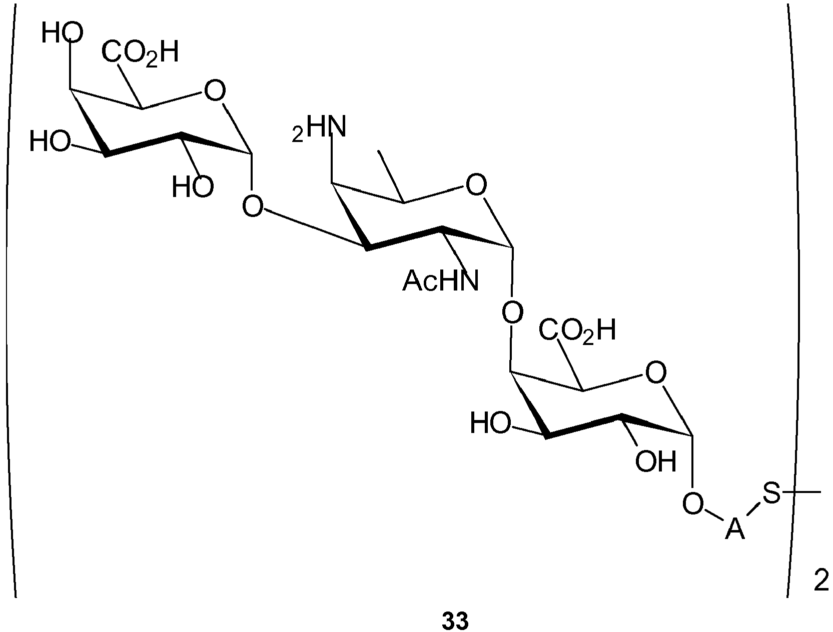 wherein A is defined as above;
   and
   conversion of trisaccharide disulfide **33** to trisaccharide **34** by treatment with a reducing agent, wherein compound **34** is of general formula: 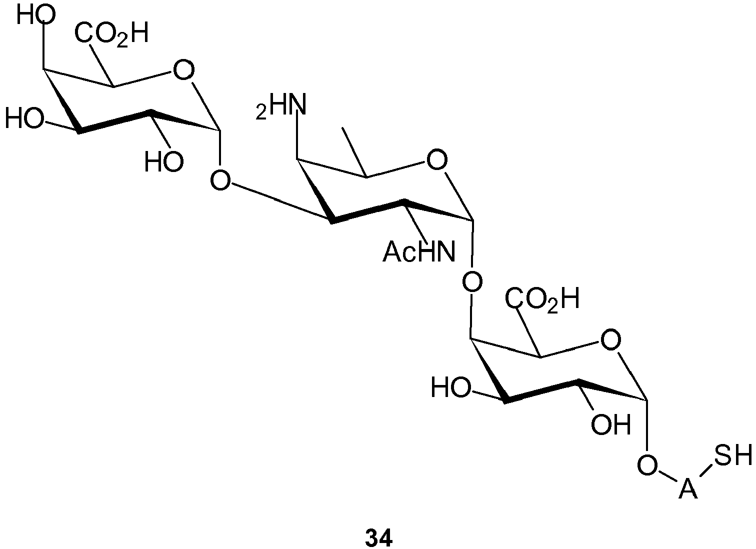 wherein A is defined as above;
   or
C3) Reacting compound **27** with compound **13** to afford compound **34** of general formula: 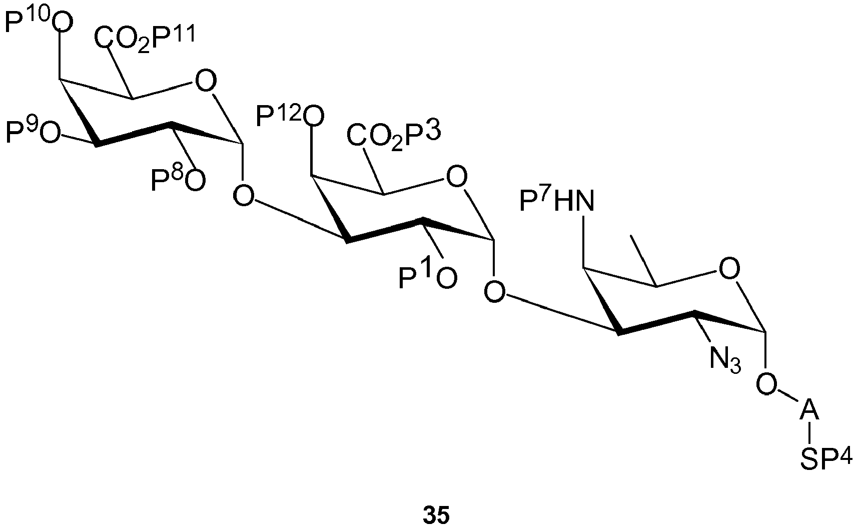 wherein P¹, P³, P⁴, P⁷-P¹¹ and A are defined as above;
   and
   conversion of compound **35** to trisaccharide disulfide **36** by conversion of the azide group in the acetamide group and cleavage of the protecting group P¹, P³, P⁴, P⁷-P¹¹, wherein compound **36** is of general formula: 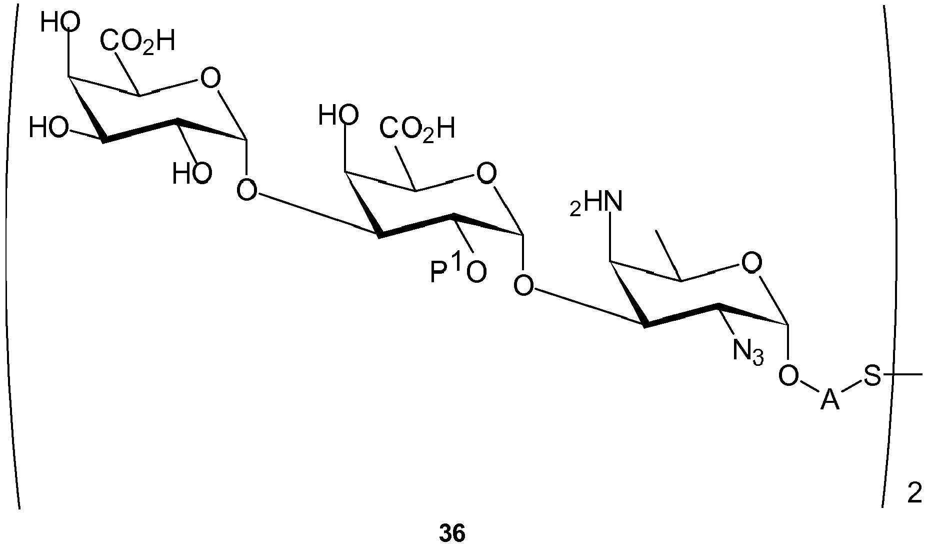 wherein A is defined as above;
   and
   conversion of trisaccharide disulfide **36** to trisaccharide **37** by treatment with a reducing agent, wherein compound **37** is of general formula: 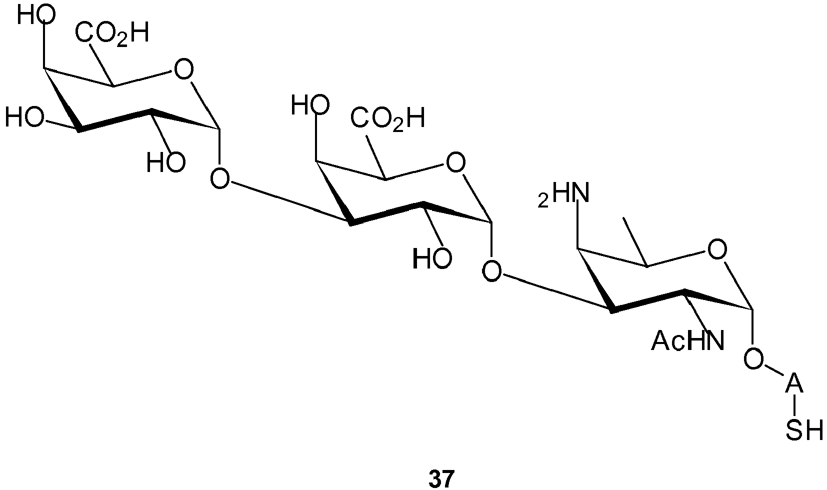 wherein A is defined as above.

The term "protecting group" as used herein refers to commonly used groups in organic synthesis, preferably used for protection of amines, hydroxyl groups, thiols, imines, carbonyls, carboxyls or other common functional groups, and particularly preferred for amines, hydroxyl groups, thiols and carboxyls.

More specifically, P¹, P², P⁵, P⁶, P⁸-P¹⁰, P¹² and P¹³ preferably are suitable protecting groups for hydroxyl groups, more preferably different suitable protecting groups for hydroxyl groups capable of being removed subsequently one after another by a suitable sequence of deprotection reactions. Therefore protecting groups for hydroxyl groups, namely P¹, P², P⁵, P⁶, P⁸-P¹⁰, P¹² and P¹³ may be selected from the group consisting of or comprising: acetyl, benzyl, isopropylidene, benzylidene, benzoyl, *p-*methoxybenzyl, *p*-methoxybenzylidene, *p*-methoxyphenyl, *p*-bromobenzyl, *p-*bromobenzyledene, *p*-nitrophenyl, allyl, acetyl, isopropyl, *p*-bromobenzyl dimethoxytrityl, trityl, 2-naphthylmethyl, pyvaloyl, triisopropylsilyl, *tert-*butyldimethylsilyl, *tert*-butyldiphenylsilyl, *tert*-butylmethoxphenylsilyl, triethylsilyl, trimethylsilyl, 2-trimethylsilylethoxymethyl, 9-fluorenylmethoxycarbonyl, benzyloxymethyl, methyloxymethyl, *tert*-butyloxymethyl, methoxyethyloxymethyl.

More specifically, in a preferred embodiment of the present invention protecting groups P¹, P⁵, P⁹, P⁹ and P¹² may be benzyl, P² may be benzylidene, P⁶ may be levulinyl, P¹⁰ may be 9-fluorenylmethoxycarbonyl and P¹³ may be benzyloxymethyl.

Amines are generally protected as carbamates. Therefore, protecting group P⁷ may be selected from the group consisting of or comprising *tert*-butyloxy carbonyl, 9-fluorenylmethoxy carbonyl, allyloxy carbonyl, 2,2,2-trichloroethyloxy carbonyl, benzyloxy carbonyl; carbonyls such as trifluoro acetyl, trichloro acetyl, acetyl, benzoyl. More specifically, in a preferred embodiment of the present invention P⁷ may be benzyloxy carbonyl.

Carboxylic acids are generally protected as esters. Therefore protecting groups P³ and P¹¹ may be selected from the group consisting of or comprising methyl, ethyl, allyl, isopropyl, *tert*-butyl, phenyl, benzyl, *p*-methoxybenzyl. More specifically, in a preferred embodiment of the present invention protecting groups P³ and P¹¹ may be methyl.

The protecting groups employed in the synthesis of saccharides of general formula (I) can be differentiated in permanent protecting groups and temporary protecting groups. Permanent protecting groups are protecting groups that are stable during the entire synthesis and that can be efficiently removed at the late stage of the synthesis. Such permanent groups include, but they are not restricted to benzyl, benzylidene, benzoate, acetate, alkyl esters. The temporary protecting groups are generally orthogonal protecting groups that can be selectively removed at different levels of the synthesis to free hydroxyl groups for subsequent introduction of different substituents, including monosaccharides or other protecting groups. The ingenious choice of protecting groups allows expedient access to a library of saccharides of general formula (I) functionalized with a linker presenting a terminal thiol group for subsequent conjugation to a carrier immunogen or a solid support.

A preferred embodiment of the present invention is directed to the synthesis of compounds of general formula (I) 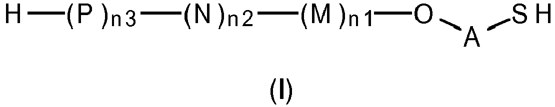 wherein A is a linker defined as above,
P represents S1,
N represents S2,
M represents S3
n1, n2 and n3 are integers selected from 0 and 1
wherein at least one of the integers n1, n2 and n3 is 1 and
comprising the steps:
A1) Reacting the compound 2 of the formula: 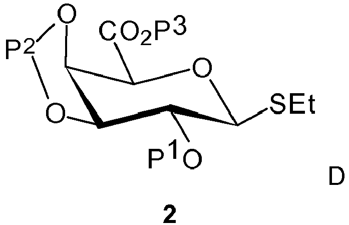 wherein P¹-P³ represent protecting groups, with the compound **3** of the formula 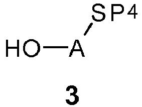 wherein P⁴ represents a protecting group, in order to obtain compound **4** of general formula: 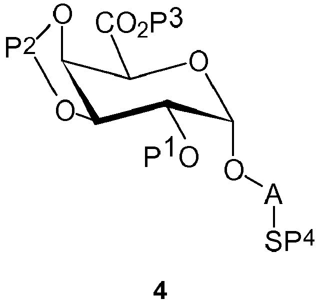 wherein P¹-P⁴ and A are defined as above;
   and
   performing removal of protecting groups P¹-P⁴ on compound **4** to afford monosaccharide disulfide 5 of general formula: 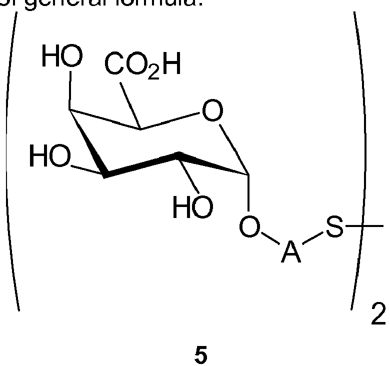 wherein A is defined as above, and wherein monosaccharide disulfide **5** is further treated with a reducing agent to afford monosaccharide **6** of general formula: 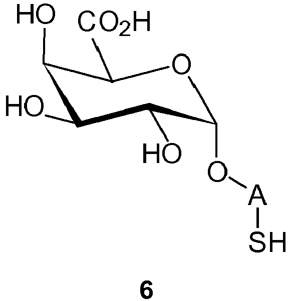 wherein A is defined as above;
   or
   performing selective deprotection on compound 4 to afford compound 7 of general formula 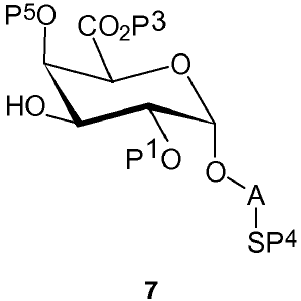 wherein P⁵ is a protecting group and P¹, P³, P4 and A are defined as above.
   or
   A2) Reacting compound **8** of general formula 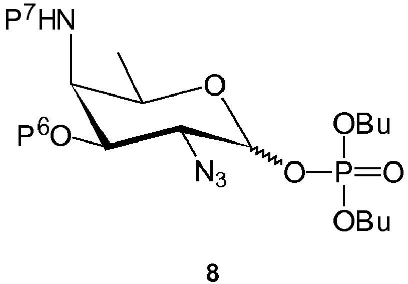 wherein P⁶ and P⁷ represent protecting groups, with compound **3** to afford compound **9** of general formula 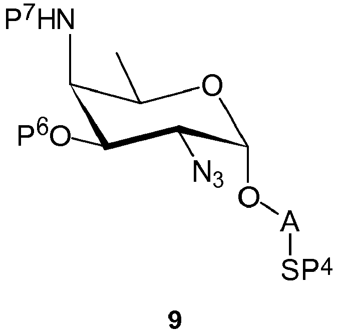 wherein P⁶, P⁷ and A are defined as above;
   and
   performing conversion of the azido group to acetamide and removal of the protecting groups P⁴, P⁶ and P⁷ on compound **9** to afford monosaccharide disulfide **10** of general formula: 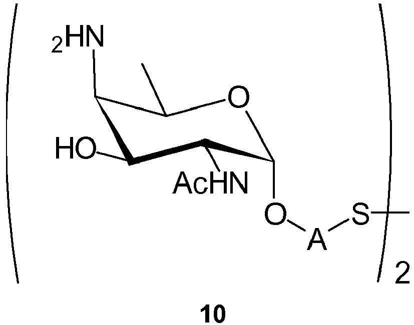 wherein A is defined as above and, wherein monosaccharide disulfide **10** is further treated with a reducing agent to afford monosaccharide **11** of general formula: 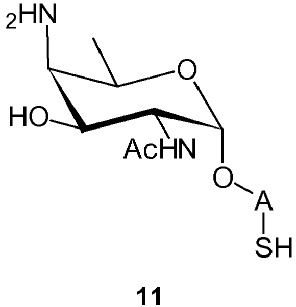 wherein A is defined as above.
   or
A3) Reacting compound **13** of general formula 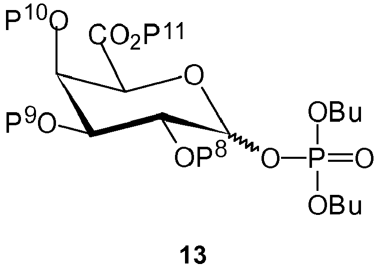 wherein P⁸-P¹¹ represent protecting groups, with compound 3 to afford compound **14** of general formula: 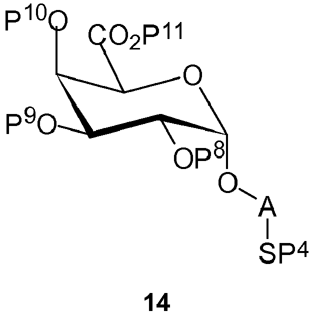 wherein P⁴, P⁸-P¹¹ are defined as above
   and
   performing selective deprotection of compound **14** to afford compound **15** of general formula: 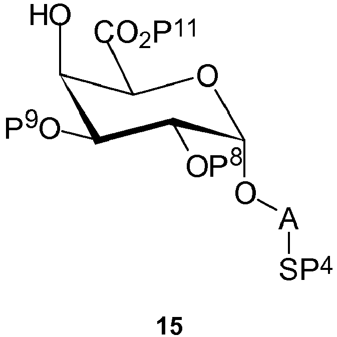 wherein P⁴, P⁹, P⁹, P¹¹ and A are defined as above.
   and
B1) Reacting compound **7** with compound **13** to afford compound **16** of general formula: 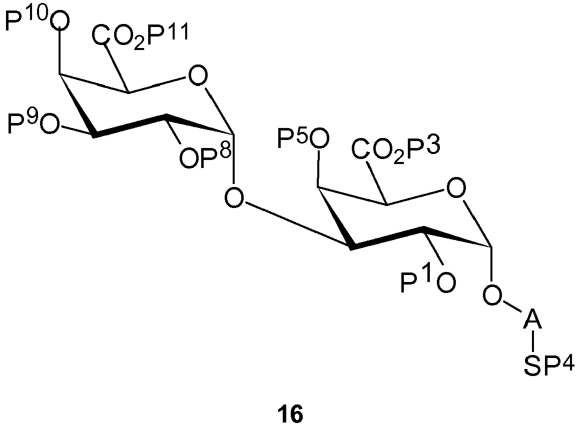 wherein P¹, P³-P⁵, P⁸-P¹¹ and A are defined as above;
   and
   performing removal of protecting groups P¹, P³-P⁵, P⁸-P¹¹ on compound **16** to afford disaccharide disulfide **17** of general formula: 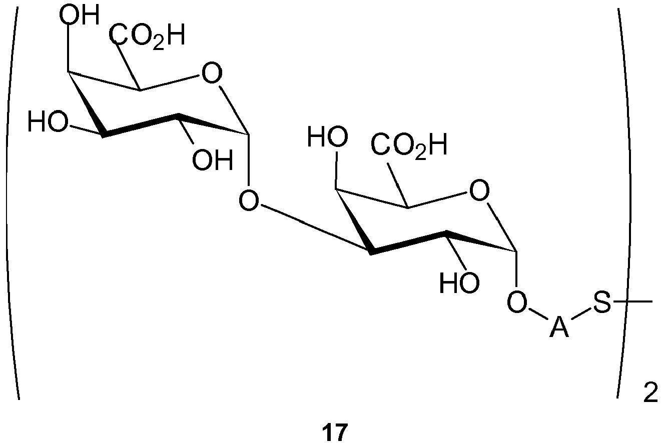 wherein A is defined as above and wherein disaccharide disulfide **17** is further treated with a reducing agent to afford disaccharide **18** of general formula: 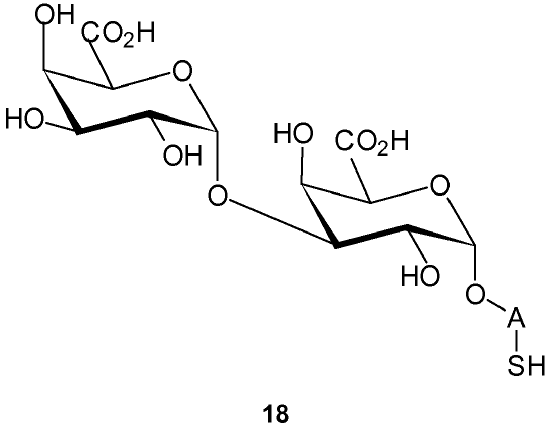 wherein A is defined as above;
   or
   performing selective removal of protecting group P¹⁰ on compound **16** to afford compound **19** of general formula: 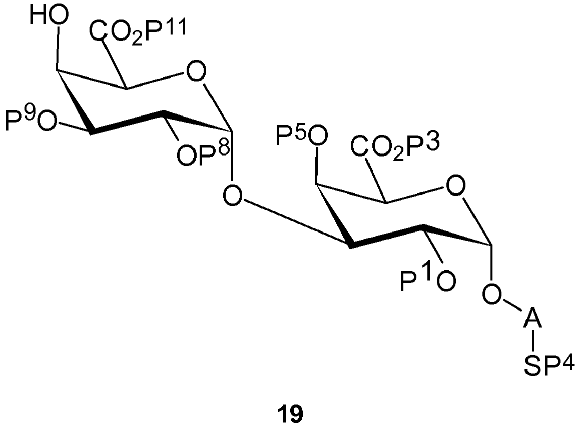 wherein P¹, P³-P⁵, P⁹, P⁹, P¹¹ and A are defined as above.
   or
B2) Reacting compound **15** with compound **8** to afford compound **20** of general formula: 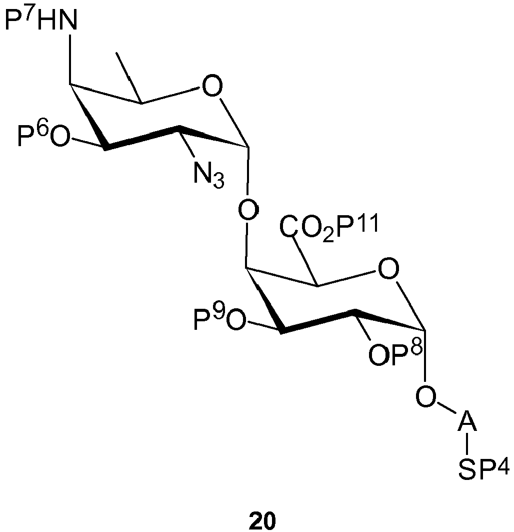 wherein P⁴, P⁶-P⁹, P¹¹ and A are defined as above;
   and
   performing conversion of the azido group to acetamide and removal of the protecting groups P⁴, P⁶-P⁹, P¹¹ on compound **20** to afford disaccharide disulfide **21** of general formula: 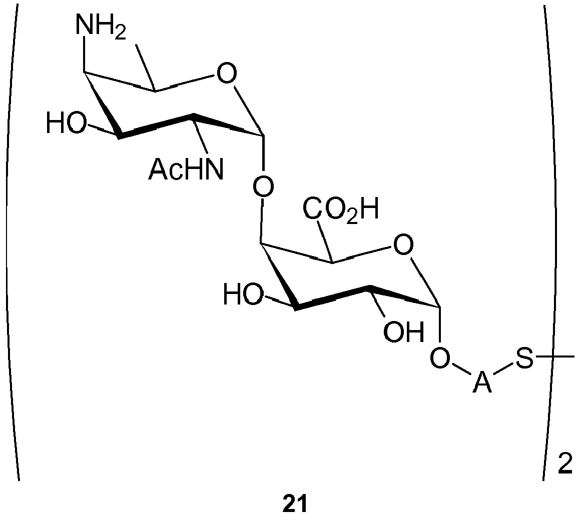 wherein A is defined as above and wherein disaccharide disulfide **21** is treated with a reducing agent to afford disaccharide **22** of general formula: 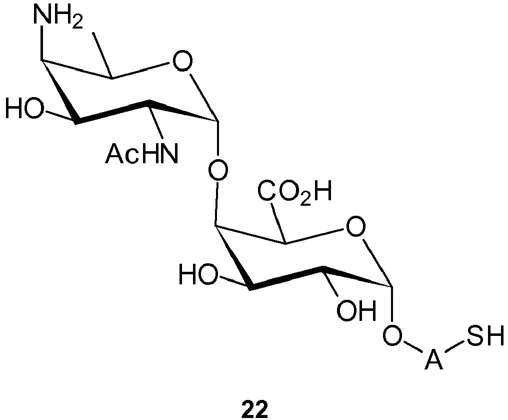 wherein A is defined as above.
   and
C1) Reacting compound **19** with compound **8** to afford compound **28** of general formula: 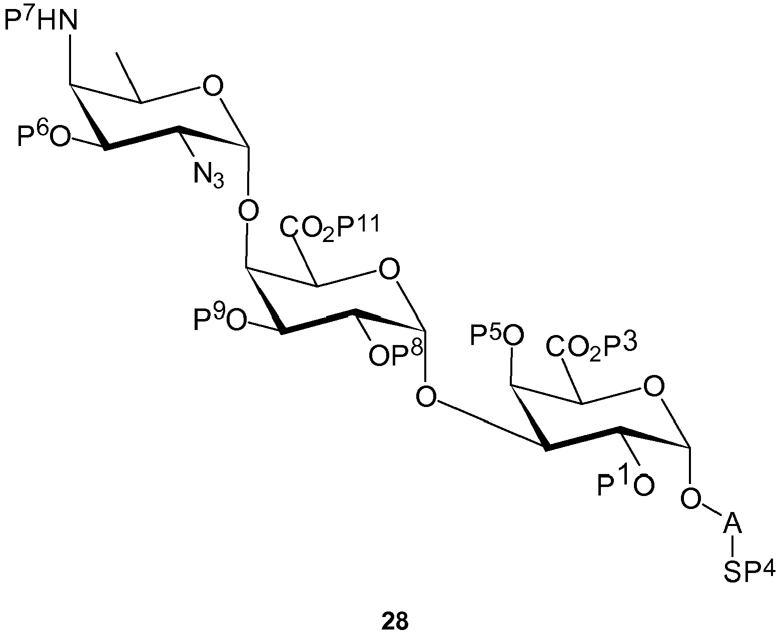 wherein P¹, P³-P⁹, P¹¹ and A are defined as above;
   and
   wherein protecting group P⁶ is replaced with protecting group P¹³ in order to obtain compound 29 of the following chemical formula: 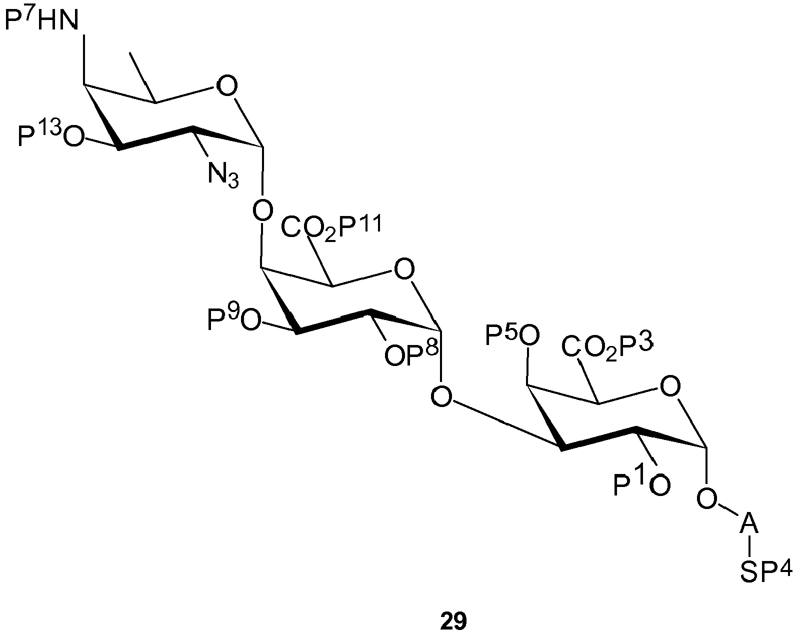 wherein P¹, P³-P⁵, P⁷-P⁹, P¹¹, P¹³ and A are defined as above;
   and
   conversion of compound **29** to trisaccharide disulfide **30** by conversion of the azide group in the acetamide group and cleavage of the protecting group P¹, P³-P⁵, P⁷-P⁹, P¹¹, P¹³, wherein compound **30** is of general formula: 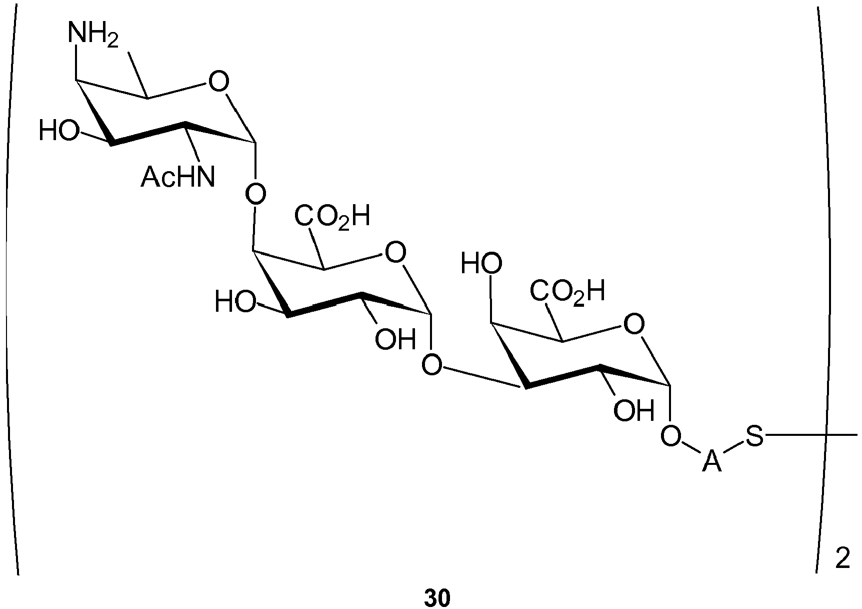 and wherein A is defined as above;
   and
   conversion of trisaccharide disulfide **30** to trisaccharide **31** by treatment with a reducing agent, wherein compound **31** is of general formula: 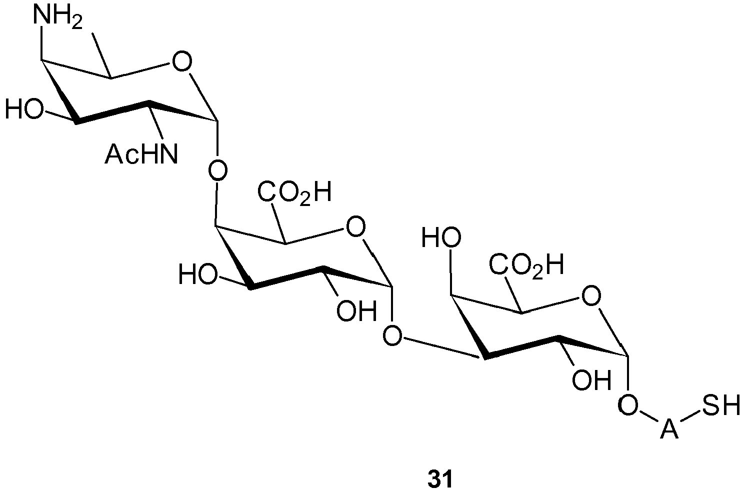 and wherein A is defined as above.

The conversion of the saccharide disulfides **36, 33, 30, 25, 21, 17, 10** and **5** to the saccharides **37, 34, 31, 26, 22, 18, 11,** and **6** respectively is performed in presence of a reducing agent. Known reducing agent for the person skilled in the art include, but they are not restricted to: mercaptoethanol, ditriotheritol, tris(2-carboxyethyl)phosphine, magnesium/methanol, sodium/ammonia followed by ammonium chloride/chlorhydric acid. Preferably the conversion of the saccharide disulfides to the corresponding saccharides is carried out with tris(2-carboxyethyl)phosphine.

It is preferred that the reaction between compounds **2** and **3,** compounds **2** and **12,** compounds **5*** and **11*,** compounds **19*** and **21*,** compounds **2** and **23** is performed in an aprotic solvent in presence of (dimethylthio)methylsulfonium trifluoromethanesulfonate (DMTST) and TTBPy. In addition activated molecular sieve (MS) such as 3A molecular sieve, 4A molecular sieves or 3A acid washed molecular sieves can be used. The reaction temperature is between -20 °C and room temperature, preferably the temperature is between -10 °C and room temperature, more preferably the temperature is between -5 °C and room temperature and most preferably the temperature is between 0 °C and room temperature. The reaction is preferably carried out in an aprotic solvent such as acetonitrile, ether such as tetrahydrofurane, diethylether or dioxane, halogenated solvents such as chloroform, methylene chloride and toluene.

It is also preferred that the reaction of compounds **13** and 7, compounds **8** and **3,** compounds **12*** and **9*,** compounds **13*** and **2*,** compounds **2*** and **21*,** compounds **2*** and **11* ,** compounds **19** and **8,** and compounds **8** and **15,** is performed in an apolar aprotic solvent in presence of silyl triflate. Examples of silyl triflate include, but are not restricted to trimethylsilyl trifluoromethanesulfonate, *tert*-butyl dimethyl trifluoromethanesulfonate, triiospropyl trifluoromtahnesulfonate. Suitable apolar aprotic solvents include diethylether, chloroform, methylene chloride and toluene. The reaction temperature is between -20 °C and +20 °C, preferably the temperature is between -10 °C and +10 °C and more preferably the temperature is between -5 °C and +5 °C and most preferably about 0 °C. Preferably, activated molecular sieve (MS) such as 3A molecular sieve, 4A molecular sieves or 3A acid washed molecular sieves can be used.

Preferably the replacement of protecting group P⁶ on compound **28** with protecting group P¹³ to obtain compound **29** is performed in two steps, first involving first involving the reaction of compound **28** with hydrazine or a hydrazinium salt in a mixture of polar and apolar solvents and second by treatment of the product obtained after the first step with BnOCH₂SCy, DMTST and TTBPy in an apolar solvent. For the first step, hydrazinium salts of weak acids are preferred such as hydrazinium acetate or hydrazinium proprionate. The second step is preferably conducted in presence of activated molecular sieves as the one mentioned above at a temperature preferably between -10 °C and 20 °C and most preferably between 0 °C and 10 °C. Suitable apolar solvents are mentioned above. It was found that the replacement of protecting group P⁶ with protecting group P¹³ is essential for avoiding side-reactions during the cleavage of the permanent protecting group.

The conversion of compound **4** to **5, 16** to **17, 24** to **25, 13*** to **27*** and **20*** to **22*** requires cleavage of the protecting group, and more specifically of the permanent protecting groups. The cleavage of said protecting groups involves first cleavage of the base-labile protecting groups by treatment with a base in a mixture of polar aprotic solvent and a polar protic solvent; and second cleavage of the protecting groups sensitive to hydrogenation by exposure to sodium and ammonia in a mixture of polar protic solvents. For the first step suitable aprotic solvents include tetrahydrofuran, ethyl acetate, acetone, *N,N*-dimethylformamide, acetonitrile and *N,N-*dimethylsulfoxide, which are mixed with a suitable protic solvent such as water and alcohols including methanol, ethanol, propanol, isopropanol or *tert*-buthanol. The basic cleavage of the protecting groups is preferably performed at room temperature comprised between 0 °C and room temperature. Appropriate base for performing first step include lithium hydroxide, sodium hydroxide and potassium hydroxide. The cleavage of the protecting groups sensitive to hydrogenation is conducted by exposure to sodium and ammonia in a mixture of polar protic solvents such as alcohols at a temperature comprised between -78 °C and room temperature. Optionally, lithium can be used as equivalent of sodium during the cleavage of the protecting groups sensitive to hydrogenation.

Previous to the above mentioned cleavage of permanent protecting groups, the conversion of compounds **9** to **10, 20** to **21, 29** to **30, 32** to **33, 35** to **36, 25*** to **26*, 23*** to **24*,** and **17*** to **18*** involves conversion of the azido group in the acetamide group, which is preferably performed in presence of thioacetic acid and pyridine. An alternative method is to conduct conversion of the azido group in the acetamide group in two steps: first chemoselective reduction of the azido group, and then acetylation. The chemoselective reduction can be carried out using by hydrogenolysis on Pd/C in presence of ammonia, ammonium acetate, triphenylphosphine or pyridine. The acetylation can be accomplished using acetyl chloride or acetic anhydride in presence of a base.

The saccharides **I, 5, 6, 10, 11, 17, 21, 25, 30, 33, 33, 36, 37, 27*, 26*, 25*, 24*, 22*, 20*** and **18*** bear basic and/or acidic substituents and they may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, *p*-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, *p*-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (*o*, *m, p*)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

The free base forms may be regenerated by treating the salt with a suitable dilute aqueous base solution such as dilute aqueous sodium hydroxide, potassium carbonate, ammonia and sodium bicarbonate. The free base forms differ from their corresponding salt forms somewhat in certain physical properties, such as solubility in polar solvents, but the salts are otherwise equivalent to their corresponding free base forms for purposes of this invention.

Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula **(I)** with a solution of an acid, selected out of the group mentioned above.

Further, it is also possible that the compounds of the present invention bear simultaneously basic and acid groups. Further, it may also occur that these basic and acid groups appear to be in close vicinity to one another enabling an intramolecular proton transfer from the acidic group the basic group. Therefore, in a preferred embodiment of the present invention the compound of the formula **(I)** may be zwitterionic, bearing at least e.g. one -O- and one -NH₃⁺ group.

This invention includes within its scope stoechiometric solvates including hydrates as well as compounds containing variable amounts of water that may be produced by processes such as lyophilisation.

Thus, the synthesis of the saccharides of the general formula **(I)** may further comprise step D:
D) preparing a salt of the compound of general formula **(I)** or preparing a lyophilisate of the compound of general formula **(I)** or of the salt of the compound of general formula **(I).**

In another preferred embodiment, the synthesis of intermediates of general formula **(II)** may further comprise step D:
D) preparing a salt of the compound of general formula **(II)** or preparing a lyophilisate of the compound of general formula **(II)** or of the salt of the compound of general formula **(II).**

In a preferred embodiment, the synthesis of saccharides of the general formula **(I)** 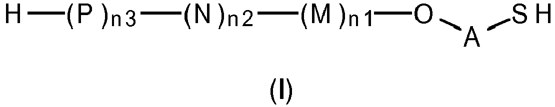 wherein A is a linker defined as above,
P represents S1,
N represents S2,
M represents S3
n1, n2 and n3 are integers selected from 0 and 1
wherein at least one of the integers n1, n2 and n3 is 1 and
may further comprise step D)
D) preparing a salt of the compound of general formula **(I)** or preparing a lyophilisate of the compound of general formula **(I)** or of the salt of the compound of general formula **(I).**

### Intermediates

Another aspect of the present invention is directed to intermediates of general formula **(II):** 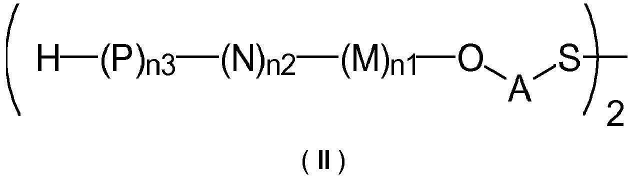 wherein A is a linker;
M, N and P represent independently of each other one of the following fragments: 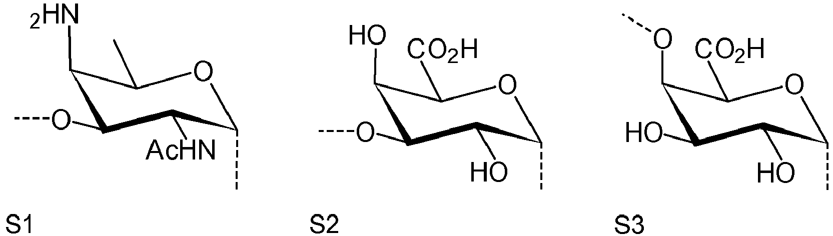 wherein each moiety S1, S2, and S3 is not more than once present in the general formula **(II),** sugar fragment S1 cannot be simultaneously bound to -O-A-S- and sugar fragment S2, sugar fragment S3 cannot be simultaneously bound to -O-A-Sand sugar fragment S1 and sugar fragment S2 cannot be simultaneously bound to -O-A-S- and sugar fragment S3, and
n1, n2 and n3 are integers selected from 0 and 1
wherein at least one of the integers n1, n2 and n3 is 1 and
and pharmaceutically acceptable salts of these saccharides.

Preferred are intermediates of general formula **(II)** 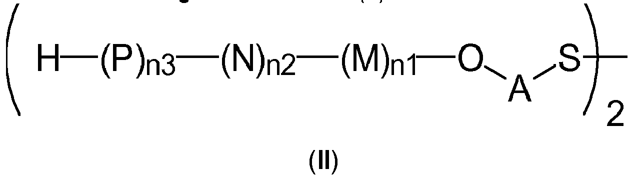 wherein A is a linker defined as above,
P represents S1,
N represents S2,
M represents S3
n1, n2 and n3 are integers selected from 0 and 1
wherein at least one of the integers n1, n2 and n3 is 1
and pharmaceutically acceptable salts of these saccharides.

More preferred are intermediates of general formula (**II**) 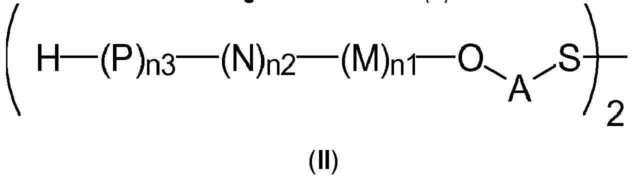 wherein A is a linker defined as above,
P represents S1,
N represents S2,
M represents S3
wherein n1 = n2 = n3 = 1 or wherein n1 = n2 = 1 and n3 = 0 or wherein n1 = 1 and n2 = n3 = 0 or wherein n1 = 0 and n2 = n3 = 1 or wherein n1 = n2 = 0 and n3 = 1.

### Glycoconjugates

Another aspect of the present invention refers to glycoconjugate obtained by reacting the saccharides of general formula (I) with an immunogenic carrier. Said glycoconjugates proved to be efficient as a vaccine for immunization against diseases associated with bacteria.

Saccharides are known by the person skilled in the art as generally TI-2 (T cell independent-2) antigens and poor immunogens. TI-2 antigens are antigens, which are recognized only by mature B cells through the cross linking of surface exposed immunoglobulin receptors. Without T cell help, no immunological memory is generated and neither isotype switching from IgM to other IgG subclasses, nor B cells affinity maturation occurs. Moreover, saccharides are known poor immunogens in humans due to the structural homology to human glycolipids and glycoproteins. Due to their poor immunogenic properties, saccharides manifest poor ability to produce both antibody production by B cells, as well as the formation of memory cells, features which are essential for the production of potent vaccines.

Therefore, to produce a potent saccharide-based vaccine, the saccharides of general formula (I) are conjugated to an immunogenic carrier to provide glycoconjugates, which present increased immunogenicity in comparison with the saccharide.

In this context the term "immunogenic carrier" is defined as a structure, which is conjugated to the saccharide to form a glycoconjugate that presents an increased immunity in comparison with the saccharide *per se.* Thus, the conjugation of the saccharides of general formula (**I**) to the immunogenic carrier has as effect the stimulation of the immune response against the saccharide of general formula (I), without inducing an immune response against the said immunogenic carrier.

Preferred immunogenic carriers are carrier proteins or glycosphingolipid with immunomodulatory properties. For the person skilled in the art, a carrier protein is a protein selected from the group comprising or consisting of: a diphtheria toxoid, a mutated diphtheria toxoid, a modified diphtheria toxoid, a mutated and modified diphtheria toxoid, a tetanus toxoid, a modified tetanus toxoid, a mutated tetanus toxoid, outer membrane protein (OMP), bovine serum albumin (BSA), keyhole limpet hemocyanine (KLH) or cholera toxoid (CT). The term "toxoid" as used herein refers to a bacterial toxin (usually an exotoxin), whose toxicity has been inactivated or suppressed either by chemical (formalin) or heat treatment, while other properties, typically immunogenicity, are maintained. A mutated toxoid as used herein is a recombinant bacterial toxin, which has been amended to be less toxic or even non-toxic by amending the wild-type amino acid sequence. Such a mutation could be a substitution of one or more amino acids. Such a mutated toxoid presents on its surface a functionality that can react with the functional group Y of the interconnecting molecule to provide a modified toxoid. Said functionality is known to the person skilled in the art and includes, but is not restricted to the primary amino functionality of a lysine residue that can react with activated esters, an isocyanate group or an aldehyde in presence of a reducing agent, to the carboxylate functionality of a glutamate or aspartate residue that can be activated by carbodiimides or to the thiol functionality of a cysteine residue. Activated esters include N-(γ-maleimidobutyryloxy) sulfosuccinimide ester (sulfo-GMBS), succinimidyl (4-iodoacetyl) aminobenzoate (sulfo-SIAB), succinimidyl-3-(bromoacetamido)propionate (SBAP), disuccinimidyl glutarat (DSG), 2-pyridyldithiol-tetraoxatetradecane-N-hydroxysuccinimide (PEG-4-SPDP). The cysteine residue on the carrier protein can be converted to the corresponding dehydroalanine that can be further reacted with a suitable interconnecting molecule to provide modified carrier protein having on their surface the functional group X of the interconnecting molecule. In this case the functional group Y on the interconnecting molecule might be a thiol group and the group X might be an alkene. Such interconnecting molecules include allylmercaptan. After reaction with such interconnecting molecule, the carrier protein is converted to a modified carrier protein presenting the vinyl group X of the interconnecting molecule, which is suitable to react with the saccharides of general formula (I).

It is especially preferred that the saccharide of general formula (I), and preferably saccharide **37, 34, 31, 26, 22, 18, 11** and **6** is conjugated to the non-toxic mutated diphtheria toxin CRM₁₉₇ presenting as a functionality a primary amine functionality of a lysine residue..

CRM₁₉₇ like wild-type diphtheria toxin is a single polypeptide chain of 535 amino acids (58 kD) consisting of two subunits linked by disulfide bridges having a single amino acid substitution of glutamic acid for glycine. It is utilized as a carrier protein in a number of approved conjugate vaccines for diseases such as Prevnar.

Thus, in a preferred embodiment of the present invention the carrier protein presents on its surface primary amino functionalities of lysine residues that are able to react with the functional group Y of the interconnecting molecule to provide modified carrier protein having on their surface said functional group X of the interconnecting molecule, which is able to react with the terminal thiol group of the linker of the compounds of general formula (I). Said functional group X of the interconnecting molecules is selected of the group comprising or consisting of maleimide; α-iodoacetyl; α-bromoacetyl; *N*-hydroxysuccinimide ester (NHS), 2-pyridyldithiols, thiol and vinyl (see **Figure 3**)**.**

Preferably, the saccharide of general formula (**I**) is conjugated to the non-toxic mutated diphtheria toxin CRM₁₉₇, which is modified by maleimide. In yet another preferred embodiment, the saccharide of general formula (I) is conjugated to the non-toxic mutated diphtheria toxin CRM₁₉₇, which is modified by vinyl. In the most preferred embodiment, the compound of general formula (I) is conjugated to the non-toxic mutated diphtheria toxin CRM₁₉₇, which is modified by α-bromoacetamide.

In another embodiment, said immunogenic carrier is preferably a glycosphingolipid with immunomodulatory properties, and more preferably (2*S*,3*S*,4*R*)-1-(α-*D-*galactopyranosyl)-2-hexacosanoylaminooctadecane-3,4-diol. The term glycosphingolipid with immunomodulatory properties, as used herein, refers to a suitable glycosphingolipid capable of stimulating the immune system's response to a target antigen, but which does not in itself confer immunity as defined above.

Glycoshingolipids as used herein are compounds containing a carbohydrate moiety α-linked to a sphingolipid. Preferably, the carbohydrate moiety is a hexopyranose and most preferably is α-D-galactopyranose. For the person skilled in the art, sphingolipids are a class of lipids containing a C18 amino alcohol connected via an amide bond to a fatty acid. The C18 amino alcohol is preferably mono-, di- or polysubstituted with hydroxyl groups. Especially preferred, the C18 amino alcohol is phytosphingosine. The fatty acid is preferably a monocarboxylic acid having a saturated alkyl chain of a number of carbons ranging from 16 to 28 and more preferably from 18 to 26. Glycosphingolipids with immunomodulatory properties include, but they are not restricted to (2*S*,3*S*,4*R*)-1-(α-*D*-galactopyranosyl)-2-hexacosanoylaminooctadecane-3,4-diol, which can stimulate natural killer (NK) activity and cytokine production by natural killer T (NKT) cells and exhibits potent antitumor activity *in vivo (*Proc. Natl Acad. Sci. USA, 1998, 95, 5690).

The conjugates of the compounds of general formula (I) to the glycosphingolipid with immunomodulatory properties have the advantage of being heat stable. To be suitable for conjugation, on the glycosphingolipid with immunomodulatory properties a functionality is introduced. Said functionality is prone to react directly with the terminal thiol group of the linker of the saccharides of general formula (I) to provide glycoconjugates of the saccharides of general formula (I) or with the functional group Y of the interconnecting molecule to provide the modified glycosphingolipid with immunomodulatory properties.

Preferably, said functionality is introduced at the C6 of the carbohydrate moiety of the glycosphingolipid with immunomodulatory properties. Thus, the glycosphingolipid with immunomodulatory properties is functionalized with a functionality, which is prone of reacting with thiol group, activated ester, isocyanate group, aldehyde, vinyl, amino group and azido group to provide directly the glycoconjugate of the saccharides of general formula (I) or the modified glycosphingolipid with immunomodulatory properties presenting the functional group X of the interconnecting molecule.

Preferably, the functionality introduced at the C6 of the carbohydrate moiety of the glycosphingolipid with immunomodulatory properties is selected from the group comprising or containing an amine, a thiol, an alcohol, a carboxylic acid, a vinyl, maleimide; α-iodoacetyl; α-bromoacetyl; N-hydroxysuccinimide ester (NHS), 2-pyridyldithiols.

Said functional group X of the interconnecting molecules is selected of the group comprising or consisting of maleimide; α-iodoacetyl; α-bromoacetyl; N-hydroxysuccinimide ester (NHS), 2-pyridyldithiols, thiol and vinyl.

It was found that the glycoconjugates obtained by reacting the saccharides of general formula (I) with an immunogenic carrier are suitable to elicit an immune response in an animal, and therefore are useful as a vaccine for immunization against diseases associated with bacteria containing in their capsular polysaccharide a saccharide structure selected from:

| |
|---|
| α-2,4,6-trideoxy-4-amino-D-GalNAc-(1→4)-α-D-GalAp-(1→3)-α-D-GalAp- |
| α-2,4,6-trideoxy-4-amino-D-GalNAc-(1→4)-α-D-GalAp |
| α-D-GalAp-(1→3)-α-D-GalAp |
| α-D-GalAp |
| α-2,4,6-trideoxy-4-amino-D-GalNAc |
| α-D-GalAp-(1→3)-α-D-GalAp-(1→3)-α-2,4,6-trideoxy-4-amino-D-GalNAc |
| α-D-GalAp-(1→3)-α-2,4,6-trideoxy-4-amino-D-GalNAc |
| α-D-GalAp-(1→3)-α-2,4,6-trideoxy-4-amino-D-GalNAc-(1→4)-α-D-GalAp |

Preferably, the bacteria containing in the capsular polysaccharide a one of the above mentioned saccharide structures is *Streptococcus pneumoniae* serotype 1.

In a preferred embodiment the glycoconjugates obtained by reacting the saccharides of general formula (I) with an immunogenic carrier are useful as a vaccine for immunization against diseases associated with bacteria, wherein said diseases include pneumonia, meningitis, otitis media, bacteremia and acute exacerbation of chronic bronchitis, sinusitis, arthritis and conjunctivitis

One aspect of the present invention relates to pharmaceutical compositions, especially vaccines comprising at least one glycoconjugate obtained by reacting any saccharide of general formula (I) with an immunogenic carrier, and/or one saccharide of general formula (**I**) and/or a intermediate of general formula (**II**), together with at least one pharmaceutically acceptable cryoprotectant, lyoprotectant, excipient and/or diluent.

Said vaccine may be prepared in the form of a suspension or may be lyophilized. The suspension form may be stored frozen. In the lyophilized form, it is preferable to add one or more stabilizers. Optionally, one or more adjuvants may be added as well. Any conventional stabilizers and adjuvants may be included in a vaccine according to this invention.

The term "adjuvant" as used herein refers to an immunological adjuvant i.e. a material used in a vaccine composition that modifies or augments the effects of said vaccine by enhancing the immune response to a given antigen contained in the vaccine without being antigenically related to it. For the persons skilled in the art, classically recognized examples immunological adjuvants include but are not restricted to oil emulsions (e.g., Freund's adjuvant), saponins, aluminium or calcium salts (e.g., alum), non-ionic block polymer surfactants, and many others.

Vaccination can be performed at any age. The vaccine many be administered subcutaneously, by spray, by injection, orally, intraocularly, intratracheally or nasally.

Another aspect of the present invention relates to pharmaceutical formulations and pharmaceutical compositions containing the vaccine as an active ingredient, together with at least one pharmaceutically acceptable carrier, excipient, solvent and/or diluents.

Further preferred, the pharmaceutical composition is formulated in the form of a lyophilisate or liquid buffer solution.

The vaccine can also be administered in form of its pharmaceutically active salt optionally using substantially nontoxic pharmaceutically acceptable carrier, excipients, adjuvants or diluents. The vaccine of the present invention is prepared in a conventional solid or liquid carrier or diluents and a conventional pharmaceutically-made adjuvant at suitable dosage level in a known way. The preferred preparations and formulations are in administrable form which is suitable for oral application. These administrable forms, for example, include pills, tablets, film tablets, coated tablets, capsules, powders and deposits. Other than oral administratable forms are also possible. The inventive vaccine may be administered by any appropriate means, including but not limited to inhalation, injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrically, intracutaneously, intravaginally, intravasally, intranasally, intrabuccally, percutaneously, sublingually, or any other means available within the pharmaceutical arts.

The vaccine of the present invention, containing the glycoconjugate obtained by reacting the saccharide of general formula (I) with an immunogenic carrier or pharmaceutically acceptable salts thereof as an active ingredient will typically be administered in admixture with suitable carrier materials suitably selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active ingredient may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the vaccine of the present invention may be formulated in sustained release form to provide the rate controlled release of any one or more of the components or active ingredients to optimize the therapeutic effects. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are solid form preparations which are intended to be converted, shortly before use, to liquid form preparations for either oral or parenteral administration. Such liquid forms include solutions, suspensions and emulsions.

The vaccine of the present invention may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refer to the active ingredients dispersed or solubilized in a hydrophilic semi-solid matrix.

Powders for constitution refer to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and D, L-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1 % to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1 %.

Techniques for the formulation and administration of the vaccine of the present invention may be found in "Remington's Pharmaceutical Sciences" Mack Publishing Co., Easton PA. A suitable vaccine composition comprising at least one glycoconjugate of the present invention and/or pharmaceutically acceptable salts thereof may be a solution of one glycoconjugate obtained by reacting any saccharide of general formula (I) with an immunogenic carrier in a suitable liquid pharmaceutical carrier or any other formulation such as tablets, pills, film tablets, coated tablets, dragees, capsules, powders and deposits, gels, syrups, slurries, suspensions, emulsions, and the like.

A therapeutically effective dosage of one glycoconjugate obtained by reacting any saccharide of general formula (I) with an immunogenic carrier refers to that amount of the compound that results in an at least a partial immunization against a disease. Toxicity and therapeutic efficacy of such compounds can be determined by standard pharmaceutical, pharmacological, and toxicological procedures in cell cultures or experimental animals. The dose ratio between toxic and therapeutic effect is the therapeutic index. The actual amount of the composition administered will be dependent on the subject being treated, on the subject's weight, the severity of the affliction, the manner of administration and the judgement of the prescribing physician.

Another preferred embodiment of the present invention is directed to pharmaceutical composition comprising the glycoconjugate obtained by reacting any saccharide of general formula (I) with an immunogenic carrier, and/or the saccharide of general formula (I), and/or the intermediate of general formula (**II**) together with at least one one pharmaceutically acceptable cryoprotectant, lyoprotectant, excipient and/or for immunization against diseases associated with *Streptococcus pneumoniae* bacteria.

*Streptococcus pneumoniae* bacteria referred herein include the following serotypes *Streptococcus pneumoniae* type 4, *Streptococcus pneumoniae* type 9V, *Streptococcus pneumoniae* type 2, *Streptococcus pneumoniae* type 19F, *Streptococcus pneumoniae* type 3, *Streptococcus pneumoniae* type 19A, *Streptococcus pneumoniae* type 12F, *Streptococcus pneumoniae* type 31, *Streptococcus pneumoniae* type 7F, *Streptococcus pneumoniae* type 5, *Streptococcus pneumoniae* type 14, *Streptococcus pneumoniae* type 6A, *Streptococcus pneumoniae* type 6B, *Streptococcus pneumoniae* type 18C and *Streptococcus pneumoniae* type 23F.

A preferred embodiment of the present invention is directed to a pharmaceutical composition, especially a vaccine comprising the glycoconjugate obtained by reacting any saccharide of general formula (I) with an immunogenic carrier for immunization against diseases associated with *Streptococcus pneumoniae* type 1 and type 3.

The saccharides derived from [→3)-β-D-GlcAp-(1→4)-β-D-Glc(1→] are functionalized with suitable linker, which allows their conjugation to an immunogenic carrier, as defined herein to provide glycoconjugates. Said glycoconjugates proved to be efficient as a vaccine for immunization against diseases associated with bacteria, and in particularly against diseases associated with *Streptococcus pneumoniae,* and particularly against *Streptococcus pneumoniae* type 3. In particular, the glycoconjugates obtained by reacting saccharide of general formula (I) with glycosphingolipids with immunomodulatory properties according to the present invention, were able to elicit in mice immunized with said glycoconjugates, high titer of antibodies specific to the saccharides of general formula (I). Said diseases include pneumonia, meningitis, otitis media, bacteremia and acute exacerbation of chronic bronchitis, sinusitis, arthritis and conjunctivitis.

Yet another aspect of the present invention refers to saccharide of general formula (I) useful as a marker in immunological assays for diagnostics of diseases caused by bacteria containing in the capsular polysaccharide a saccharide structure selected from:

| |
|---|
| α-2,4,6-trideoxy-4-amino-D-GalNAc-(1→4)-α-D-GalAp-(1→3)-α-D-GalAp- |
| α-2,4,6-trideoxy-4-amino-D-GalNAc-(1→4)-α-D-GalAp |
| α-D-GalAp-(1→3)-α-D-GalAp |
| α-D-GalAp |
| α-2,4,6-trideoxy-4-amino-D-GalNAc |
| α-D-GalAp-(1→3)-α-D-GalAp-(1→3)-α-2,4,6-trideoxy-4-amino-D-GalNAc |
| α-D-GalAp-(1→3)-α-2,4,6-trideoxy-4-amino-D-GalNAc |
| α-D-GalAp-(1→3)-α-2,4,6-trideoxy-4-amino-D-GalNAc-(1→4)-α-D-GalAp |

Such assays comprise, for instance, microarray and ELISA useful for diagnosis of diseases caused by bacteria containing or comprising the saccharides of the present invention, said diseases including pneumonia, meningitis, otitis media, bacteremia and acute exacerbation of chronic bronchitis, sinusitis, arthritis and conjunctivitis.

The saccharides of the present invention can be easily conjugated to solid supports for providing assays useful for diagnosis of diseases caused by bacteria containing or comprising the saccharides of the present invention. Said solid supports present on their surface a functionality that is prone to react with the functional group Y of the interconnecting molecule to provide modified solid supports, presenting on their surface the functional group X of the interconnecting molecule, which are able to react with the thiol group of saccharides of general formula (I). Said solid supports include, but are not restricted to microarray slides, which present on their surface a functionality that is prone to react with the functional group Y of the interconnecting molecule to provide modified microarray slides, presenting of their surface the functional group X of the interconnecting molecule. Preferably, the microarray slides present on their surface an amino group. Microarray slides presenting on their surface an amino group include, but are not restricted to amine-coated GAPS II slides (Corning) or CodeLink NHS slides on which the amino functionality was introduced by incubation with Bovin Serum Albumin (BSA).

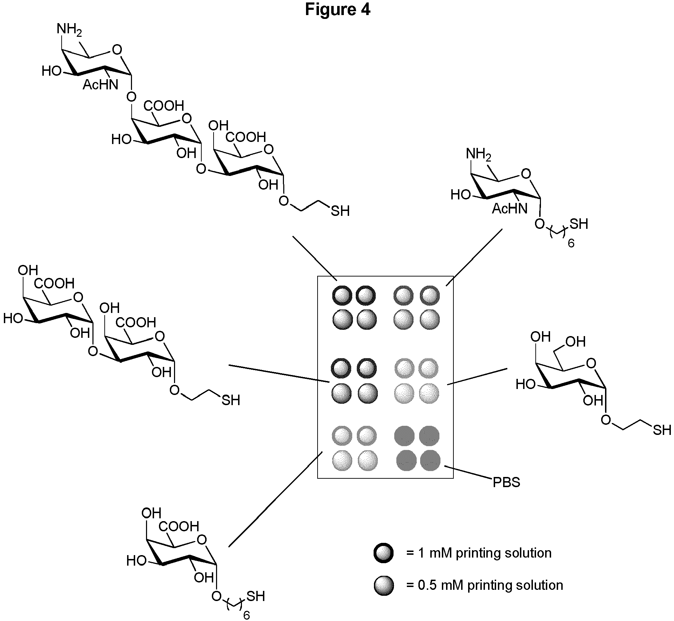

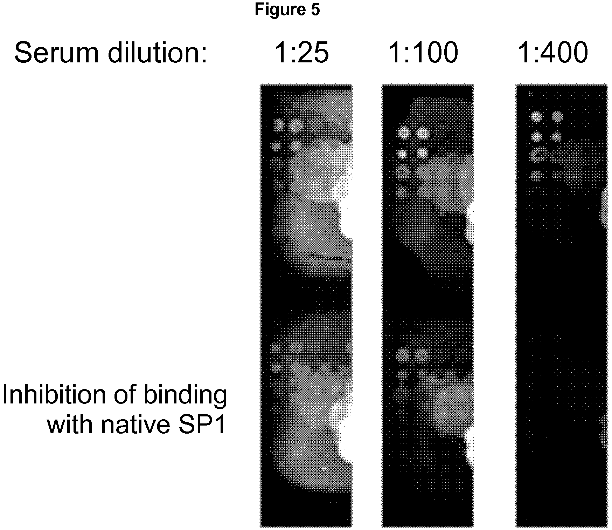

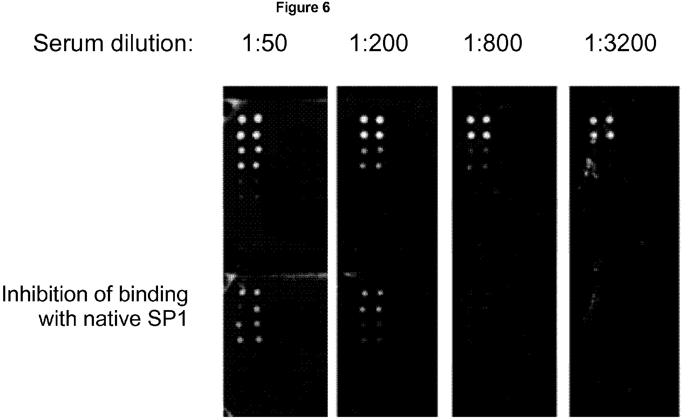

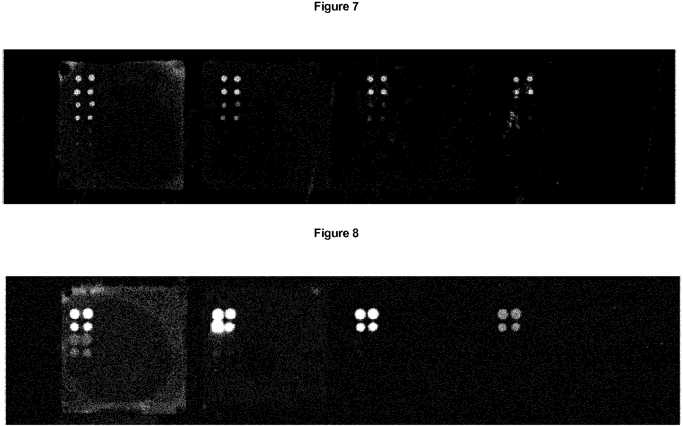

Microarray slides coated with the saccharides of general formula (I) were synthesized by conjugating the sacharides of general formula (I) to said modified microarray slides, and incubated with rabbit anti-SP1 typing serum or human pneumococcal serum 007sp in the presence or absence of native SP1 polysaccharide. The binding experiments show that both rabbit anti-SP1 typing serum and human pneumococcal serum 007sp bound to α-2,4,6-trideoxy-4-amino-D-GalNAc-(1→4)-α-D-GalAp-(1→3)-α-D-GalAp and α-D-GalAp-(1→3)-α-D-GalAp saccharide structures (see Figures 4-8). Moreover, the binding could be inhibited with the native SP1 polysaccharide, suggesting that the saccharides according top the present invention share epitopes that are recognized by the immune system (see **Figure 4** and **Figure 5**)**.**

### Description of the figures

**Figure 1** shows the global distribution of *Streptococcus pneumoniae* serotypes
**Figure 2** provides examples of interconnecting molecules according to the present invention.
**Figure 3** provides examples of functional group X of the interconnecting molecule according to the present invention.
**Figure 4** shows the printing pattern of the saccharides of general formula (I) on microarray slides
**Figure 5** shows the binding of human pneumococcal serum 007sp (pooled sera of 287 humans immunized with Pneumovax vaccine) to saccharides of general formula (I), which are coated on modified CodeLink NHS slides in presence and in absence of native SP1 polysaccharide.
**Figure 6** shows the binding of the rabbit anti-SP1 typing serum to saccharides of general formula (**I**), which are coated on modified amine-coated GAPS II slides (Corning) in presence and in absence of native SP1 polysaccharide.
**Figure 7** shows the binding of the rabbit anti-SP1 typing serum to saccharides of general formula (I), which are coated on modified amine-coated GAPS II slides (Corning).
**Figure 8** shows the binding of the rabbit anti-SP1 typing serum to saccharides of general formula (**I**), which are coated on modified CodeLink NHS slides.

The following examples are included to demonstrate preferred embodiments of the invention. It should be appreciated by those of skill in the art that the techniques disclosed in the examples, which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Examples

### Chemical synthesis

General information for chemical synthesis. Commercial reagents were used without further purification except where noted. Solvents were dried and redistilled prior to use in the usual way. All reactions were performed in oven-dried glassware under an inert atmosphere unless noted otherwise. Analytical thin layer chromatography (TLC) was performed on Kieselgel 60 F254 glass plates precoated with a 0.25 mm thickness of silica gel. The TLC plates were visualized with UV light and by staining with Hanessian solution (ceric sulfate and ammonium molybdate in aqueous sulfuric acid) or sulfuric acid-ethanol solution. Column chromatography was performed on Fluka Kieselgel 60 (230-400 mesh). Optical rotations (OR) were measured with a Schmidt & Haensch UniPol L1000 polarimeter at a concentration (c) expressed in g/100 mL. ¹H and ¹³C NMR spectra were measured with a Varian 400-MR or Varian 600 spectrometer with Me₄Si as the internal standard. NMR chemical shifts (δ) were recorded in ppm and coupling constants (J) were reported in Hz. High-resolution mass spectra (HRMS) were recorded with an Agilent 6210 ESI-TOF mass spectrometer at the Freie Universität Berlin, Mass Spectrometry Core Facility.

**Example 1: 4-(Benzyloxycarbonyl)amino-3-*O*-levulinoyl-4,6-dideoxy-D-galactal (1*):** To a stirred solution of 4-*O*-(benzyloxycarbonyl)amino-3-hydroxy-4,6-dideoxy-D-galactal (Org. Lett. 2010, 12, 1624) (1.64 g, 6.21 mmol) (1.64 g, 6.21 mmol) in CH₂Cl₂ (40 ml) were added at 0 °C pyridine (0.501 ml, 6.21 mmol), levulinic acid (0.96 ml, 9.31 mmol), DMAP (0.152 g, 1.242 mmol) and EDC (1.205 ml, 6.83 mmol). The mixture was warmed to room temperature and stirred at that temperature. After 3 h, 0.5 eq. levulinic acid and 0.5 eq. EDC were added to drive the reaction to completion. After 5 h, the mixture was diluted with 100 ml DCM and washed with water (50 ml), sat. aq. NH₄Cl (50 ml), sat. aq. NaHCO₃ (50 ml) and brine (50 ml). The organic fraction was dried over Na₂SO₄ and concentrated. The residue was purified by flash chromatography (EtOAc/hexanes 1:1) to give ester **1*** (2.07 g, 5.73 mmol, 92%) as a clear oil. HRMS (ESI) calcd for C₁₉H₂₃NO₆ (M+Na⁺) 384.1423 found 384.1415 m/z.

**Example 2: Dibutyl [2-azido-4-(benzyloxycarbonyl)amino-3-*O*-levulinoyl-2,4,6-trideoxy-D-galactopyranosyl] phosphate (2*):** To a stirred solution of galactal **1*** (3.17 g, 8.77 mmol) in dry MeCN (44 ml) were added at -25°C ceric ammonium nitrate (14.42 g, 26.3 mmol) and sodium azide (0.86 g, 13.15 mmol). The reaction was stirred vigorously between -25 °C and -20 °C for 6 h. The mixture was diluted with cold Et₂O (50 ml). The organic layer was washed with cold water (3x30 ml), dried over Na₂SO₄ and concentrated. The residue was filtered through a plug of silica gel (EtOAc/hexanes/Et₃N 1:1:0.01) to give the crude glycosyl nitrate as 4:1 *galacto*/*talo* mixture (2.01 g) as a slightly yellow oil.

To the crude glycosyl nitrate (2.01 g) was added at room temperature a solution of cesium dibutyl phosphate (2.21 g, 6.45 mmol) in dry DMF (28 ml). The mixture was stirred at that temperature for 4.5 h, diluted with EtOAc (100 ml) and poured into water (100 ml). The organic phase was washed with water (5x50 ml) and the combined aqueous fractions were extracted with EtOAc (50 ml). The organic phase was dried over Na₂SO₄ and concentrated. The residue was purified by flash chromatography (EtOAc/hexanes 45:55 to 50:50) to give glycosyl phosphate **2*** (1.84 g, 3.00 mmol, 37%, 1:10 α/β) as a clear oil. HRMS (ESI) calcd for C₂₇H₄₁N₄O₁₀P (M+Na⁺) 635.2458 found 635.2422 *m*/*z*.

**Example 3: Ethyl 2-*O*-benzyl-3,4-isopropylidene-1-thio-β-D-galactopyranoside (3*):** To a stirred solution of ethyl 6-*O*-*tert*-butyldimethylsilyl-3,4-isopropylidene-1-thio-β-D-galactopyranoside (Bioorg. Med. Chem. 2001, 9, 1395) (45.7 g, 121 mmol) in DMF (150 ml) and THF (75 ml) were added at 0 °C portionwise sodium hydride (60%, 7.24 g, 181 mmol) and then benzyl bromide (17.2 ml, 145 mmol). The mixture was stirred for 1 h at 0 °C, slowly warmed to room temperature and stirred for 16 h at that temperature. The reaction was quenched at 0 °C with sat. aq. NH₄Cl (20 ml), diluted with water (200 ml) and EtOAc (150 ml) and stirred for 15 min at 0°C. After separation, the organic phase was washed with water (5x100 ml) and the combined aqueous fractions were re-extracted with EtOAc (2x100 ml). The combined organic extracts were dried over Na₂SO₄ and concentrated to give the crude benzyl ether (61 g) as a yellow oil.
To a stirred solution of the crude benzyl ether (61 g) in THF (370 ml) was added at 0 °C tetrabutylammonium fluoride (1 M in THF, 166 ml, 166 mmol). The mixture was warmed to room temperature and stirred for 1 h. The reaction was diluted with sat. aq. NaHCO₃ (200 ml) and EtOAc (100 ml). After separation, the aqueous phase was extracted with EtOAc (3x100 ml), the combined organic fractions were dried over MgSO₄ and concentrated. The residue was purified by flash chromatography (EtOAc/hexanes 0:1 to 1:3 to 1:1) to give alcohol **3*** as a white solid. HRMS (ESI) calcd for C₁₈H₂₆O₅S (M+Na⁺) 377.1398 found 377.1416 *m*/*z*.

**Example 4: Methyl (ethyl 2-*O*-benzyl-1-thio-β-D-galactopyranosid)uronate (4*):** To a vigorously stirred solution of alcohol **4*** (6.0 g, 16.93 mmol) in CH₂Cl₂ (50 ml) and H₂O (25 ml) were added at 0 °C TEMPO (0.53 g, 3.39 mmol) and BAIB (10.9 g, 33.9 mmol). The mixture was warmed to room temperature and stirred for 1 h at that temperature. The reaction was quenched with 10% aq. Na₂S₂O₃ (10 ml) and diluted with EtOAc (30 ml). After separation, the organic phase was washed with 10% Na₂S₂O₃ (4x20 ml). The aqueous phase was extracted with EtOAc (2x20 ml) and the combined organic fractions were dried over Na₂SO₄ and concentrated to give the crude acid (7.92 g) as yellow oil.
To a stirred solution of acetyl chloride (6.04 ml, 85 mmol) in MeOH (300 ml) was added dropwise at 0 °C a solution of the crude acid (7.92 g) in MeOH (40 ml). The mixture was warmed to room temperature, stirred for 2 h at that temperature and cooled to 0 °C. The reaction was quenched with sat. aq. NaHC03 (30 ml) and neutralized to pH 7 with solid NaHCO₃. The volatiles were evaporated and the mixture was diluted with EtOAc (70 ml). After separation, the aqueous phase was extracted with EtOAc (5x50 ml). The combined organic fractions were dried over Na₂SO₄ and concentrated. Flash chromatography was performed (EtOAc/hexanes 2:3 to 1:1, then 1:0) to give the crude product, which was crystallized in methanol at - 20 °C (5 ml/g crude product) to give diol **4*** (3.47 g, 10.13 mmol, 60%) as a white solid. HRMS (ESI) calcd for C₁₆H₂₂O₆S (M+Na)⁺ 365.1034 found 365.1058 *m*/*z*.

**Example 5: Methyl (ethyl 2-*O*-benzyl-3,4-*O*-endo-benzylidene-1-thio-β-D-galactopyranosid)uronate (5*) and Methyl (ethyl 2-*O*-benzyl-3,4-*O*-exo-benzylidene-1-thio-β-D-galactopyranosid)uronate (6*):** To a stirred solution of diol **4*** (2.99 g, 8.73 mmol) in dry acetonitrile (29 ml) were added at room temperature benzaldehyde dimethyl acetal (6.57 ml, 43.6 mmol) and DL-camphorsulfonic acid (0.51 g, 2.18 mmol). The mixture was stirred at room temperature for 5 h and the reaction was quenched by addition of triethylamine (0.35 ml). The mixture was concentrated under reduced pressure to give a residue, which was filtered through a short plug of silica gel (hexanes/EtOAc 8:1 (2% Et₃N) to 1:1 (2% Et₃N)) to give a 1:1 mixture of benzylidene acetals **5*** *(endo)* and **6*** (*exo*) (3.46 g, 8.03 mmol, 92%). The isomers were separated by selective crystallization of exo-isomer **6*** from EtOAc/hexanes and chromatographic separation of the mother liquor (Biotage, flat gradient of 10% to 40% EtOAc in hexanes + 0.5% Et₃N). Analytical data for **5*:** Clear oil. HRMS (ESI) calcd for C₂₃H₂₆O₆S (M+Na)⁺ 453.1348, found 453.1352 *m*/*z*. Analytical data for **6*:** White solid. HRMS (ESI) calcd for C₂₃H₂₆O₆S (M+Na)⁺ 453.1348, found 453.1338 m/z.

**Example 6: Methyl (ethyl 2,3-*O*-benzyl-1-thio-β-D-galactopyranosyl)uronate (7*):** To a solution of acetal **6*** (162 mg, 0.38 mmol) and sodium cyanoborohydride (296 mg, 4.70 mmol) in THF (9.4 ml) was added at room temperature a solution of hydrogen chloride (1 M in Et₂O) until the evolution of gas ceased. After 10 min, sodium cyanoborohydride (296 mg, 4.70 mmol) was added, followed by the addition of HCl. The reaction was stirred at room temperature, diluted with EtOAc (30 ml) and quenched with sat. aq. NaHCO₃ (30 ml). After separation, the organic layer was washed with sat. aq. NaHCO₃ (20 ml) and the aqueous layer was re-extracted with EtOAc (2x20 ml). The organic extracts were pooled, dried over MgSO₄ and concentrated. The residue was purified by flash chromatography (EtOAc/hexanes 1:1) to give alcohol **7*** (67.5 mg, 0.156 mmol, 42%) as a white solid. HRMS (ESI) calcd for C₂₃H₂₈O₆S (M+Na⁺) 455.1504 found 455.1511 *m*/*z*.

**Example 7: Methyl (ethyl 2,3-*O*-benzyl-4-*O*-fluorenylmethoxycarbonyl-1-thio-β-D-galactopyranosyl)uronate (8*):** To a stirring solution of alcohol **7*** (160 mg, 0.370 mmol) in pyridine (1.2 ml) was added at 0 °C FmocCl (383 mg, 1.48 mmol). The mixture was warmed to room temperature and stirred for 3 h. The mixture was then diluted with EtOAc (50 ml) and washed with 1 N HCl (2x30 ml) and sat. aq. NaHCO₃ (30 ml). The organic phase was dried over Na₂SO₄ and concentrated. The residue was purified by flash chromatography (EtOAc/hexanes 1:2) to give carbonate **8*** (217 mg, 0.331 mmol, 90%) as a white foam. HRMS (ESI) calcd for C₃₈H₃₈O₈S (M+Na)⁺ 677.2185 found 677.2167 *m*/*z*.

**Example 8: Dibutyl [methyl (2,3-*O*-benzyl-4-*O*-fluorenylmethoxycarbonyl-α/β-D-galactopyranosyl)uronate] phosphate (9*):** Thioglycoside **8*** (200 mg, 0.305 mmol) was co-evaporated with dry toluene (2x30 ml), kept under high vacuum for 1 h and dissolved in dry CH₂Cl₂ (3 ml). Activated molecular sieves (3 A-AW) were added and the solution was stirred for 15 min at room temperature. The solution was then cooled to 0 °C, treated with dibutyl phosphoric acid (128 mg, 0.611 mmol) and stirred for another 15 min. The mixture was then treated with NIS (89 mg, 0.397 mmol), warmed to room temperature and stirred for 3 h at that temperature. The reaction was diluted with CH₂Cl₂ (20 ml) and quenched with a 1:1 (v/v) mixture of 10% aq. Na₂S₂O₃ and sat. aq. NaHCO₃ (20 ml). The aqueous phase was extracted with CH₂Cl₂ (3x30 ml), the combined organic fractions were dried over Na₂SO₄ and concentrated. The residue was purified by flash chromatography (EtOAc/hexanes 1:1 to 2:1) to give glycosyl phosphate **9*** (218 mg, 0.272 mmol, 89%, 10:1 α/β as clear oil. Analytical data of **9*α:** HRMS (ESI) calcd for C₄₄H₅₁O₁₂P (M+Na)⁺ 825.3015 found 825.3020 m/z. Analytical data of **9*β:** HRMS (ESI) calcd for C₄₄H₅₁O₁₂P (M+Na)⁺ 825.3015 found 825.2970 *m*/*z*.

**Example 9: Methyl (2-*O*-benzyl-3,4-*O*-*endo*-benzylidene-α/β-D-galactopyranosyl)uronate-(1→1)-2-(benzylthio)ethanol (10*):** Thioglycoside **5*** (102 mg, 0.237 mmol), 2-(benzylthio)ethanol **11*** (60 mg, 0.355 mmol) and TTBPy. (117 mg, 0.474 mmol) were co-evaporated with anh. toluene (3x10 ml) and kept under high vacuum for 30 min. The mixture was dissolved in THF (4.8 ml) and stirred over activated molecular sieves (3 A) for 30 min at room temperature. The solution was cooled to 0 °C and treated with DMTST (92 mg, 0.355 mmol in 0.2 ml dry CH₂Cl₂). The reaction was warmed to room temperature and stirred for 2 h at that temperature. The reaction was quenched with a 1:1 (v/v) MeOH/Et₃N mixture (0.1 ml) and concentrated. The residue was purified by flash chromatography (EtOAc/hexanes/Et₃N 0:1:0.01 to 30:70:0.01 to 45:55:0.01) to give thioether **10*α** (59 mg, 0.110 mmol, 46%) as a clear oil, along with the corresponding β-isomer **10*β** (35 mg, 0.065 mmol, 27%). Analytical data for **10*α:** HRMS (ESI) calcd for C₃₀H₃₂O₇S (M+Na)⁺ 559.1766 found 559.1731 *m*/*z*.

**Example 10: Methyl (2,4-di-*O-*benzyl-α-D-galactopyranosid)uronate-(1→1)-2-(benzylthio)ethanol (12*):** To a stirred solution of acetal **10*α** (100.0 mg, 0.186 mmol) in dry THF (5.3 ml) was added first borane trimethylamine complex (57.4 mg, 0.745 mmol) and then aluminium chloride (149 mg, 1.118 mmol) at room temperature. The mixture was stirred for 4.5 h. The reaction was quenched by addition of water (10 ml) and 1 M aq. HCl (5 ml). The mixture was extracted with EtOAc (3x 10 ml) and the combined organic fractions were dried over Na₂SO₄ and concentrated. The residue was purified by flash chromatography (EtOAc/hexanes 2:5 to 1:1) to give alcohol **12*** (70.0 mg, 0.13 mmol, 70%) as a clear oil. HRMS (ESI) calcd for C₃₀H₃₄O₇S (M+Na)⁺ 561.1923 found 561.1879 *m*/*z*.

**Example 11: Methyl (2,3-di-*O*-benzyl-4-*O*-fluorenylmethoxycarbonyl-α-D-galactopyranosyl)uronate-(1→3)-methyl (2,4-di-*O*-benzyl-α-D-galactopyranosyl)uronate-(1→3)-(2-(benzylthio)ethanol (13*):** Alcohol **12*** (90 mg, 0.166 mmol) and glycosyl phosphate **9*** (208 mg, 0.259 mmol) were co-evaporated with dry toluene (3x10 ml) and kept under high vacuum for 1 h. The mixture was dissolved in dry CH₂Cl₂ (3.3 ml) and stirred over activated molecular sieves (3 A-AW) for 30 min at room temperature. The solution was cooled to 0 °C and treated dropwise with TBSOTf (0.133 mmol in 0.2 ml dry CH₂Cl₂). The solution was warmed to room temperature and stirred for 20 h. The reaction was diluted with CH₂Cl₂ (10 ml) and quenched with a 1:1 (v/v) MeOH/pyridine mixture (0.2 ml). The solution was filtered through Celite and concentrated. The crude product was filtered through a short plug of silica gel (EtOAc/hexanes 1:1) to give the intermediate disaccharide mixture (150 mg, 0.133 mmol, 80%, 3:1 α/β) as a clear oil.
To a stirred solution of the carbonate mixture (150 mg) in CH₂Cl₂ (2.6 ml) was added at room temperature triethylamine (1.1 ml, 7.96 mmol).The reaction was stirred for 3 h at that temperature and co-evaporated with toluene (2x10 ml). The residue was purified by flash chromatography (EtOAc/hexanes 1:6 to 2:3 to 1:1) to give alcohol **13*** (62 mg, 0.068 mmol, 51 %) along with the corresponding β-anomer (20 mg, 0.022 mmol, 17%). HRMS (ESI) calcd for C₅₁H₅₆O₁₃S (M+Na)⁺ 931.3339, found 931.3340 *m*/*z*.

**Example 12: 2-Azido-4-(benzyloxycarbonyl)amino-3-*O*-levulinoyl-2,4,6-trideoxy-α-D-galactopyranosyl-(1→4)-methyl (2,3-di-*O*-benzyl-α-D-galactopyranosyl)uronate-(1→3)-methyl (2,4-di-*O*-benzyl-α-D-galactopyranosyl)uronate-(1→1)-2-(benzylthio)ethanol (14*):** Alcohol **13*** (65 mg, 0.062 mmol) and glycosyl phosphate **2*** (61 mg, 0.100 mmol) were co-evaproated with dry toluene (3x10 ml) and kept under high vacuum for 30 min. The mixture was dissolved in CH₂Cl₂ (2.1 ml) and stirred over activated molecular sieves (4 A-AW) for 1 h at room temperature. The solution was then cooled to 0 °C and treated with TMSOTf (17 µl, 0.093 mmol in 0.2 ml dry CH₂Cl₂). The mixture was allowed to stir for 3 h at 0 °C, when TLC (EtOAc/hexanes 2:3) indicated complete consumption of the acceptor. The reaction was quenched with a 1:1 (v/v) MeOH/NEt₃ mixture (0.5 ml), diluted with CH₂Cl₂ (20 ml) and filtered through Celite. The crude product was purified by flash chromatography (EtOAc/hexanes 1:2 to 1:1) to give trisaccharide **14*** (69 mg, 0.053 mmol, 85%) as a clear oil. HRMS (ESI) calcd. for C₇₀H₇₈N₄O₁₉S (M+Na)⁺ 1333.4879 found 1333.4911 *m*/*z*.

**Example 13: 2-Azido-4-(benzyloxycarbonyl)amino-2,4,6-trideoxy-α-D-galactopyranosyl-(1→4)-methyl (2,3-di-*O*-benzyl-α-D-galactopyranosyl)uronate-(1→3)-methyl (2,4-di-*O*-benzyl-α-D-galactopyranosyl)uronate-(1→1)-2-(benzylthio)ethanol (15*):** To a stirred solution of levulinoyl ester **14*** (30 mg, 0.023 mmol) in dry CH₂Cl₂ (1.0 ml) was added at room temperature first a mixture of pyridine (56 µl, 0.692 mmol) and acetic acid (37 µl, 0.646 mmol), and then hydrazine hydrate (2 µl, 0.041 mmol). The mixture was allowed to stir for 4 h at room temperature, diluted with EtOAc (2 ml), quenched with acetone (0.1 ml) and poured into water (15 ml). The aqueous phase was extracted with EtOAc (4x10 ml), and the combined organic extracts were dried over Na₂SO₄ and concentrated under reduced pressure. The residue was purified by flash chromatography (EtOAc/hexanes 0:1 to 1:2 to 2:3) to give alcohol **15*** (28 mg, 0.023 mmol, 100%) as a clear oil. HRMS (ESI) calcd. for C₆₅H₇₂N₄O₁₇S (M+Na)⁺ 1235.4511 found 1235.4539 *m*/*z*.

**Example 14: 2-Azido-4-(benzyloxycarbonyl)amino-3-*O*-benzyloxymethyl-2,4,6-trideoxy-α-D-galactopyranosyl-(1→4)-methyl (2,3-di-*O*-benzyl-α-D-galactopyranosyl)uronate-(1 →3)-methyl (2,4-di-*O*-benzyl-α-D-galactopyranosyl)uronate-(1→1)-2-(benzylthio)ethanol (16*):** Alcohol **15*** (8.6 mg, 7.1 µmol), benzyloxymethyl thiocyclohexane (79 mg, 0.354 mmol) and TTBPy. (105 mg, 0.425 mmol) were coevaproated with dry toluene (3x10 ml) and kept under high vacuum for 30 min. The mixture was dissolved in dry CH₂Cl₂ (0.4 ml) and stirred over activated molecular sieves (3 A) for 30 min at room temperature. The mixture was cooled to 0 °C and DMTST (7.1 mg, 0.18 mmol in 0.1 ml CH₂Cl₂) was added dropwise over 45 min, while the reaction temperature was kept below 10 °C. The reaction was stirred for another 45 min, quenched by addition of a 1:1 (v/v) MeOH/Et₃N mixture and concentrated. The residue was purified by flash chromatography (EtOAc/hexanes 1:10 to 1:2) to give acetal **16*** (6.0 mg, 4.5 µmol, 64%) as a clear oil. HRMS (ESI) calcd for C₇₃H₈₀N₄O₁₈S (M+Na)⁺ 1355.5086 found 1355.5071 *m*/*z*.

**Example 15: 2-Acetamido-4-(benzyloxycarbonyl)amino-3-*O*-benzyloxymethyl-2,4,6-trideoxy-α-D-galactopyranosyl-(1→4)-methyl (2,3-di-*O*-benzyl-α-D-galactopyranosyl)uronate-(1 →3)-methyl (2,4-di-*O*-benzyl-α-D-galactopyranosyl)uronate-(1→1)-2-(benzylthio)ethanol (17*):** To a stirred solution of azide **16*** (14.0 mg, 10.5 µmol) in dry pyridine (0.35 ml) was added at 0 °C thioacetic acid (0.35 ml). The mixture was warmed to room temperature and stirred for 24 h at that temperature. The solution was co-evaporated with toluene (2x5 ml) and the residue was purified by flash chromatography (EtOAc/hexanes 1:10 to acetone/hexanes 1:7 to 1:5 to 1:3) to give acetamide **17*** (9.4 mg, 7.0 µmol, 66%) as a white solid. HRMS (ESI) calcd for C₇₅H₈₄N₂O₁₉S (M+Na)⁺ 1371.5281 found 1371.5314 *m*/*z*.

**Example 16: 2,2'-Dithiobis[2-acetamido-4-amino-2,4,6-trideoxy-α-D-galactopyranosyl-(1→4)-α-D-galactopyranosyluronate-(1→3)-α-D-galactopyranosyluronate-(1→1)-1-ethanol] (18*):** To a stirred solution of diester **17*** in THF (4.0 ml) and MeOH (0.8 ml) was added at 0 °C a 1 M solution of NaOH in water (1.5 ml). The reaction was slowly warmed to room temperature and stirred for 16 h. The reaction was diluted with EtOAc (5 ml) and acidified to pH 4 with 0.5 M aq. NaHSO₄. After separation, the aqueous fraction was extracted with EtOAc (8x10 ml), the combined organic fractions were dried over Na₂SO₄ and concentrated to give the intermediate diacid as a white solid.
To a stirring solution of liquid ammonia (5 ml) was added at -78 °C a solution of the crude diacid in THF (1.5 ml). The mixture was treated with *t*BuOH (0.5 ml) and lumps of freshly cut sodium (45 mg) were added until a deeply blue color persisted. The reaction was stirred at -78 °C for 45 min and quenched by addition of solid ammonium acetate (200 mg). The solution was warmed to room temperature under a stream of argon and co-evaporated with MeOH (2x10 ml) and water (2x5 ml). The residue was left under air for 16 h, purified by size exclusion chromatography (Sephadex G-25, 1:1 MeOH/5 mM aq. NH₄OAc) and lyophilized repeatedly to give disulfide **18*** (1.4 mg, 1.65 µmol, 32%) as a white solid. HRMS (MALDI) calcd for C₄₄H₇₀N₄O₃₂S₂ (M-H⁺) 1229.3330 found 1229.3342 *m*/*z*.

**Example 17: Methyl (ethyl 2,3-*O*-benzyl-4-*O*-levulinoyl-1-thio-β-D-galactopyranosyl)uronate (19*):** To a stirred solution of alcohol **7*** (94 mg, 0.217 mmol) in CH₂Cl₂ (1.9 mL) were added at room temperature levulinic acid (386 mg, 3.26 mmol), DCC (673 mg, 3.26 mmol) and pyridine (0.26 mL, 3.26 mmol). The mixture was stirred at that temperature for 35 h, diluted with CH₂Cl₂ (5 mL) and filtered through Celite. The mixture was concentrated, the residue was dissolved in a minimal volume of CH₂Cl₂ (1-3 mL) and filtered through cotton wool. The same procedure was repeated 3 times. The residue was purified by flash chromatography (EtOAc/toluene 1:1) to give ester **19*** (91 mg, 0.171 mmol, 79%) as a slightly yellow oil. HRMS (ESI) calcd for C₂₈H₃₄O₈S (M+Na)⁺ 553.1872 found 553.1872 *m*/*z*.

**Example 18: Methyl (2,3-di-*O*-benzyl-α-D-galactopyranosid)uronate-(1→1)-6-(benzylthio)hexanol (20*):** Thioglycoside **19*** (87 mg, 0.164 mmol), 6-(benzylthio)hexanol **21*** (85 mg, 0.379 mmol) and TTBPy. (97 mg, 0.392 mmol) were co-evaporated with anh. toluene (3x10 ml) and kept under high vacuum for 30 min. The mixture was dissolved in Et₂O (2.5 ml) and CH₂Cl₂ (0.83 ml) and stirred over activated molecular sieves (3 A) for 30 min at room temperature. The solution was cooled to 0 °C and treated with DMTST (63.5 mg, 0.246 mmol). The reaction was warmed to room temperature and stirred for 8 h at that temperature. The reaction was quenched with a 1:1 (v/v) mixture of MeOH and triethylamine (0.1 ml) and concentrated. The residue was purified by flash chromatography (EtOAc/CH₂Cl₂/hexanes 0:0:1 to 1:2:1) to give the corresponding glycosides (60 mg) as an inseparable α/β mixture.
To a stirred solution of the glycoside mixture in dry CH₂Cl₂ (2.2 ml) were added at room temperature first a mixture of pyridine (195 µl, 2.411 mmol) and acetic acid (137 µl, 2.393 mmol), and then hydrazine hydrate (5.9 µl, 0.121 mmol). The mixture was stirred for 2 h at that temperature, diluted with EtOAc (2 ml), quenched with acetone (0.1 ml) and poured into water (15 ml). The aqueous phase was extracted with EtOAc (4x10 ml), the combined organic extracts were dried over Na₂SO₄ and concentrated. The residue was purified by flash chromatography (EtOAc/hexanes 1:2) to give alcohol **20*** (29 mg, 0.049 mmol, 30% over two steps) as a clear oil, along with the corresponding β-isomer (22 mg, 0.037 mmol, 22%). HRMS (ESI) calcd. for C₃₄H₄₂O₇S (M+Na)⁺ 617.2544 found 617.2542 *m*/*z*.

**Example 19: 6,6'-Dithiobis[α-D-galactopyranosyluronate-(1→1)-1-hexanol] (22*):** To a stirred solution of ester **20*** (10 mg, 0.017 mmol) in THF (1.0 ml) and MeOH (0.5 ml) was added at 0 °C a 1 M solution of NaOH in water (0.8 ml). The reaction was slowly warmed to room temperature and stirred for 16 h. The reaction was diluted with EtOAc (5 ml) and water (5 ml) and acidified to pH 4 with 0.5 M aq. NaHSO₄. After separation, the aqueous fraction was extracted with EtOAc (8x5 ml), the combined organic fractions were dried over Na₂SO₄ and concentrated to give the intermediate acid as a white solid.
To a stirred solution of liquid ammonia (8 ml) was added at -78 °C a solution of the crude diacid in THF (2 ml). The mixture was treated with *t*BuOH (0.4 ml) and lumps of freshly cut sodium (45 mg) were added until a deeply blue color persisted. The reaction was stirred at -78 °C for 45 min and quenched by addition of solid ammonium acetate (100 mg). The solution was warmed to room temperature under a stream of argon and co-evaporated with MeOH (2x10 ml) and water (2x5 ml). The residue was left under air for 16 h, purified by size exclusion chromatography (Sephadex G-25, 9:1 MeOH/5 mM aq. NH₄OAc) and lyophilized repeatedly to give disulfide **22*** (3.1 mg, 5.1 µmol, 60% over two steps) as a white solid. HRMS (MALDI) calcd for C₂₄H₄₂O₁₄S₂ (M-H⁺) 617.1938 found 617.1954 *m*/*z*.

**Example 20: 2-Acetamido-4-(benzyloxycarbonyl)amino-3-*O*-levulinoyl-2,4,6-trideoxy-α-D-galactopyranosyl-(1→1)-6-(benzylthio)hexanol (23*):** 6-(Benzylthio)hexanol **21*** (29 mg, 0.171 mmol) and glycosyl phosphate **2*** (70 mg, 0.114 mmol) were co-evaproated with dry toluene (3x10 ml) and kept under high vacuum for 30 min. The mixture was dissolved in CH₂Cl₂ (1.8 ml) and stirred over activated molecular sieves (4 A-AW) for 1 h at room temperature. The solution was then cooled to 0 °C and treated with TMSOTf (31 µl, 0.171 mmol in 0.2 ml dry CH₂Cl₂). The mixture was stirred for 3 h at that temperature, quenched with a 1:1 (v/v) mixture of MeOH and triethylamine (0.5 ml), diluted with CH₂Cl₂ (20 ml) and filtered through Celite. The residue was purified by flash chromatography (EtOAc/hexanes 2:3 to 3:2) to give the corresponding glycosides (57 mg) as an inseparable α/β mixture.
To a stirred solution of the glycoside mixture in dry pyridine (0.9 ml) was added at 0 °C thioacetic acid (0.9 ml). The mixture was warmed to room temperature and stirred for 24 h at that temperature. The solution was co-evaporated with toluene (2x5 ml) and the residue was purified by flash chromatography (EtOAc/hexanes 1:2 to 2:1 to 6:1) to give acetamide **23*** (22 mg, 0.034 mmol, 29% over two steps) as a white solid, along with the corresponding β-isomer (21.6 mg, 0.034 mmol, 29%). HRMS (ESI) calcd for C₃₄H₄₆N₂O₈S (M+Na)⁺ 665.2872 found 665.2865 *m*/*z*.

**Example 21: 6,6'-Dithiobis[2-acetamido-4-amino-2,4,6-trideoxy-α-D-galactopyranosyl-(1→1)-1-hexanol] (24*):** To a stirred solution of ester **23*** (10 mg, 0.016 mmol) in dry CH₂Cl₂ (1.0 ml) were added at room temperature first a mixture of pyridine (38 µl, 0.467 mmol) and acetic acid (24.9 µl, 0.436 mmol), and then hydrazine hydrate (1.0 µl, 0.020 mmol). The mixture was stirred for 2 h at that temperature, quenched with acetone (0.1 ml) and purified by size exclusion chromatography (Sephadex LH-20, CH₂Cl₂/MeOH 2:1) to give the corresponding alcohol as a clear oil.
To a stirred solution of liquid ammonia (5 ml) was added at -78 °C a solution of the intermediate alcohol in THF (1.2 ml). The mixture was treated with *t*BuOH (0.5 ml) and lumps of freshly cut sodium (80 mg) were added until a deeply blue color persisted. The reaction was stirred at -78 °C for 45 min and quenched by addition of solid ammonium acetate (100 mg). The solution was warmed to room temperature under a stream of argon and co-evaporated with MeOH (2x10 ml) and water (2x5 ml). The residue was left under air for 16 h, purified by size exclusion chromatography (Sephadex G-25, 9:1 MeOH/5 mM aq. NH₄OAc) and lyophilized repeatedly to give disulfide **24*** (1.7 mg, 2.7 µmol, 33% over two steps) as a white solid. HRMS (ESI) calcd. for C₂₈H₅₄N₄O₈S₂ (M+Na)⁺ 661.3281 found 661.3306 *m*/*z*.

**Example 22: 2-Acetamido-4-(benzyloxycarbonyl)amino-3-*O*-levulinoyl-2,4,6-trideoxy-α-D-galactopyranosyl-(1→1)-2-(benzylthio)ethanol (25*):** 2-(benzylthio)ethanol **11*** (71 mg, 0.421 mmol) and glycosyl phosphate **2*** (171 mg, 0.281 mmol) were co-evaproated with dry toluene (3x10 ml) and kept under high vacuum for 30 min. The mixture was dissolved in CH₂Cl₂ (1.8 ml) and stirred over activated molecular sieves (4 A-AW) for 1 h at room temperature. The solution was then cooled to -40 °C and treated with TMSOTf (56 µl, 0.309 mmol in 0.2 ml dry CH₂Cl₂). The mixture was slowly warmed to 0 °C (2 h), quenched with a 1:1 (v/v) mixture of MeOH and triethylamine (0.5 ml), diluted with CH₂Cl₂ (20 ml), filtered through Celite and concentrated. The residue was purified by flash chromatography (EtOAc/hexanes 1:3 to 1:1) to give the corresponding α-glycoside (55 mg, 0.096 mmol, 34%) along with the corresponding β-glycoside (22 mg, 0.039 mmol, 14%). To a stirred solution of the intermediate α-glycoside (40 mg, 0.070 mmol) in dry pyridine (0.4 ml) was added at 0 °C thioacetic acid (0.4 ml). The mixture was warmed to room temperature and stirred for 24 h at that temperature. The solution was co-evaporated with toluene (2x5 ml) and the residue was purified by flash chromatography (EtOAc/hexanes 1:3 to acetone/hexanes 1:2 to 2:3) to give acetamide **25*** (31 mg, 0.053 mmol, 76%) as a white solid. HRMS (ESI) calcd for C₃oH₃₈N₂O₈S (M+Na)⁺ 609.2246 found 609.2256 m/z.

**Example 23: 6,6'-Dithiobis[2-acetamido-4-amino-2,4,6-trideoxy-α-D-galactopyranosyl-(1→1)-1-hexanol] (26*):** To a stirred solution of ester **25*** (20.7 mg, 0.035 mmol) in dry CH₂Cl₂ (3.0 ml) were added at room temperature first a mixture of pyridine (86 µl, 1.058 mmol) and acetic acid (57 µl, 0.988 mmol), and then hydrazine hydrate (3.4 µl, 0.071 mmol). The mixture was stirred for 5 h at that temperature, diluted with EtOAc (2 ml), quenched with acetone (0.1 ml) and poured into water (10 ml). The aqueous phase was extracted with EtOAc (4x5 ml), the combined organic fractions were dried over Na₂SO₄ and concentrated. The residue was purified by flash chromatography (acetone/hexanes 1:1) to give the intermediate alcohol (17.5 mg) as a white solid.
To a stirred solution of liquid ammonia (6 ml) was added at -78 °C a solution of the intermediate alcohol in THF (1.5 ml). The mixture was treated with *t*BuOH (0.5 ml) and lumps of freshly cut sodium (45 mg) were added until a deeply blue color persisted. The reaction was stirred at -78 °C for 45 min and quenched by addition of solid ammonium acetate (100 mg). The solution was warmed to room temperature under a stream of argon and co-evaporated with MeOH (2x10 ml) and water (2x5 ml). The residue was left under air for 16 h, purified by size exclusion chromatography (Sephadex G-25, 1:10 MeOH/5 mM aq. NH₄OAc) and lyophilized repeatedly to give disulfide **26*** as the diacetate salt (7.91 mg, 12.3 µmol, 70% over two steps) as a white solid. HRMS (ESI) calcd. for C₂₀H₃₈N₄O₈S₂ (M+Na)⁺ 549.2029 found 549.2086 m/z.

**Example 24: 2,2'-Dithiobis[α-D-galactopyranosyluronate-(1→3)-α-D-galactopyranosyluronate-(1→1)-1-ethanol] (27*):** To a stirred solution of ester **13*** (8.6 mg, 9.5 µmol) in THF (0.6 ml) and MeOH (0.3 ml) was added at 0 °C a 1 M solution of NaOH in water (0.5 ml). The reaction was slowly warmed to room temperature and stirred for 16 h. The reaction was diluted with EtOAc (5 ml) and water (5 ml) and acidified to pH 4 with 0.5 M aq. NaHSO₄. After separation, the aqueous fraction was extracted with EtOAc (8x5 ml), the combined organic fractions were dried over Na₂SO₄ and concentrated to give the intermediate diacid as a white solid.
To a stirred solution of liquid ammonia (6 ml) was added at -78 °C a solution of the crude diacid in THF (1.5 ml). The mixture was treated with *t*BuOH (0.4 ml) and lumps of freshly cut sodium (75 mg) were added until a deeply blue color persisted. The reaction was stirred at -78 °C for 45 min and quenched by addition of solid ammonium acetate (100 mg). The solution was warmed to room temperature under a stream of argon and co-evaporated with MeOH (2x10 ml) and water (2x5 ml). The residue was left under air for 16 h, purified by size exclusion chromatography (Sephadex G-25, 1:9 MeOH/5 mM aq. NH₄OAc) and lyophilized repeatedly to give disulfide **27*** (2.5 mg, 2.9 µmol, 61 % over two steps) as a white solid. HRMS (MALDI) calcd for C₂₈H₄₂O₂₆S₂ (M-H⁺) 901.0966 found 901.0981 m/z.

### Synthesis of glycoconjugates

**Example 25: Conjugation of saccharides of general formula (I) to CRM₁₉₇:** To a stirred solution of CRM₁₉₇ (1 mg, 17.2 nmol) in 0.1 M sodium phosphate buffer (NaPi) pH 7.4 (1 ml) was added at room temperature a solution of succinimidyl-3-(bromoacetamido)propionate (SBAP) (264 µg, 863 nmol) in DMF (20 µl). The mixture was stirred for 1 h at that temperature, and concentrated using membrane filtration (Amicon Ultra centrifuge membranes, 10 kDa cut-off). The protein solution was diluted with 0.1 M NaPi pH 7.4 and concentrated again. This process was repeated three times and the solution was diluted to 1 ml using 0.1 M NaPi pH 7.4. The intermediate of general formula (**II**) (690 mmol) in 120 µl 0.1 M NaPi pH 7.4 was treated at room temperature with tris(2-carboxyethyl)phosphine (TCEP) (690 mmol), left for 1 h at that temperature under an argon atmosphere and added to the solution of the bromoacetamido-modified CRM₁₉₇ protein at room temperature. The mixture was left at room temperature for 2 h and then at 4 °C for 16 h, and purified using membrane filtration (see above). The purified glycoconjugate in 0.1 M NaPi pH 7.4 (1 ml) was then treated at room temperature with L-cysteine (417 µg, 3.45 µmol) in 100 µl water. The mixture was left for 2 h at that temperature and purified by membrane filtration. Incorporation of the saccharide of general formula (**I**) into the glycoconjugate was assessed by MALDI-TOF-MS, SDS-PAGE and size exclusion chromatography with right angle light scattering detection (SEC-RALS).

### Example 26: In flow conjugation of the saccharides of general formula (I) to a glycosphingolipid with immunomodulatory properties

By using a photochemical flow reactor *(*Chem. Eur. J. 2013, 19, 3090) that was fitted with a loop of Teflon AF2400 tubing (566 µL), a solution of saccharide of general formula (I) (1.5 equiv.) in water (300 µL) was reacted with pentenyl modified (2*S*,3*S*,4*R*)-1-(α-D-galactopyranosyl)-2-hexacosanoylaminooctadecane-3,4-diol (1 equiv.) in water (300 µL) and AcOH (8 µL; residence time: 10 min, flow rate: 28.3 µL/min⁻¹ per syringe). The reactor output was lyophilized and the crude material was purified using size exclusion chromatography (Sephadex-G25, 5% EtOH in water, 10 mmx150 mm) to yield the glycoconjugate of saccharide of general formula (I) covalently linked to the modified (2*S*,3*S*,4*R*)-1-(α-D-galactopyranosyl)-2-hexacosanoylaminooctadecane-3,4-diol as white solid.

### Example 27: Conjugation of 2-acetamido-4-amino-2,4,6-trideoxy-α-D-galactopyranosyl-(1→4)-(α-D-galactopyranosyl)uronate-(1→3)-(α-D-galactopyranosyl)uronate-(1→1)-2-(thio)ethanol to BSA

To a stirred solution of BSA (0.5 mg, 7.6 nmol) in 0.1 M sodium phosphate buffer (NaPi) pH 7.4 (1 mL) was added at room temperature a solution of N-succinimidyl-3-(bromoacetamido)propionate (SBAP) (89 µg, 290 nmol) in DMF (20 µL). The mixture was stirred for 1 h at room temperature, and concentrated using membrane filtration (Amicon 0.5 mL Ultra centrifuge membranes, 10 kDa cut-off). The protein solution was diluted with 0.1 M NaPi pH 7.4 and concentrated again. This process was repeated three times and the solution was diluted to 0.5 mL using water. 20 µL were taken for analysis, and the protein solution was re-buffered to 0.1 M NaPi pH 7.4 using membrane filtration. Disulfide **18*** (140 µg, 228 nmol resp. to the monomer) in 120 µL 0.1 M NaPi pH 7.4 was treated at room temperature with tris(2-carboxyethyl)phosphine (TCEP) (250 nmol), left for 1 h at that temperature under an argon atmosphere and added to the solution of the activated protein at room temperature. The mixture was left at 4 °C for 16 h, and purified using membrane filtration (see above). After washing with water and diluting to 0.5 mL, another analytical sample (20 µL) was taken, and the solution was re-buffered. The purified glycoconjugate in 0.1 M NaPi pH 7.4 (0.5 mL) was then treated at room temperature with L-cysteine (417 µg, 3.45 µmol) in 100 µl water. The mixture was left for 2 h at that temperature and purified by membrane filtration. Incorporation of 2-acetamido-4-amino-2,4,6-trideoxy-α-D-galactopyranosyl-(1→4)-(α-D-galactopyranosyl)uronate-(1→3)-(α-D-galactopyranosyl)uronate-(1→1)-2-(thio)ethanol into the glycoconjugate was assessed by MALDI-TOF-MS (positive mode):
Molecular weight measured:
   BSA: 66341 m/z.
   BSA-SBAP conjugate: 68316 m/z (incorporation of approximately 10 SBAP groups).
   BSA-SBAP glycoconjugate: 69101 m/z (incorporation of approximately 1.3 molecules of 2-acetamido-4-amino-2,4,6-trideoxy-α-D-galactopyranosyl-(1→4)-(α-D-galactopyranosyl)uronate-(1→3)-(α-D-galactopyranosyl)uronate-(1→1)-2-(thio)ethanol).
   BSA-SBAP glycoconjugate after quenching with L-cysteine: 72074 m/z (incorporation of approx. 24.5 L-cysteine molecules).

### Example 28: Conjugation of 2-acetamido-4-amino-2,4,6-trideoxy-α-D-galactopyranosyl-(1→4)-(α-D-galactopyranosyl)uronate-(1→3)-(α-D-galactopyranosyl)uronate-(1→1)-2-(thio)ethanol to BSA

To a stirred solution of BSA (0.5 mg, 7.6 nmol) in 0.1 M sodium phosphate buffer (NaPi) pH 7.4 (1 mL), a solution of N-Succinimidyl-3-maleimidopropionate (101 µg, 380 nmol) in DMF (20 µL) was added at room temperature. The mixture was stirred for 1 h at room temperature, and concentrated using membrane filtration (Amicon 0.5 mL Ultra centrifuge membranes, 10 kDa cut-off). The protein solution was diluted with 0.1 M NaPi pH 7.4 and concentrated again. This process was repeated three times and the solution was diluted to 0.5 mL using water. 20 µL were taken for analysis, and the protein solution was re-buffered to 0.1 M NaPi pH 7.4 using membrane filtration. Disulfide **18*** (140 µg, 228 nmol resp. to the monomer) in 120 µl 0.1 M NaPi pH 7.4 was treated at room temperature with tris(2-carboxyethyl)phosphine (TCEP) (250 nmol), left for 1 h at that temperature under an argon atmosphere and added to the solution of the activated protein at room temperature. The mixture was left at 4 °C for 16 h, and purified using membrane filtration (see above). After washing with water and diluting to 0.5 mL, another analytical sample (20 µL) was taken, and the solution was re-buffered. The purified glycoconjugate in 0.1 M NaPi pH 7.4 (0.5 mL) was then treated at room temperature with L-cysteine (417 µg, 3.45 µmol) in 100 µl water. The mixture was left for 2 h at that temperature and purified by membrane filtration. Incorporation of glycan into the glycoconjugate was assessed by MALDI-TOF-MS (positive mode):
Molecular weight measured:
   BSA: 66341 *m*/*z*;
   BSA-maleimide conjugate: 69254 *m*/*z* (incorporation of approximately 19 maleimide groups);
   BSA-maleimide glycoconjugate: 71340 *m*/*z* (incorporation of approximately 3.4 molecules of **2-acetamido-4-amino-2,4,6-trideoxy-α-D-galactopyranosyl-(1→4)-(α-D-**galactopyranosyl)uronate-(1→3)-(α-D-galactopyranosyl)uronate-(1→1)-2-(thio)ethanol);
   BSA-maleimide glycoconjugate after quenching with L-cysteine: 72106 *m*/*z* (incorporation of approx. 6.3 L-cysteine molecules).

### Conjugation to a solid support: synthesis of microarrays coated with saccharides of general formula (I)

### Example 29: Synthesis of microarrays using GAPSII slides

Maleimide-functionalized microarrays were produced by submerging amine-coated slides (GAPS II slides, Corning) in 6-maleimidohexanoic acid *N*-hydroxysuccinimide ester (2 mM) in dry DMF with diisopropylethylamine (2.5% v/v) for 24 h at room temperature. Slides were washed three times with water and three times with ethanol, centrifuged to dryness, and stored under argon until spotting. Diluted saccharides of general formula (I) were printed onto the modified microarray slides at 0.4 nL per spot by an automatic piezoelectric arraying robot (Scienion, Berlin, Germany). For completion of the immobilization reaction, printed slides were stored for 24 h in a humidified chamber.

Microarray slides were washed three times with water. Unreacted maleimide was quenched by submerging the slides in β-mercaptoethanol (0.1%, v/v) in PBS for 1 h at room temperature. Slides were washed three times with water and with ethanol, centrifuged to dryness, and blocked with BSA (1%, w/v) in PBS for 1 h at room temperature. Blocked slides were washed (2x PBS, 3 x water), centrifuged, and incubated with the sera dilutions.

### Example 30: Synthesis of microarrays using CodeLink NHS slides

CodeLink NHS slides were incubated for 24 h (1% w/v in PBS) at 4 °C. Slides were incubated in blocking buffer (100 mM ethanolamine in 50 mM NaPi pH > 9) for 30 min at room temperature, washed three times each with water and ethanol, and dried. Slides were then subjected to maleimide functionalization and printing (see **Example 29).**

### Example 31: Binding experiments using the microarrays synthesized according to the procedure described at examples 29 and 30.

Binding experiments were performed by incubating microarray slides coated with the saccharides of general formula (I) with either a rabbit anti-SP1 typing serum or human pneumococcal reference serum 007sp (pooled sera of 287 humans immunized with Pneumovax vaccine) in the dilutions indicated in the presence or absence of native SP1 polysaccharide, and using fluorescently labeled anti-rabbit or anti-human secondary antibodies.

## Claims

1. Saccharide of general formula (I): 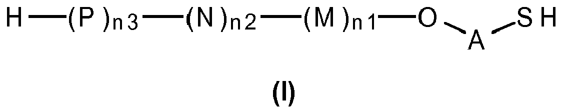 wherein A is a linker;
M, N and P represent independently of each other one of the following sugar fragments: 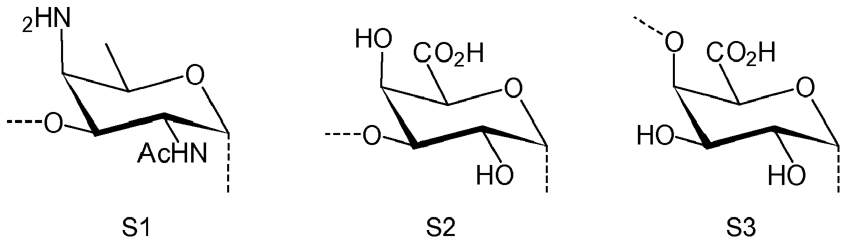 wherein each moiety S1, S2, and S3 is not more than once present in the general formula (I) sugar fragment S1 cannot be simultaneously bound to -O-A-SH and sugar fragment S2, sugar fragment S3 cannot be simultaneously bound to -O-A-SH and sugar fragment S1 and sugar fragment S2 cannot be simultaneously bound to -O-A-SH and sugar fragment S3 and
n1, n2 and n3 are integers selected from 0 and 1
wherein at least one of the integers n1, n2 and n3 is 1 and
and pharmaceutically acceptable salts of these saccharides.

2. Synthesis of the saccharide of the general formula (**I**): 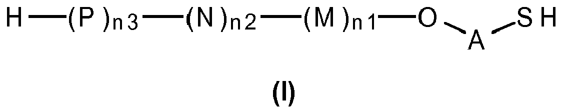 wherein A is a linker;
M, N and P represent independently of each other one of the following sugar fragments: 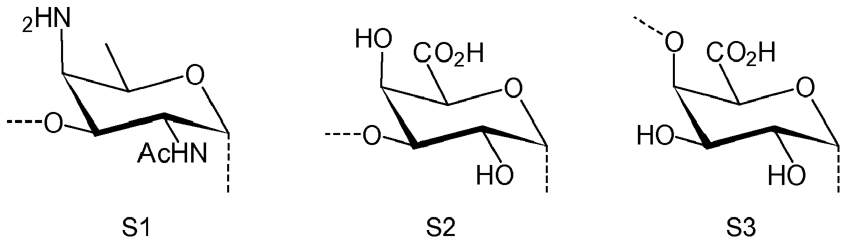 wherein each moiety S1, S2, and S3 is not more than once present in the general formula (I), sugar fragment S1 cannot be simultaneously bound to -O-A-SH and sugar fragment S2, sugar fragment S3 cannot be simultaneously bound to -O-A-SH and sugar fragment S1 and sugar fragment S2 cannot be simultaneously bound to -O-A-SH and sugar fragment S3, and
n1, n2 and n3 are integers selected from 0 and 1
wherein at least one of the integers n1, n2 and n3 is 1 and
comprising the steps:
A1) Reacting the compound **2** of the formula: 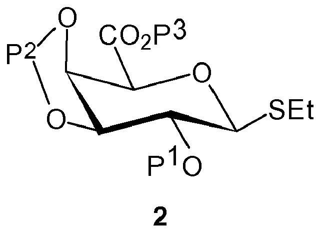 wherein P¹ - P³ represent protecting groups, with the compound 3 of the formula 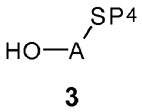 wherein P⁴ represents a protecting group, in order to obtain compound **4** of general formula: 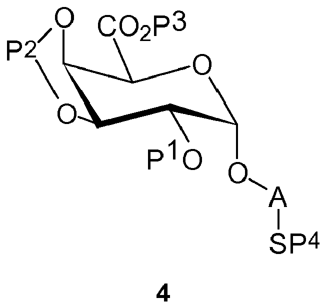 wherein P¹-P⁴ and A are defined as above;
and
performing removal of protecting groups P¹-P⁴ on compound **4** to afford monosaccharide disulfide 5 of general formula: 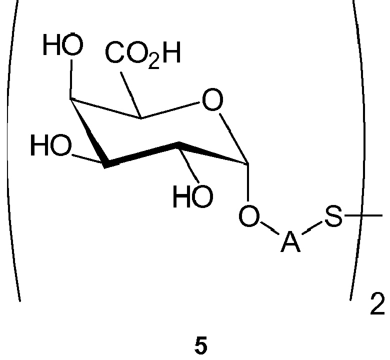 wherein A is defined as above, and wherein monosaccharide disulfide 5 is further treated with a reducing agent to afford monosaccharide 6 of general formula: 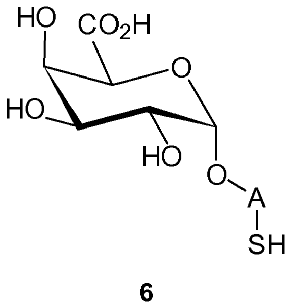 wherein A is defined as above;
or
performing selective deprotection on compound **4** to afford compound 7 of general formula 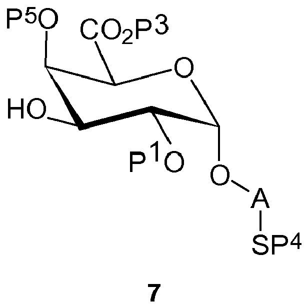 wherein P⁵ is a protecting group and P¹, P³, P4 and A are defined as above. or
A2) Reacting compound 8 of general formula 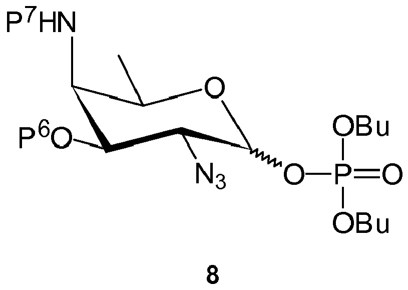 wherein P⁶ and P⁷ represent protecting groups, with compound **3** to afford compound 9 of general formula 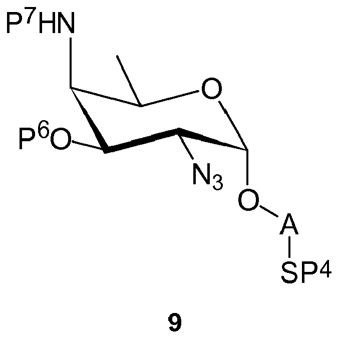 wherein P⁶, P⁷ and A are defined as above;
and
performing conversion of the azido group to acetamide and removal of the protecting groups P⁴, P⁶ and P⁷ on compound **9** to afford monosaccharide disulfide **10** of general formula: 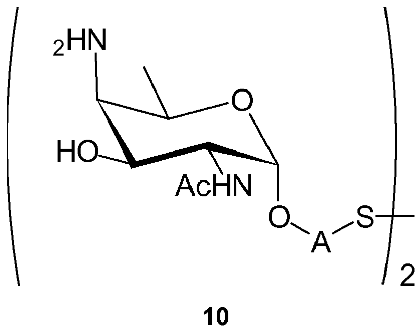 wherein A is defined as above and, wherein monosaccharide disulfide 10 is further treated with a reducing agent to afford monosaccharide **11** of general formula: 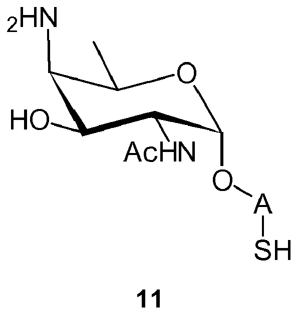 wherein A is defined as above;
or
performing selective deprotection on compound 9 to afford compound 12 of general formula: 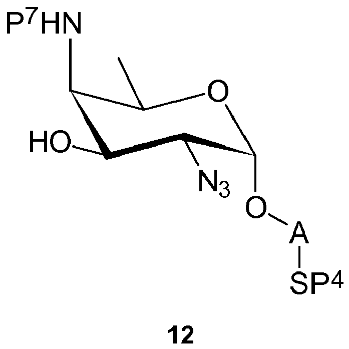 wherein P⁴, P⁷ and A are defined as above.
or
A3) Reacting compound 13 of general formula 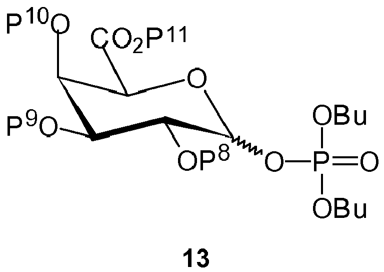 wherein P⁸ - P¹¹ represent protecting groups, with compound 3 to afford compound **14** of general formula: 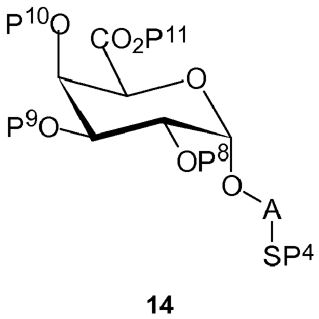 wherein P⁴, P⁸ - P¹¹ are defined as above
and
performing selective deprotection of compound **14** to afford compound 15 of general formula: 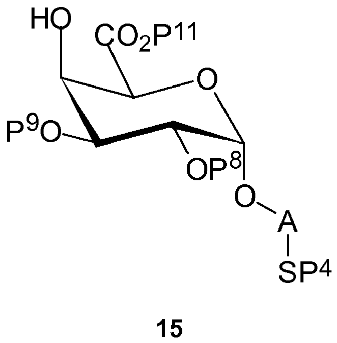 wherein P⁴, P⁸, P⁹, P¹¹ and A are defined as above.
and
B1) Reacting compound 7 with compound 13 to afford compound 16 of general formula: 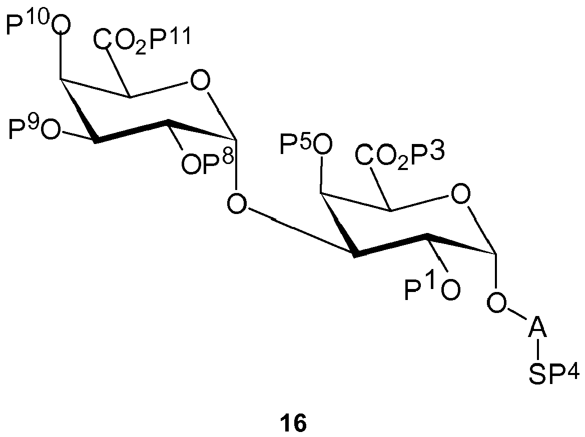 wherein P¹, P³-P⁵, P⁸-P¹¹ and A are defined as above;
and
performing removal of protecting groups P¹, P³-P⁵, P⁸-P¹¹ on compound **16** to afford disaccharide disulfide **17** of general formula: 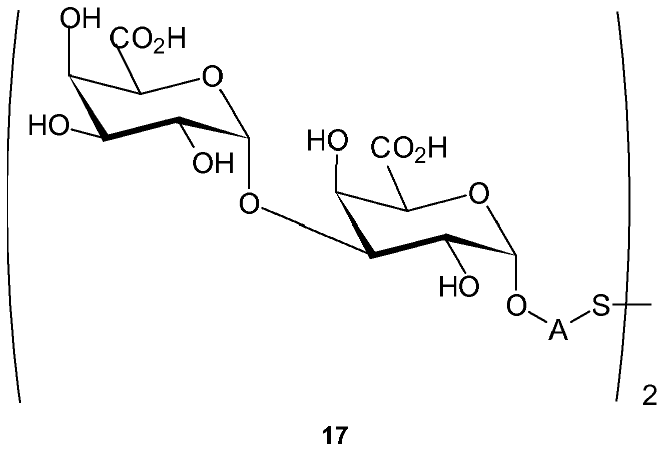 wherein A is defined as above and wherein disaccharide disulfide **17** is further treated with a reducing agent to afford disaccharide 18 of general formula: 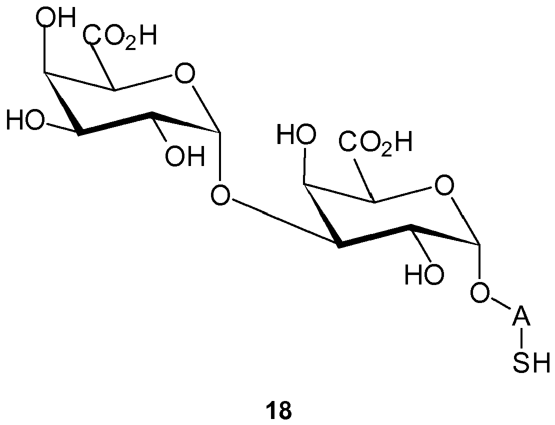 wherein A is defined as above;
or
performing selective removal of protecting group P¹⁰ on compound **16** to afford compound **19** of general formula: 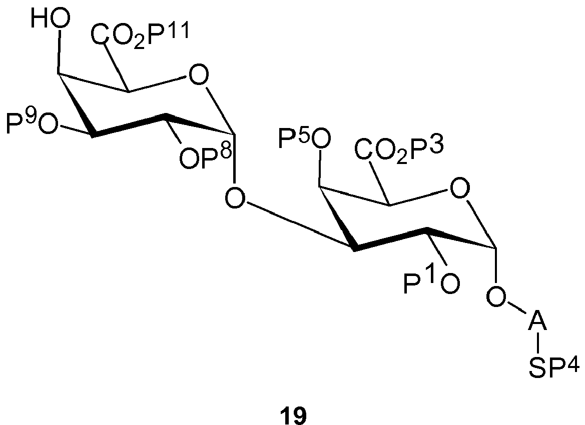 wherein P¹, P³-P⁵, P⁸, P⁹, P¹¹ and A are defined as above.
or
B2) Reacting compound **15** with compound 8 to afford compound **20** of general formula: 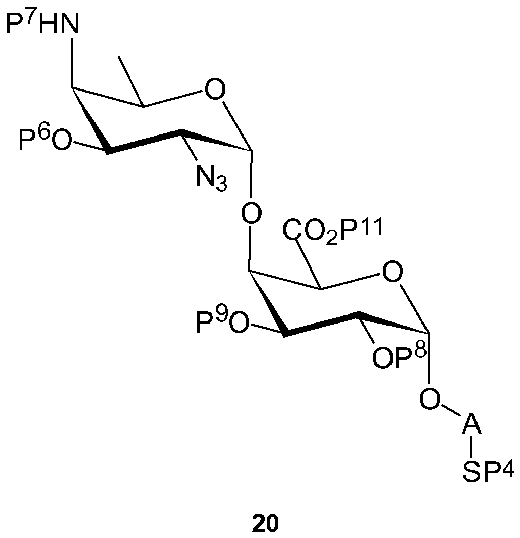 wherein P⁴, P⁶-P⁹, P¹¹ and A are defined as above
and
performing conversion of the azido group to acetamide and removal of the protecting groups P⁴, P⁶-P⁹, P¹¹ on compound **20** to afford disaccharide disulfide **21** of general formula: 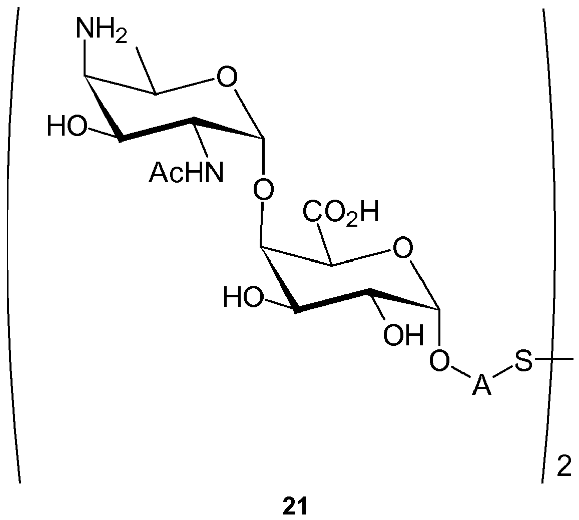 wherein A is defined as above and wherein disaccharide disulfide **21** is treated with a reducing agent to afford disaccharide **22** of general formula: 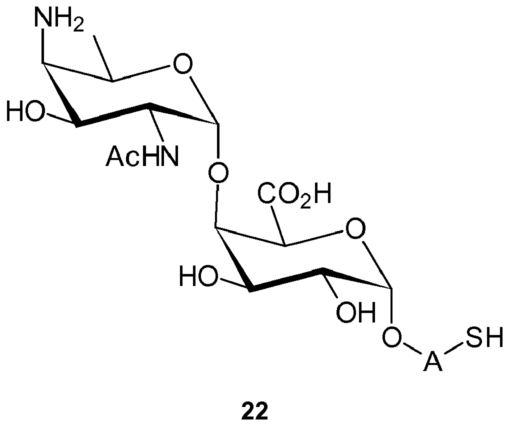 wherein A is defined as above;
or
performing selective removal of protecting group P⁶ on compound **20** to afford compound **23** of general formula: 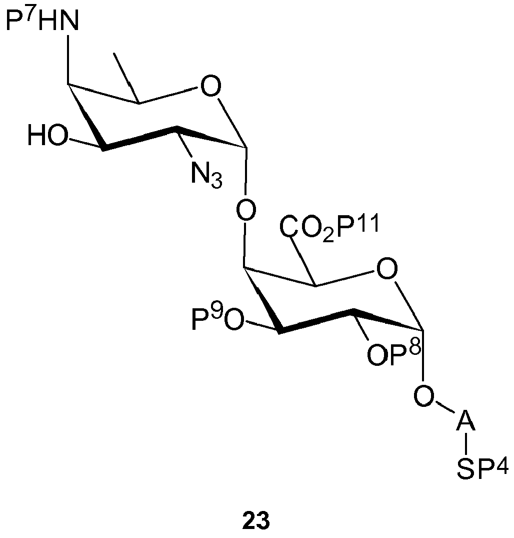 wherein P⁴, P⁷-P⁹, P¹¹ and A are defined as above;
or
B3) Reacting compound **12** with compound **2** to afford compound **24** of general formula: 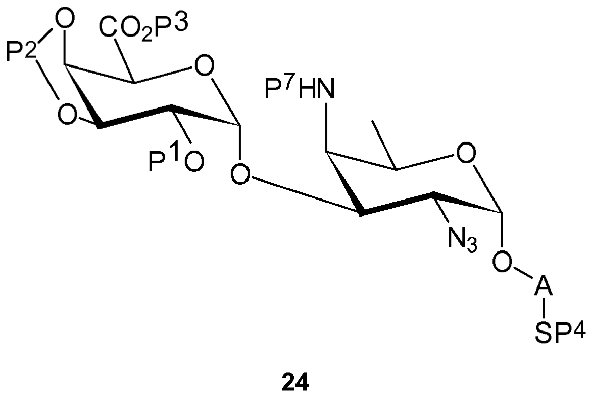 wherein P¹-P⁴, P⁷ and A are defined as above,
**and**
performing conversion of the azido group to acetamide and removal of protecting groups P¹-P⁴ and P⁷ on compound **24** to afford disaccharide disulfide **25** of general formula: 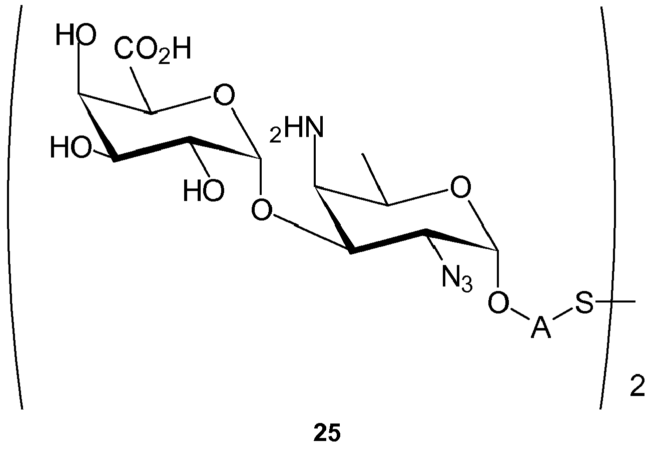 wherein A is defined as above, and wherein disaccharide disulfide **25** is further treated with a reducing agent to afford disaccharide **26** of general formula: 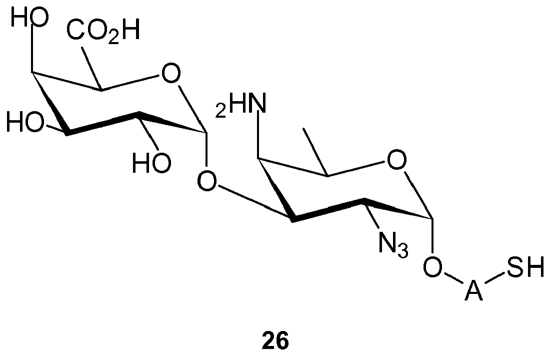 wherein A is defined as above;
or
performing selective deprotection on compound **24** to afford compound **27** of general formula: 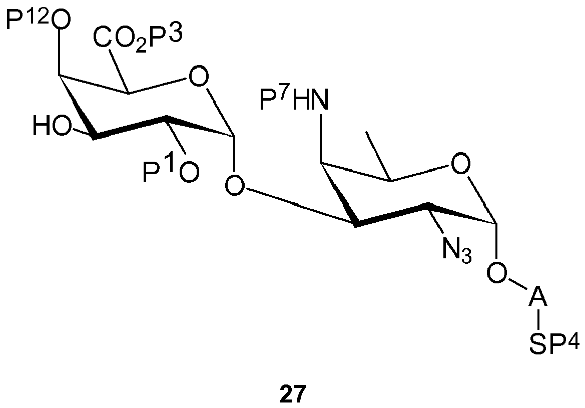 wherein P¹² is a protecting group and P¹, P³, P⁴, P⁷ and A are defined as above. and
C1) Reacting compound **19** with compound **8** to afford compound **28** of general formula: 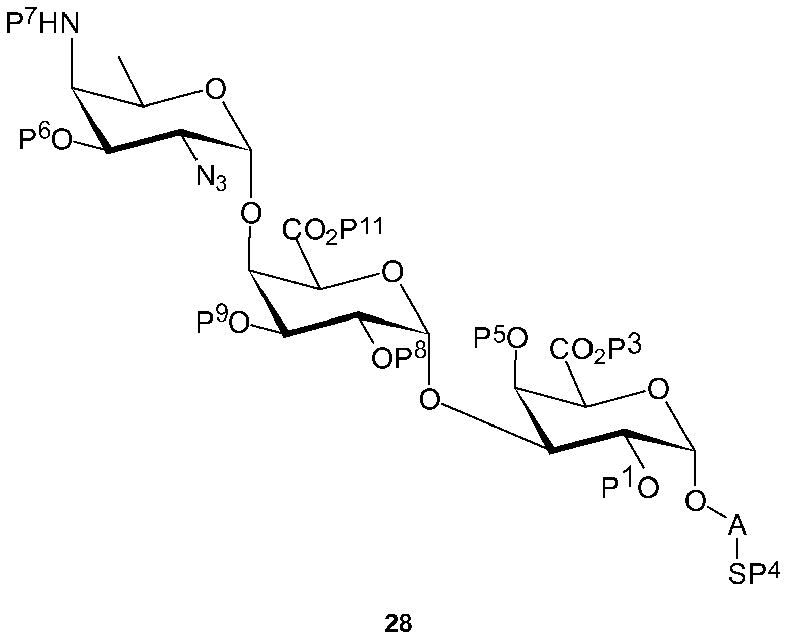 wherein P¹, P³-P⁹, P¹¹ and A are defined as above;
and
wherein protecting group P⁶ is replaced with protecting group P¹³ in order to obtain compound **29** of the following chemical formula: 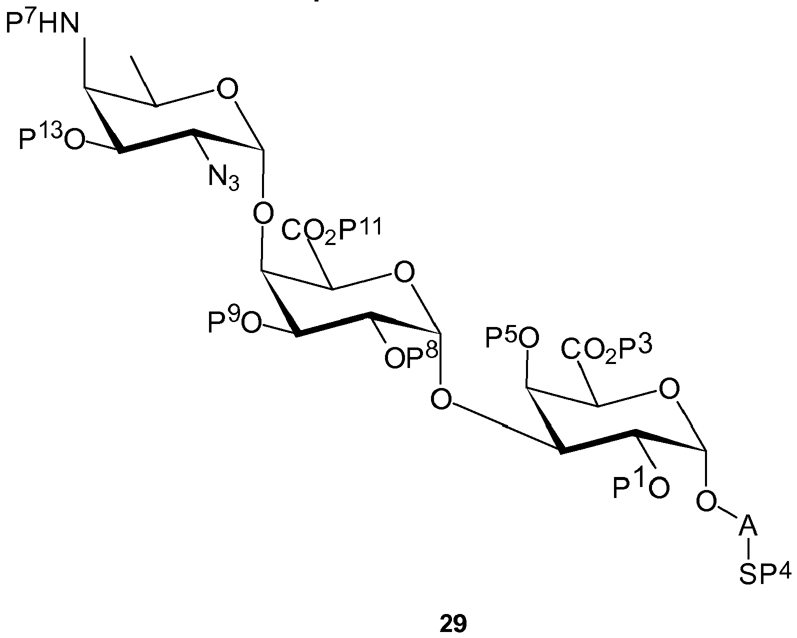 wherein P¹, P³-P⁵, P⁷-P⁹, P¹¹, P¹³ and A are defined as above;
and
conversion of compound **29** to trisaccharide disulfide **30** by conversion of the azide group in the acetamide group and cleavage of the protecting group P¹, P³-P⁵, P⁷-P⁹, P¹¹, P¹³, wherein compound **30** is of general formula: 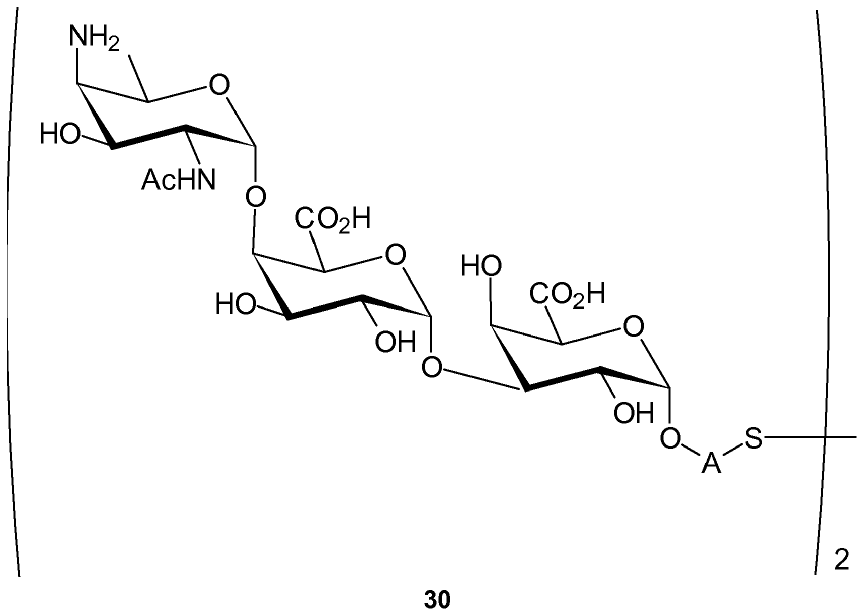 and wherein A is defined as above;
and
conversion of trisaccharide disulfide **30** to trisaccharide **31** by treatment with a reducing agent, wherein compound **31** is of general formula: 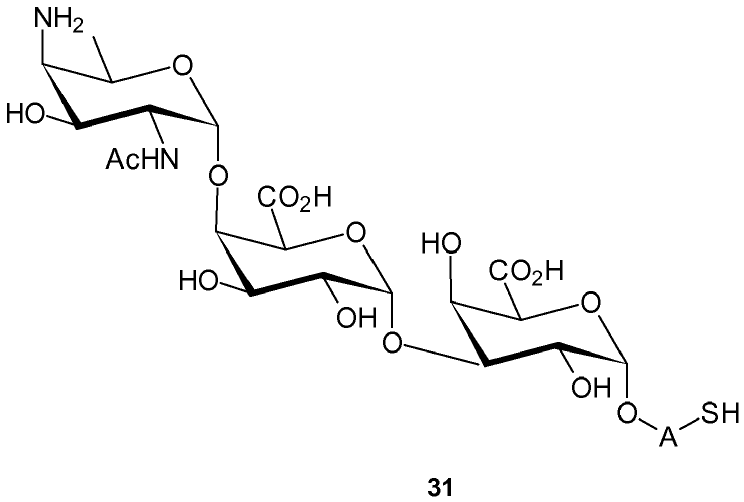 and wherein A is defined as above.
or
C2) Reacting compound **23** with compound **2** to afford compound **32** of general formula: 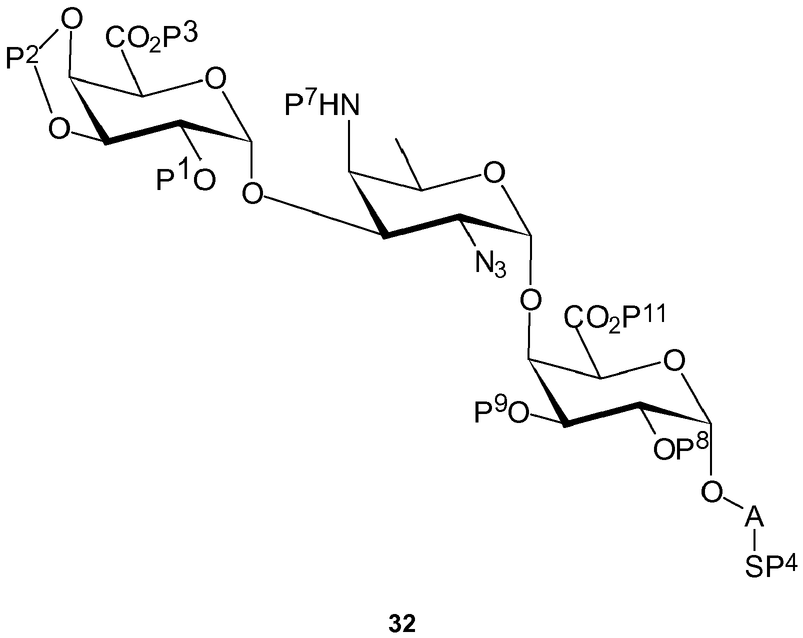 wherein P¹ - P⁴, P⁷ - P⁹, P¹¹ and A are defined as above;
and
conversion of compound **32** to trisaccharide disulfide **33** by conversion of the azide group in the acetamide group and cleavage of the protecting group P¹ - P⁴, P⁷ - P⁹, P¹¹, wherein compound **33** is of general formula: 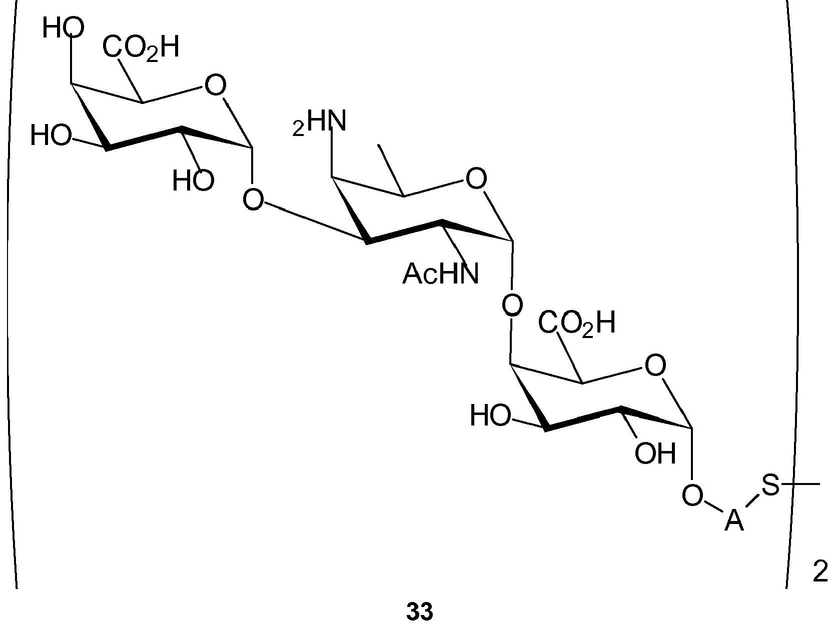 wherein A is defined as above;
and
conversion of trisaccharide disulfide **33** to trisaccharide **34** by treatment with a reducing agent, wherein compound **34** is of general formula: 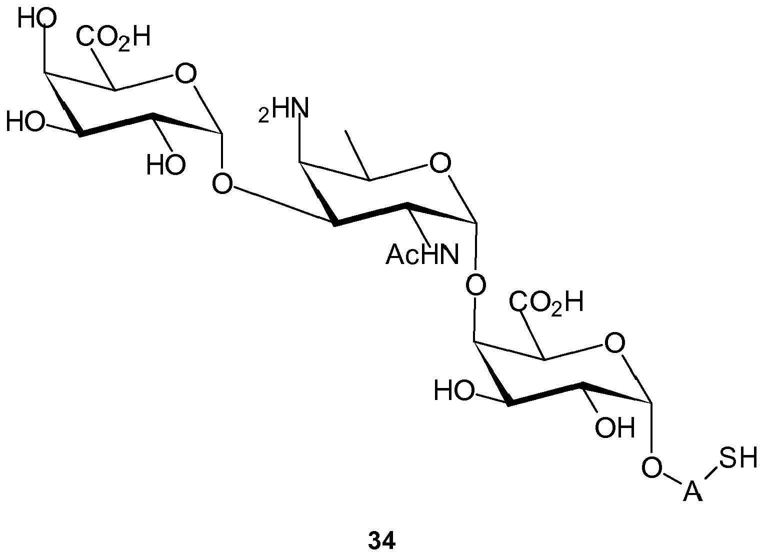 wherein A is defined as above;
or
C3) Reacting compound **27** with compound **13** to afford compound **34** of general formula: 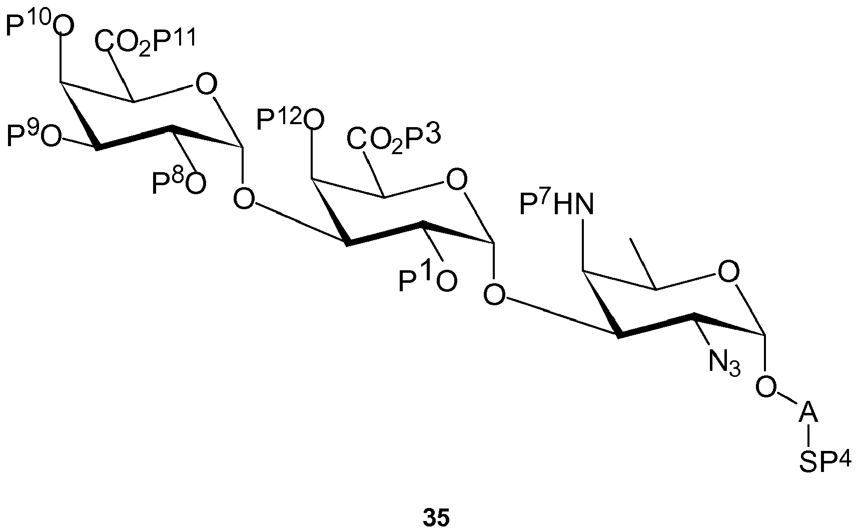 wherein P¹, P³, P⁴, P⁷-P¹¹ and A are defined as above;
and
conversion of compound **35** to trisaccharide disulfide **36** by conversion of the azide group in the acetamide group and cleavage of the protecting group P¹, P³, P⁴, P⁷-P¹¹, wherein compound **36** is of general formula: 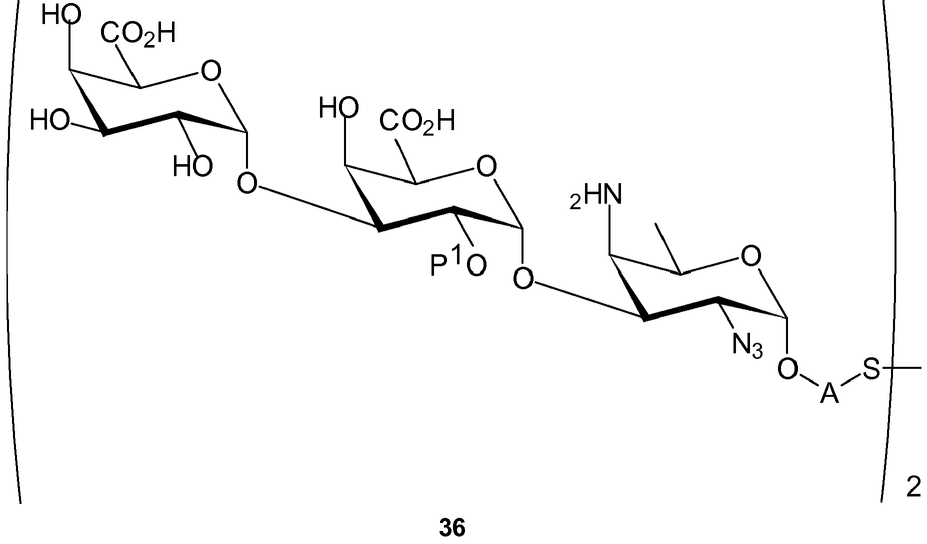 wherein A is defined as above;
and
conversion of trisaccharide disulfide **36** to trisaccharide **37** by treatment with a reducing agent, wherein compound **37** is of general formula: 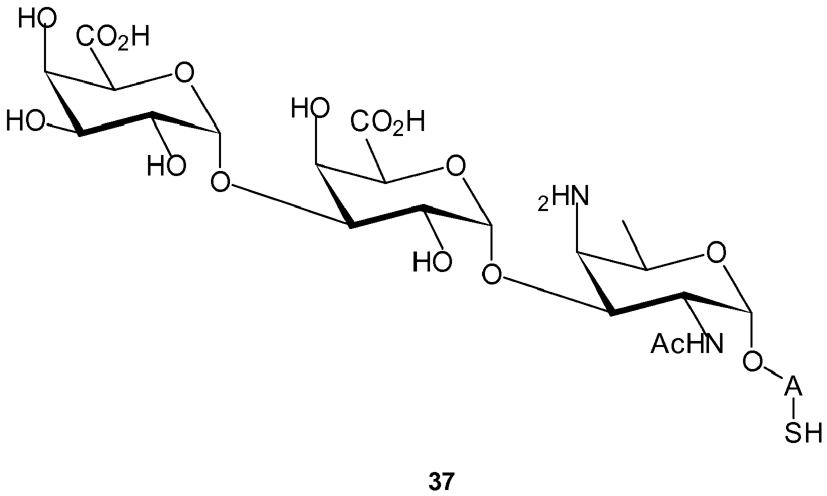 wherein A is defined as above.

3. Synthesis according to claim 2 further comprising step D:
D) preparing a salt of the compound of general formula (**I**) or preparing lyophilisate of the compound of general formula (**I**) or of the salt of the compound of general formula (**I**).

4. Synthesis according to claim 2, wherein the reactions between compounds **2** and **3,** compounds **2** and **12,** and compounds **2** and **23** are performed in tetrahydrofurane in presence of DMTST and TTBPy.

5. Synthesis according to claim 2, wherein the replacement of protecting group P⁶ on compound **28** with protecting group P¹³ to obtain compound **29** is performed in two steps, first involving the reaction of compound **28** with hydrazine or a hydrazinium salt in a mixture of polar and apolar solvents and second by treatment of the product obtained after the first step with BnOCH₂SCy, DMTST and TTBPy in an apolar solvent.

6. Synthesis according to claim 2, wherein the cleavage of the protecting groups involves first cleavage of the base-labile protecting groups by treatment with a base in a mixture of polar aprotic solvent and a polar protic solvent; and second cleavage of the protecting groups sensitive to hydrogenation by exposure to sodium and ammonia in a mixture of polar protic solvents.

7. Intermediate of the general formula (**II**): 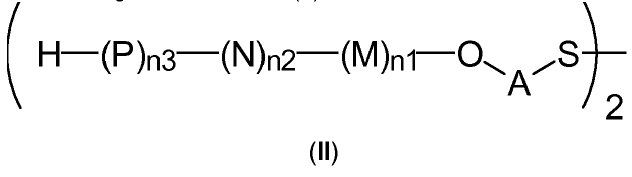 wherein A is a linker;
M, N and P represent independently of each other one of the following fragments: 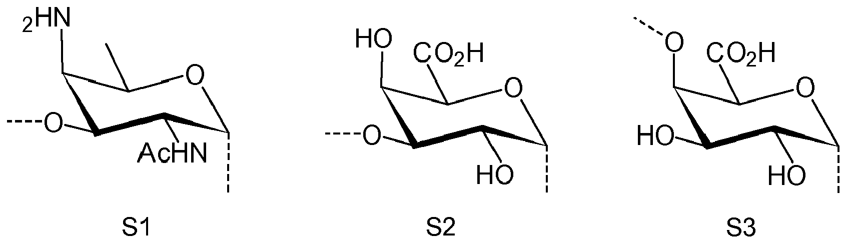 wherein each moiety S1, S2, and S3 is not more than once present in the general formula (**II**), sugar fragment S1 cannot be simultaneously bound to -O-A-S- and sugar fragment S2, sugar fragment S3 cannot be simultaneously bound to -O-A-S- and sugar fragment S1 and sugar fragment S2 cannot be simultaneously bound to -O-A-S-and sugar fragment S3, and
n1, n2 and n3 are integers selected from 0 and 1
wherein at least one of the integers n1, n2 and n3 is 1 and
and pharmaceutically acceptable salts of these saccharides.

8. Glycoconjugate obtained by reacting the saccharides of general formula (I) with an immunogenic carrier.

9. Glycoconjugate according to claim 8 useful as a vaccine for immunization against diseases associated with bacteria containing in their capsular polysaccharide a saccharide structure selected from:
| |
|---|
| α-2,4,6-trideoxy-4-amino-D-GalNAc-(1→4)-α-D-GalAp-(1→3)-α-D-GalAp- |
| α-2,4,6-trideoxy-4-amino-D-GalNAc-(1→4)-α-D-GalAp |
| α-D-GalAp-(1→3)-α-D-GalAp |
| α-D-GalAp |
| α-2,4,6-trideoxy-4-amino-D-GalNAc |
| α-D-GalAp-(1→3)-α-D-GalAp-(1→3)-α-2,4,6-trideoxy-4-amino-D-GalNAc |
| α-D-GalAp-(1→3)-α-2,4,6-trideoxy-4-amino-D-GalNAc |
| α-D-GalAp-(1→3)-α-2,4,6-trideoxy-4-amino-D-GalNAc-(1→4)-α-D-GalAp |

10. Glycoconjugate according to claim 9, wherein the bacteria is *Streptoccocus pneumoniae* serotype 1.

11. Glycoconjugate according to claim 9, wherein the diseases associated with bacteria include pneumonia, meningitis, otitis media, bacteremia and acute exacerbation of chronic bronchitis, sinusitis, arthritis and conjunctivitis.

12. Pharmaceutical composition comprising the glycoconjugate according to claim 8 and/or the saccharide according to claim 1 and/or the intermediate according to claim 7 together with at least one pharmaceutically acceptable cryoprotectant, lyoprotectant, excipient and/or diluent.

13. Pharmaceutical composition according to claim 12, for immunization against diseases associated with *Streptococcus pneumoniae* bacteria.

14. Pharmaceutical composition according to claim 13, wherein the *Streptococcus pneumoniae* bacteria is selected from the group containing or consisting of *Streptococcus pneumoniae* type 4, *Streptococcus pneumoniae* type 9V, *Streptococcus pneumoniae* type 2, *Streptococcus pneumoniae* type 19F, *Streptococcus pneumoniae* type 3, *Streptococcus pneumoniae* type 19A, *Streptococcus pneumoniae* type 12F, *Streptococcus pneumoniae* type 31, *Streptococcus pneumoniae* type 7F, *Streptococcus pneumoniae* type 5, *Streptococcus pneumoniae* type 14, *Streptococcus pneumoniae* type 6A, *Streptococcus pneumoniae* type 6B, *Streptococcus pneumoniae* type 18C and *Streptococcus pneumoniae* type 23F.

15. Use of the saccharide according to claim 1 and/or the intermediate according to claim 7 as a marker in immunological assays for diagnostics of diseases caused by bacteria containing in their capsular polysaccharide a saccharide structure selected from:
| |
|---|
| α-2,4,6-trideoxy-4-amino-D-GalNAc-(1→4)-α-D-GalAp-(1→3)-α-D-GalAp- |
| α-2,4,6-trideoxy-4-amino-D-GalNAc-(1→4)-α-D-GalAp |
| α-D-GalAp-(1→3)-α-D-GalAp |
| α-D-GalAp |
| α-2,4,6-trideoxy-4-amino-D-GalNAc |
| α-D-GalAp-(1→3)-α-D-GalAp-(1→3)-α-2,4,6-trideoxy-4-amino-D-GalNAc |
| α-D-GalAp-(1→3)-α-2,4,6-trideoxy-4-amino-D-GalNAc |
| α-D-GalAp-(1→3)-α-2,4,6-trideoxy-4-amino-D-GalNAc-(1→4)-α-D-GalAp |
